(19) Europäisches Patentamt
European Patent Office
Office européen des brevets

(11) EP 1 355 151 A2

(12) EUROPEAN PATENT APPLICATION

(43) Date of publication:
**22.10.2003 Bulletin 2003/43**

(51) Int Cl.[7]: **G01N 33/48**, C07H 21/04, C12Q 1/68

(21) Application number: **03252024.9**

(22) Date of filing: **31.03.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **29.03.2002 US 368687 P**

(71) Applicant: **Ortho-Clinical Diagnostics, Inc.
Rochester, NY 14626-5101 (US)**

(72) Inventor: **Wang, Yixin
San Diego, CA 92130 (US)**

(74) Representative: **Mercer, Christopher Paul et al
Carpmaels & Ransford
43, Bloomsbury Square
London WC1A 2RA (GB)**

# (54) Assessing colorectal cancer

(57) A method of assessing the presence or absence of colorectal cancer or the likely condition of a person believed to have colorectal cancer is conducted by analyzing the expression of a group of genes. Gene expression profiles in a variety of medium such as microarrays are included as are kits that contain them.



Printed by Jouve, 75001 PARIS (FR)

SEQUENCE LISTING

<110> Wang, Yixin

<120> COLORECTAL CANCER DIAGNOSTICS

<130> CDS 266 US NP

<150> 60/368,687
<151> 2002-03-29

<160> 49

<170> PatentIn version 3.1

<210> 1
<211> 1500
<212> DNA
<213> human

<400> 1
atggcttcga ccaccacctg caccaggttc acggacgagt atcagctttt cgaggagctt 60

ggaaaggggg cattctcagt ggtgagaaga tgtatgaaaa ttcctactgg acaaggatat 120

gctgccaaaa ttatcaacac caaaaagctt tctgctaggg atcatcagaa actagaaaga 180

gaagctagaa tctgccgtct tttgaagcac cctaatattg tgcgacttca tgatagcata 240

tcagaagagg gctttcacta cttggtgttt gatttagtta ctggaggtga actgtttgaa 300

gacatagtgg caagagaata ctacagtgaa gctgatgcca gtcattgtat acagcagatt 360

ctagaaagtg ttaatcattg tcacctaaat ggcatagttc acagggacct gaagcctgag 420

aatttgcttt tagctagcaa atccaaggga gcagctgtga aattggcaga ctttggctta 480

gccatagaag ttcaagggga ccagcaggcg tggtttggtt ttgctggcac acctggatat 540

ctttctccag aagttttacg taaagatcct tatggaaagc cagtggatat gtgggcatgt 600

ggtgtcattc tctatattct acttgtgggg tatccaccct tctgggatga agaccaacac 660

agactctatc agcagatcaa ggctggagct tatgattttc catcaccaga atgggacacg 720

gtgactcctg aagccaaaga cctcatcaat aaaatgctta ctatcaaccc tgccaaacgc 780

atcacagcct cagaggcact gaagcaccca tggatctgtc aacgttctac tgttgcttcc 840

atgatgcaca gacaggagac tgtagactgc ttgaagaaat ttaatgctag aagaaaacta 900

aagggtgcca tcttgacaac tatgctggct acaaggaatt tctcagcagc caagagtttg 960

ttgaagaaac cagatggagt aaaggagtca actgagagtt caaatacaac aattgaggat 1020

gaagatgtga aagcacgaaa gcaagagatt atcaaagtca ctgaacaact gatcgaagct 1080

atcaacaatg ggactttga agcctacaca aaaatctgtg acccaggcct tactgctttt 1140

gaacctgaag ctttgggtaa tttagtggaa gggatggatt ttcaccgatt ctactttgaa 1200

aatgctttgt ccaaaagcaa taaaccaatc cacactatta ttctaaaccc tcatgtacat 1260

ctggtagggg atgatgccgc ctgcatagca tatattaggc tcacacagta catggatggc 1320

agtggaatgc caaagacaat gcagtcagaa gagactcgtg tgtggcaccg ccgggatgga 1380

aagtggcaga atgttcattt tcatcgctcg gggtcaccaa cagtacccat caagccaccc 1440

tgtattccaa atgggaaaga aaacttctca ggaggcacct ctttgtggca aaacatctga 1500

<210> 2
<211> 5761
<212> DNA
<213> human

<400> 2
cacaccgcag tatgcggtgc cctttactct gagctgcgca gccggccggc cggcgctggt 60

tgaacagact gccgctgtac tggcgtggcc tggagggact cagcaaattc tcctgccttc 120

aacttggcaa cagttgcctg ggtagctct acacaactct gtccagccca cagcaatgat 180

tccagaggcc atggggagtg gacagcagct agctgactgg aggaatgccc actctcatgg 240

caaccagtac agcactatca tgcagcagcc atccttgctg actaaccatg tgacattggc 300

cactgctcag cctctgaatg ttggtgttgc ccatgttgtc agacaacaac aatccagttc 360

cctcccttcg aagaagaata agcagtcagc tccagtctct tccaagtcct ctctagatgt 420

tctgccttcc caagtctatt ctctggttgg gagcagtccc ctccgcacca catcttctta 480

taattccttg gtccctgtcc aagatcagca tcagcccatc atcattccag atactcccag 540

ccctcctgtg agtgtcatca ctatccgaag tgacactgat gaggaagagg acaacaaata 600

caagcccagt agctctggac tgaagccaag gtctaatgtc atcagttatg tcactgtcaa 660

tgattctcca gactctgact cttctttgag cagcccttat tccactgata ccctgagtgc 720

tctccgaggc aatagtggat ccgttttgga ggggcctggc agagttgtgg cagatggcac 780

tggcacccgc actatcattg tgcctccact gaaaactcag cttggtgact gcactgtagc 840

aacccaggcc tcaggtctcc tgagcaataa gactaagcca gtcgcttcag tgagtgggca 900

gtcatctgga tgctgtatca cccccacagg gtatcgagct caacgcgggg ggaccagtgc 960

agcacaacca ctcaatctta gccagaacca gcagtcatcg gcggctccaa cctcacagga 1020

gagaagcagc aacccagccc cccgcaggca gcaggcgttt gtggcccctc tctcccaagc 1080

cccctacacc ttccagcatg gcagcccgct acactcgaca gggcacccac accttgcccc 1140

ggcccctgct cacctgccaa gccaggctca tctgtatacg tatgctgccc cgacttctgc 1200

tgctgcactg ggctcaacca gctccattgc tcatcttttc tccccacagg gttcctcaag 1260

gcatgctgca gcctatacca ctcaccctag cactttggtg caccaggtcc ctgtcagtgt 1320

tgggcccagc ctcctcactt ctgccagcgt ggcccctgct cagtaccaac accagtttgc 1380

cacccaatcc tacattgggt cttcccgagg ctcaacaatt tacactggat acccgctgag 1440

tcctaccaag atcagccagt attcctactt atagttggtg agcatgaggg aggaggaatc 1500

atggctacct tctcctggcc ctgcgttctt aatattgggc tatggagaga tcctccttta 1560

ccctcttgaa atttcttagc cagcaacttg ttctgcaggg gcccactgaa gcagaaggtt 1620

tttctctggg ggaacctgtc tcagtgttga ctgcattgtt gtagtcttcc caaagtttgc 1680

cctattttta aattcattat ttttgtgaca gtaattttgg tacttggaag agttcagatg 1740

cccatcttct gcagttacca aggaagagag attgttctga agttaccctc tgaaaaatat 1800
tttgtctctc tgacttgatt tctataaatg cttttaaaaa caagtgaagc ccctctttat 1860
ttcattttgt gttattgtga ttgctggtca ggaaaaatgc tgatagaagg agttgaaatc 1920
tgatgacaaa aaaagaaaaa ttactttttg tttgtttata aactcagact tgcctatttt 1980
attttaaaag cggcttacac aatctccctt ttgtttattg gacatttaaa cttacagagt 2040
ttcagttttg ttttaatgtc atattatact taatgggcaa ttgttatttt tgcaaaactg 2100
gttacgtatt actctgtgtt actattgaga ttctctcaat tgctcctgtg tttgttataa 2160
agtagtgttt aaaaggcagc tcaccatttg ctggtaactt aatgtgagag aatccatatc 2220
tgcgtgaaaa caccaagtat tctttttaaa tgaagcacca tgaattcttt tttaaattat 2280
tttttaaaag tctttctctc tctgattcag cttaaatttt tttatcgaaa aagccattaa 2340
ggtggttatt attacatggt ggtggtggtt ttattatatg caaaatctct gtctattatg 2400
agatactggc attgatgagc tttgcctaaa gattagtatg aattttcagt aatacacctc 2460
tgttttgctc atctctccct tctgttttat gtgatttgtt tggggagaaa gctaaaaaaa 2520
cctgaaacca gataagaaca tttcttgtgt atagctttta tacttcaaag tagcttcctt 2580
tgtatgccag cagcaaattg aatgctctct tattaagact tatataataa gtgcatgtag 2640
gaattgcaaa aaatatttta aaaatttatt actgaattta aaaatatttt agaagttttg 2700
taatggtggt gttttaatat tttacataat taaatatgta catattgatt agaaaaatat 2760
aacaagcaat ttttcctgct aacccaaaat gttatttgta atcaaatgtg tagtgattac 2820
acttgaattg tgtacttagt gtgtatgtga tcctccagtg ttatcccgga gatggattga 2880
tgtctccatt gtatttaaac caaaatgaac tgatacttgt tggaatgtat gtgaactaat 2940
tgcaattata ttagagcata ttactgtagt gctgaatgag caggggcatt gcctgcaagg 3000
agaggagacc cttggaattg ttttgcacag gtgtgtctgg tgaggagttt ttcagtgtgt 3060
gtctcttcct tccctttctt cctccttccc ttattgtagt gccttatatg ataatgtagt 3120
ggttaataga gtttacagtg agcttgcctt aggatggacc agcaagcccc cgtggaccct 3180
aagttgttca ccgggattta tcagaacagg attagtagct gtattgtgta atgcattgtt 3240
ctcagtttcc ctgccaacat tgaaaaataa aaacagcagc ttttctcctt taccaccacc 3300
tctacccctt tccattttgg attctcggct gagttctcac agaagcattt tccccatgtg 3360
gctctctcac tgtgcgttgc taccttgctt ctgtgagaat tcaggaagca ggtgagagga 3420
gtcaagccaa tattaaatat gcattctttt aaagtatgtg caatcacttt tagaatgaat 3480
tttttttcc ttttcccatg tggcagtcct tcctgcacat agttgacatt cctagtaaaa 3540
tatttgcttg ttgaaaaaaa catgttaaca gatgtgttta taccaaagag cctgttgtat 3600
tgcttaccat gtccccatac tatgaggaga agttttgtgg tgccgctggt gacaaggaac 3660
tcacagaaag gtttcttagc tggtgaagaa tatagagaag gaaccaaagc ctgttgagtc 3720
attgaggctt ttgaggtttc ttttttaaca gcttgtatag tcttggggcc cttcaagctg 3780

tgaaattgtc cttgtactct cagctcctgc atggatctgg gtcaagtaga aggtactggg 3840
gatggggaca ttcctgccca taaaggattt ggggaaagaa gattaatcct aaaatacagg 3900
tgtgttccat ccgaattgaa aatgatatat ttgagatata attttaggac tggttctgtg 3960
tagatagaga tggtgtcaag gaggtgcagg atggagatgg gagatttcat ggagcctggt 4020
cagccagctc tgtaccaggt tgaacaccga ggagctgtca aagtatttgg agtttcttca 4080
ttgtaaggag taagggcttc caagatgggg caggtagtcc gtacagccta ccaggaacat 4140
gttgtgtttt ctttattttt taaaatcatt atattgagtt gtgttttcag cactatattg 4200
gtcaagatag ccaagcagtt tgtataattt ctgtcactag tgtcatacag ttttctggtc 4260
aacatgtgtg atctttgtgt ctccttttttg ccaagcacat tctgattttc ttgttggaac 4320
acaggtctag tttctaaagg acaaattttt tgttccttgt cttttttctg taagggacaa 4380
gatttgttgt ttttgtaaga aatgagatgc aggaaagaaa accaaatccc attcctgcac 4440
cccagtccaa taagcagata ccacttaaga taggagtcta aactccacag aaaaggataa 4500
taccaagagc ttgtattgtt accttagtca cttgcctagc agtgtgtggc tttaaaaact 4560
agagattttt cagtcttagt ctgcaaactg gcatttccga ttttccagca taaaaatcca 4620
cctgtgtctg ctgaatgtgt atgtatgtgc tcactgtggc tttagattct gtccctgggg 4680
ttagccctgt tggccctgac aggaagggag gaagcctggt gaatttagtg agcagctggc 4740
ctgggtcaca gtgacctgac ctcaaaccag cttaaggctt taagtcctct ctcagaactt 4800
ggcatttcca acttcttcct ttccgggtga gagaagaagc ggagaagggt tcagtgtagc 4860
cactctgggc tcatagggac acttggtcac tccagagttt ttaatagctc ccaggaggtg 4920
atattatttt cagtgctcag ctgaaatacc aaccccagga ataagaactc catttcaaac 4980
agttctggcc attctgagcc tgcttttgtg attgctcatc cattgtcctc cactagaggg 5040
gctaagcttg actgccctta gccaggcaag cacagtaatg tgtgttttgt tcagcattat 5100
tatgcaaaaa ttcactagtt gagatggttt gttttaggat aggaaatgaa attgcctctc 5160
agtgacagga gtggcccgag cctgcttcct attttgattt tttttttttt taactgatag 5220
atggtgcagc atgtctacat ggttgtttgt tgctaaactt tatataatgt gtggtttcaa 5280
ttcagcttga aaaataatct cactacatgt agcagtacat tatatgtaca ttatatgtaa 5340
tgttagtatt tctgctttga atccttgata ttgcaatgga attcctactt tattaaatgt 5400
atttgatatg ctagttattg tgtgcgattt aaactttttt tgctttctcc cttttttttgg 5460
ttgtgcgctt tcttttacaa caagcctcta gaaacagata gtttctgaga attactgagc 5520
tatgtttgta atgcagatgt acttagggag tatgtaaaat aatcatttta acaaaagaaa 5580
tagatattta aaatttaata ctaactatgg gaaaagggtc cattgtgtaa aacatagttt 5640
atctttggat tcaatgtttg tctttggttt tacaaagtag cttgtatttt cagtattttc 5700
tacataatat ggtaaaatgt agagcaattg caatgcatca ataaaatggg taaattttct 5760
g 5761

<210> 3
<211> 2129
<212> DNA
<213> human

<400> 3
ctgtattgag acaaaggaag ggatctgtca gaaagcaaca cttgttatct tgggcttggc 60

agcaaggaag aggacaggta gtggagatcc tgcaatctga aaagcagact gaaaggtgac 120

aaagaagctg aagatgggtg gtggagagag gtataacatt ccagcccctc aatctagaaa 180

tgttagtaag aaccaacaac agcttaacag acagaagacc aaggaacaga attcccagat 240

gaagattgtt cataagaaaa aagaaagagg acatggttat aactcatcag cagctgcctg 300

gcaggccatg caaaatgggg ggaagaacaa aaattttcca aataatcaaa gttggaattc 360

tagcttatca ggtcccaggt tactttttaa atctcaagct aatcagaact atgctggtgc 420

caaatttagt gagccgccat caccaagtgt tcttcccaaa ccaccaagcc actgggtccc 480

tgtttccttt aatccttcag ataaggaaat aatgacattt caacttaaaa ccttacttaa 540

agtacaggta taaaataaga caaatgttta aatttagtta tgttcacggg tagttgtcaa 600

ttggtctgaa acaaatttgc tagggaatct atttgtgtag aactaattaa tgtaaaaaaa 660

atagaccatc tcgtgttgtg tgcactgtga tataatggta gtatcagtgc aacttaaact 720

aatgattgta cttgatatta agtgttctca actgagtaac ttttaagtgg aaaccaagtt 780

tagatttggg gagtggtaaa ggaatcagct tttctattg ttaggggaag acagtaattt 840

atcattcatg gaccagtaga ttgttgaaag ttggtgaatc ggattataag cttctagcta 900

acacaaggat tcagaattag gtaaacatct gaaggtttag tatattagaa acacccaaac 960

cagtaatatg ctaacctgat gcactgctga aagaaaatgt gaatttttcg taataattgc 1020

attttagtga attgtacagt gggtggaaag ggcatttgga gctcattaga atgagacata 1080

gtacacccca atggccctgt ttattaaatg tagtggatta agtgtctgtc aacaaataca 1140

ccaaaaccat tttttataga aacagtattt aatggtcact caatagcttt caaaatacat 1200

ttttgtatta cagcactgca caagctattc taatagtgct ctggcctcat cattcctgca 1260

aagcttgctt tggggagttg gataatgtga aaattttaag tacctagggg agaaagagcc 1320

atgtaaatat ctgtaataaa cttgtagcat atgtaaagtt ttcttggcct ttatcttaca 1380

aaaatggagt attttagtat gaatttgctg aatgtaagac cgtggactgt tttttataat 1440

atggcctaat tttaaaggtc caaaataact tgtttttaaa gtttgccctt gtgctaaagt 1500

gccagtgtat gtatgttata cttgatttgg ttgtaaacta tatttcaaag taaaccctag 1560

tgtaataagt tttataacta aaaaggttta agctgctaaa actattttta agagatgtga 1620

aatgcagtat gggactatct ttttttcctc ctctaagccc aaagattaac tagagtccct 1680

ccaaccttat agattgttgg ctttcacaat cttataacct aggatacagg tagtttcgag 1740

tatggtgcca gtgatgtttt gtttttgttt ggtcaagggg taggtgcaac ccaatggacc 1800

acttatgcaa aagatgtaaa ctcttgcata atacattgat aacatgtttt gccaacttta 1860

aatgcttaaa cataagcgaa accagtagca agtatgtggg tcagcttaaa aattttgatt 1920

gttaatgccc tattttctaa tttggcacct cttgatgcct aagcaggtaa gcagatgcct 1980

aagctgtatt tctccaaata aatcaagatg aagtactgcc caagttaaat attgatagcc 2040

taaagacaag tttatgtagt acttaatgta catgatatga agcataaaat taaataaaat 2100

ttttccccat tgaaaaaaaa aaaaaaaaa 2129

<210> 4
<211> 3950
<212> DNA
<213> human

<400> 4
cgagaactag ttttgttccg tgccctctgg actggaacct tttggagaga acccccggca 60

ggaccaaccc cgcacccgcc agcaccgcgg caatgtccag caatagtttt ccttacaatg 120

agcagtccgg aggaggggag gcgacggagc tgggtcagga ggcgacctca accatttccc 180

cctcgggggc cttcggcctc tttagcagcg atttgaagaa gaatgaagat ctaaagcaaa 240

tgttagagag caacaaagat tctgctaaac tggatgctat gaagcggatt gttgggatga 300

ttgcaaaagg gaaaaatgca tctgaactgt ttcctgctgt tgtgaagaat gtggccagta 360

aaaatattga gatcaagaag ttggtatatg tttacctggt tcgatatgct gaagaacagc 420

aggatcttgc actcctgtcc ataagcactt ttcagcgagc tctgaaggac cccaaaccaac 480

taattcgtgc aagcgctttg agagttctgt caagtattag agtgccaatt attgtaccta 540

tcatgatgct tgctattaag gaagcttctg ctgacttatc accatatgtt aggaagaatg 600

cagcccatgc aatacaaaaa ttatacagcc ttgatccaga gcagaaggaa atgttaattg 660

aagtaattga aaaacttctg aaagataaaa gcacattggt agctggcagt gttgtgatgg 720

cttttgaaga agtatgcccg gacagaatag atctgattca taaaaattac cgcaagctat 780

gtaacttact agtggatgtt gaagagtggg ggcaggttgt cataatccac atgctaactc 840

gatatgctcg gacacagttt gtcagcccctt ggaaagaggg tgatgaatta gaagacaatg 900

gaaagaattt ctacgaatct gatgatgatc agaaggaaaa gactgacaaa aagaagaagc 960

cgtatactat ggatccagat catagactct taattagaaa tacaaagcct ttgcttcaga 1020

gcaggaatgc tgcggtggtt atggcagttg ctcagctgta ttggcacata tcaccaaaat 1080

ctgaagctgg cataatttct aaatcactag tgcgtttact tcgtagcaat agggaggtgc 1140

agtatattgt cctacaaaat atagcaacta tgtcaattca aagaaagggg atgtttgaac 1200

cttatctgaa gagtttctat gttaggtcaa ctgatccaac tatgatcaag acactgaagc 1260

ttgaaatttt gacaaacttg gcaaatgaag ccaacatatc aactcttctt cgagaatttc 1320

agacctatgt gaaaagccag gataaacaat ttgcagcagc cactattcag actataggca 1380

gatgtgcaac caacatcttg gaagtcactg acacgtgcct caatggcttg gtctgtctgc 1440

tgtccaacag ggatgaaata gttgttgctg aaagtgtggt tgttataaag aaattactgc 1500

aaatgcaacc tgcacaacat ggtgaaatta ttaaacatat ggccaaactc ctggacagta 1560
tcactgttcc tgttgctaga gcaagtattc tttggctaat tggagaaaac tgtgaacgag 1620
ttcctaaaat tgcccctgat gttttgagga agatggctaa aagcttcact agtgaagatg 1680
atctggtaaa actgcagata ttaaatctgg gagcaaaatt gtatttaacc aactccaaac 1740
agacaaaatt gcttacccag tacatattaa atctcggcaa gtatgatcaa aactacgaca 1800
tcagagaccg tacaagattt attaggcagc ttattgttcc gaatgaaaag agtggagctt 1860
taagtaaata tgccaaaaaa atattcctag cacaaaagcc tgcaccactg cttgagtctc 1920
cttttaaaga tagagatcat ttccagcttg gcaccttatc tcatactctc aacattaaag 1980
ctactgggta cctggaatta tctaattggc cagaggtggc gcccgaccca tcagttcgaa 2040
atgtagaagt aatagagttg gcaaaagaat ggaccccagc aggaaaagca aagcaagaga 2100
attctgctaa gaagttttat tctgaatctg aggaagagga ggactcttct gatagtagca 2160
gtgacagtga gagtgaatct ggaagtgaaa gtggagaaca aggcgaaagt ggggaggaag 2220
gagacagcaa tgaggacagc agtgaggact cctccagtga gcaggacagt gagagtggac 2280
gggagtcagg cctagaaaac aaaagaacag ccaagaggaa ctcaaaagcc aaaggaaaaa 2340
gtgattctga agatggggag aaggaaaatg aaaaatctaa aacttcagat tcttcaaatg 2400
acgaatctag ttcaatagaa gacagttctt ccgattctga atcagagtca gaacctgaaa 2460
gtgaatctga atccagaaga gtcactaagg agaaagaaaa gaaaacaaag caagatagaa 2520
ctcctcttac caaagatgtt tcacttctag atctggatga ttttaaccca gtatccactc 2580
cagttgcact tcccacacca gctctttctc caagtttgat ggctgatctt gaaggtttac 2640
acttgtcaac ttcctcttca gtcatcagtg tcagtactcc tgcatttgta ccaacgaaaa 2700
ctcacgtgct gcttcatcga atgagtggaa aaggactagc tgcccattat ttctttccaa 2760
gacagccttg catttttggt gataagatgg tctctataca aataacactg aataacacta 2820
ctgatcgaaa gatagaaaat atccacatag gggaaaaaaa acttcctata ggcatgaaaa 2880
tgcatgtttt taatccaata gactctcttg agcctgaggg atccattaca gtttcaatgg 2940
gtattgactt ttgtgattct actcagactg ccagtttcca gttgtgtacc aaggatgatt 3000
gcttcaatgt taatattcag ccacctgttg gagaactgct tttacctgtg gccatgtcag 3060
agaaagattt taagaaagag caaggagtgc taacaggaat gaatgaaact tctgctgtaa 3120
tcattgctgc accacagaat ttcactccct ctgtgatctt tcagaaggtt gtaaatgtag 3180
ccaatgtagg tgcagtccct tctggccagg ataatataca caggtttgca gctaaaactg 3240
tgcacagtgg gtcattgatg ctagtcacag tggaactgaa ggaaggctct acagcccagc 3300
ttatcataaa cactgagaaa actgtgattg gctctgttct gctgcgggaa ctgaagcctg 3360
tcctgtctca ggggtaacct gcttacatct ggactttaga atctggcaca caacaaaagt 3420
gcctggcatc cactactgct gcctttcatt tataataata gcccttccat ctggcagtgg 3480
gggtagaata cactcttgac attcttgtct cctgctttag aatgctagtg tgtatctatc 3540

atgtatgcaa tactttcccc ctttttgctt tgctaaccga agagcatata ttttactgtc 3600
agttgtctca actcttgaat ccatgtggcg ttttctctgt cctgctgctt cttttggcct 3660
cctcgttttc cttctctttt tcgacaatgg tagacatgaa tgagatattt aaagttcatt 3720
ggaaatcttc ttccctacag cagtaagcaa aaattagcaa agagatagtc taaatggcct 3780
ctcagcttgg tatgtgaaaa tgagatcaca tactttttaa atccaaatac aaaagcatag 3840
tctctgcaag attttgttct ttgaatttct tgatattgta attgattatt gataactgtc 3900
atcatgaaat tatctctcaa taataagata aataaactag catatgaatc 3950

<210> 5
<211> 5191
<212> DNA
<213> human

<400> 5
gagaaagaaa aacagctcga gacctcatgc aaagagaaaa ctgagtatct acagaaaatg 60
gttcagagga atgaaagata taaacaagat gtggagaggt tctatgaacg gaagcgacat 120
ttagatttaa ttgagatgct tgaagcaaaa aggccatggg tggaatatga aaatgttcgt 180
caggaatatg aagaagtaaa actagttcgt gaccgagtga aggaagaggt cagaaaactt 240
aaagaagggc agattcctat aacatgtcga attgaagaaa tggaaaacga gcgtcacaat 300
ttggaggctc gaatcaaaga aaaggcaaca gatattaagg aggcatctca aaaatgcaaa 360
cagaagcaag atgttataga aaggaaagat aaacatattg aggaacttca gcaggcttta 420
atagtaaagc aaaatgaaga gcttgaccga cagaggagaa taggtaatac ccgcaaaatg 480
atagaggatt tgcaaaatga actaaagacc acggaaaact gcgagaatct tcagccccag 540
attgatgcca ttacaaatga tctgagacgg attcaggatg aaaaggcatt atgtgaaggc 600
gaaataattg ataagcgaag agagagggaa actctagaga aggagaaaaa gagtgtggac 660
gatcatattg tacgttttga caatcttatg aatcagaagg aagataagct aagacagaga 720
ttccgtgaca cgtatgatgc tgttttatgg ctaagaaata acagagacaa atttaaacaa 780
agagtctgtg agcccataat gctcacgatc aatatgaaag ataataaaaa tgccaaatat 840
attgaaaatc atattccatc aaatgactta agagcctttg tatttgaaag tcaagaagat 900
atggaggttt tcctcaaaga ggttcgtgac aataaaaaat taagagtaaa tgctgttatt 960
gctcccaaga gttcatatgc agacaaagca ccttcaagat ctttgaatga acttaaacaa 1020
tacggatttt tctcttattt gagagaatta tttgatgcac ctgatcctgt aatgagttac 1080
ctttgctgtc agtatcatat tcatgaagtt cctgtaggaa ctgaaaagac cagagaaaga 1140
attgaacggg taatacaaga aacccgatta aaacagattt atacagcaga agaaaagtat 1200
gtggtgaaaa cttcttttta ttcaaacaaa gttatttcta gtaacacatc tctaaaagta 1260
gcgcagtttc tcactgtcac tgtggaccta gagcagagaa gacacttaga agaacagcta 1320
aaggaaattc atagaaaatt gcaagcagtg gattcagggt tgattgcctt acgtgaaaca 1380
agcaaacatc tggagcacaa agacaatgaa cttagacaaa agaagaagga gcttcttgag 1440

agaaaaacca agaaaagaca actggaacaa aaaatcagtt ccaaactagg aagtttaaag 1500
ctgatggaac aggatacttg caatcttgaa gaggaagagc gaaaagcaag taccaaaatc 1560
aaagaaataa atgttcaaaa agcgaaactt gttaccgaat taacaaacct aataaagatt 1620
tgtacttctt tgcatataca aaaagtagat ttaattctcc aaaatactac agtgatctct 1680
gagaagaaca aattagaatc agattatatg gccgcatctt cacaactccg tcttacagag 1740
caacatttca ttgaattgga tgaaaataga cagagattat tgcagaaatg caaggaactt 1800
atgaaaagag ctaggcaagt atgtaacctg ggtgcagagc agactcttcc tcaagaatac 1860
cagacacaag tacccaccat tccaaatgga cacaactcct cactccccat ggttttccaa 1920
gaccttccaa acacattgga tgaaattgat gctttattaa ctgaagaaag atcaagagct 1980
tcctgcttca cgggactgaa tcctacaatt gttcaggaat atacaaaaag agaagaagaa 2040
atagaacagt taactgagga actaaaggga aagaaagttg aactagatca atacagggaa 2100
aacatttcac aggtaaaaga aaggtggctt aatcctttaa aagagctggt agaaaaaatt 2160
aatgaaaaat tcagcaattt ttttagttcc atgcagtgtg ctggtgaagt tgatctccat 2220
acagaaaatg aggaagatta tgataaatat ggaattcgaa ttagagtcaa atttcgaagt 2280
agtactcaac tgcatgaatt aactcctcat catcaaagtg gaggtgaaag aagtgtttct 2340
accatgttat acttgatggc acttcaggag ctaaatagat gtccattcag agtagttgat 2400
gaaatcaatc agggaatgga cccaatcaat gaacggagag tgtttgaaat ggttgtaaat 2460
actgcctgta aagaaaatac atctcaatac tttttcataa caccaaagct cctgcaaaat 2520
cttccttatt ctgaaaagat gacagttttg tttgtctaca atggccctca tatgctggaa 2580
ccaaacacat ggaatttaaa ggctttccaa aggcggcggc gccgtattac attcactcaa 2640
ccttcttaat aaaagtaaag agagggaact tgggaatttt ttttgttaaa ttctgtttat 2700
aagtatggct caactgaata aaaggagatt cactaaaacg aaaagcagtt atttttggaa 2760
acctgctttt aaatacaaat aggttgataa tggaaactat aatgaccttt ccaaaatagc 2820
agctggtagt aaaagttaag tcttcttcag tcttggttga acttgagttc ttggcactct 2880
gaccatgagt cattcagttc tcatgttaaa atgtacttaa tattacaaat caaaggtaca 2940
gtggaagaag ggttaatcac aagaagttac ttatatggta gccctgagct ttaattgcag 3000
agtaacttta attactttta gagcctaaag atgactctag agcctaagtc ctagtttctc 3060
ccaatgttat atttaatttt aaaaaattga tatgaaaatg tctaatgtat agtaataatt 3120
tatgacagat ctagtcattt cttcctatta aaaaagatta ccttatctcc agtaggaaat 3180
ggaattttat gggcctttaa aagaaagttt tatgaaactt gatgctataa ttttattggt 3240
atttcaaggg gaaaaaagca ctggggttca aaaatggtag cagaactgct ttgaaatgct 3300
gcaaggtggc cactagatga tgcaaaatac aaccaaaaga ttgactgaga ataaaattag 3360
gtgacaaggg tttttaaaga ataacctttt aaagtgtggg ggcaggggtt gctttttttt 3420
attttattta aagtcaatta tattttacat cttacatttc taaaagcatt ttataattat 3480

ttttagtaag atttttctta aaatttcata tactggtttc tacaatttat atttgaaatt 3540
tctcagtgtt atgtaaagag tgatggaaaa gcattgattt ctttaaaacc gtaatgtttt 3600
tagaacttaa gcctataggg cctttcttac aatgttgatg tacccattat cttagaaaat 3660
ctagtttaaa ctgttttctt tcaccgcaaa agaattaaat gggaaaatca tttgtttatc 3720
tctaagttat actaattagt agaaccaaac aaattatctt cttttaaaaa ataaatctta 3780
taggaaaata gacagtccaa agtcatgtct ttgaacagtg gattggatct gtgccagtaa 3840
tgacaaaatt attttttga cttgcttgcc tgaataaatt gaagaattgc tttcagtttg 3900
ggttttgtat attcttaagt agccattgaa atttatattc ttaactaggt caaaaaataa 3960
tgagccataa gtttatgtcc tctcacttag acattttctc tttaaaaagg tattttcttc 4020
tttataaaca ttttaaaaga gccttccctt cttaaactaa ctccagtgca tgaagtgtga 4080
aaatatttta aaatgacatt tttactaata tgagcaagtc atgtaaacat tgaagaactt 4140
ggtaacatat tagtaaatgg atattaccaa atgttttcat cgttaattac tttgcgttcc 4200
accaaaatat ctttactaaa atgtgcttgg tgtagtttgt ttattgtcta aattagtacc 4260
agtcatctta tttctgcaaa atgagtatca atgtgaaaaa gacacgtgaa gattaagcat 4320
gtttgaaaat aaaatggtca attacatttc aatttacata ggccaacaac tgttccatac 4380
tttgtttgta aacatttaat ttctctactg gacaaaatta atatttggct ttacattgaa 4440
ttttgagctg tgaagaataa attatgtatc attttagcat attaaacagt agtaagtcta 4500
gcacatagtc tcagccactt aaaacaaaag tttttttgtt tgtttgtttg tttgtttttt 4560
tgagatggag tctcactctg ttgcccaggc tggagtgcag tggcgtgatc tcggcttact 4620
gcaacctccg cctcccgggt tcaagcgatt ctcctgcctc agcctcccaa gtaactggga 4680
caacaggcgc gtcccaccac acccagctaa tttttatac ttttagtaga gatggggttt 4740
cagcatattg gccaggctgg tctcgaactc ctgaccttgt gatccacccg cctcggcctc 4800
ccaaagtgct gggattatag gcgtgagccc ctgcacccgg ccaaaagttg atttttaatt 4860
acataaaaat cgtaaaaact tctagtaaaa acttgatttg gtgaatacag ttatatttta 4920
aaaccttaag gtgacaagca ttttctatgc ctaaatcttc attggtttgc ctggaaagag 4980
tctctgttaa aagattttcc atattcaaag taaaaggaaa gatttcttgc ttcctaattg 5040
tcttttggac acatgcctat tttctttgag gtataaacct ttagatgtga aaaatgtaat 5100
ttcattctgc tattgtgtgt gcttgtgtgt gtgtaattga aaaaactggg aaatcctgct 5160
ttgttggtaa taaatcaata tttttatatt c 5191

<210> 6
<211> 4755
<212> DNA
<213> human

<400> 6
aagagatctt ccaggctctc agagccctgg gagggcgatt tccaggaaga ccacaatgcc 60

aacctctgga ggaggctgga gagagaaggc ctaggccaga gcctgtcagg caactttggc 120
aagaccaagt cagccttctc atctctccag aacattcctg agagtctgag aagacacagc 180
agcctggagc taggccgggg aacccaggag ggttaccccg ggggcaggcc cacctgtgca 240
gtcaacacca aggcagaaga ccctgggagg aaagccgctc ctgacctcgg gagccatctg 300
gaccggcagg tttcctaccc gcggcccgag gggaggaccg gtgcctcggc ttctttcaac 360
agcacagacc caagtcccga agagccgcct gccccctcgc acccgcacac atccagtctg 420
ggccggaggg ggcccggccc aggcagcgcc tcggctcttc agggctttca gtacgggaag 480
ccccactgct cggtgctgga gaaggtctcc aaattcgagc agcgagagca agggagccag 540
agaccgagtg tgggcggctc tggttttggc cataactata ggccccacag gaccgtctca 600
acttccagta cttctgggaa tgacttcgag gagacaaaag cacacattcg tttctctgag 660
tcagctgaac ccctaggcaa cggggagcag cacttcaaaa acggggagct gaagttggaa 720
gaggcttccc ggcagccctg cggtcagcag ctgagcggag gagcgtcgga cagcggccgt 780
ggcccccaga ggccggacgc tcggctcctc cgtagccaga gcaccttcca gctctccagc 840
gagccagaga gggagcccga gtggcgggac aggcccggct cgcccgaatc gcccctgctg 900
gatgcccccct tcagccgcgc ctaccggaac agcatcaagg acgcacagtc ccgtgtcttg 960
ggggccacct cctttcgacg tcgagacctg gagctggggg cgcccgtggc gtcgaggtcc 1020
tggcggccac ggccttcctc ggcccacgtg gggctgcgga gccccgaggc gtcggcctcc 1080
gcctccccgc acacgccccg ggagcggcac agcgtgaccc ctgctgaggg cgacctggcc 1140
aggcccgtgc cccctgccgc ccggagaggt gctcgccggc gcctgactcc cgagcagaag 1200
aagcgctcct actcggagcc cgagaagatg aacgaggtgg ggatcgtgga ggaggccgaa 1260
ccggcacccc tgggcccgca gagaaatggg atgcgtttcc cggagagcag cgtggccgac 1320
cggcgccgtc tcttcgagcg cgatggcaag gcctgctcca cgctcagcct gtcggggccc 1380
gagctgaagc agttccagca gagcgccctg gcggactaca tccagcgcaa gaccggcaag 1440
cggcctacct ccgccgccgg ctgcagcctc caggagcccg ggccactgcg tgagcgcgcc 1500
cagagtgcct acctccagcc cggccccgcg gcgctcgaag gctccggcct cgcctcggcc 1560
tccagcttga gctcactgcg gggagcccagc ctgcagcccc gcagggaggc cacgctcctg 1620
ccggccacag ttgcagaaac ccagcaggct ccccgagatc gcagcagctc cttcgccggt 1680
ggccgccgcc tcggggaacg gcgacgcggg gacctgctta gcggagcaaa cggtggaaca 1740
aggggcaccc agagaggggga tgagaccccc agggagccat cctcctgggg ggccagggcc 1800
gggaagtcca tgtcggccga ggacctgctg gaacgctcgg acgtccttgc gggccctgtc 1860
catgtgaggt ccaggtcatc tcccgccacc gcagacaagc gccaggatgt gcttttgggg 1920
caagacagtg gctttggtct tgtgaaggat ccatgttatt tggctggtcc tggatctagg 1980
tcactcagtt gttcagaaag aggccaagaa gagatgctgc tgctcttcca ccatctcacc 2040
cctcgttggg gtggttcagg ctgcaaagcc attggtgatt cctccgttcc tagtgaatgt 2100

cctggaaccc tggaccatca gaggcaagcc agtaggacac cctgccccag gccaccactg 2160
gcaggaacgc aagggctggt cacagacacc agggctgcac ccctgacccc aattggcacc 2220
cctctgcctt cagccattcc ctctggctac tgctcacagg acggtcagac agggcgacag 2280
cctctcccgc cctacacccc tgccatgatg cacagaagca atggtcacac cctgacccag 2340
cctcccggtc caagaggctg tgagggcgat ggcccagagc atggggtaga agagggaacg 2400
aggaagaggg tctcgctgcc tcagtggcca cctccttctc gagcaaagtg ggcccacgca 2460
gccagagagg acagccttcc tgaggaatcc tcagcccctg attttgcaaa cctgaagcac 2520
tatcaaaaac agcagagtct tccaagttta tgcagcactt ctgacccaga cacacctctt 2580
ggggccccga gcactccagg gaggatctcc ctccgaatat ctgagtctgt cctgcgggac 2640
tccccgccac ctcatgagga ttatgaagac gaagtgtttg tgagggatcc gcaccccaag 2700
gccacgtcca gccccacatt tgaacctctt cccccacccc cacctcctcc accgagtcag 2760
gaaaccccgg tgtatagcat ggatgacttc cctccacctc ctccccacac tgtatgtgag 2820
gcgcagctgg acagtgagga tcccgagggg ccacgcccca gcttcaacaa actttctaaa 2880
gtgacaattg caagggaaag gcacatgcct ggtgcagccc atgtggtagg tagtcagaca 2940
ctggcttcca gactccaaac ttctatcaag ggttcagagg ctgagtccac accaccctcc 3000
ttcatgagcg ttcacgccca acttgctggg tctcttggtg ggcagccagc acccatccag 3060
actcaaagcc tcagccatga tccagtcagt ggaactcagg gtttagaaaa gaaagtcagt 3120
cctgatcctc agaagagttc agaagacatc agaacagagg ctttggccaa ggaaattgtc 3180
caccaagaca aatctctagc agacattttg gatccagact ccaggctgaa gacaacaatg 3240
gacctgatgg aaggtttgtt tccccgagat gtgaacttgc tgaaggaaaa cagtgtaaag 3300
aggaaggcca tacagagaac tgtcagctct tcaggatgtg aaggcaagag gaatgaagac 3360
aaggaagcag tgagcatgtt ggttaactgc cctgcctact acagtgtgtc tgctcccaag 3420
gctgagctac tgaacaaaat caaagagatg ccagcagaag tgaatgagga agaggaacag 3480
gcagatgtca atgaaaagaa ggctgagctc attggaagtc tcacccacaa gctggagacc 3540
ctccaggagg cgaaggggag cctgctcacg gacatcaagc tcaacaacgc cctgggagaa 3600
gaggtggagg ctctgatcag cgagctctgc aagcccaatg agtttgacaa gtataggatg 3660
ttcatagggg atttggacaa ggtggtcaac ctgctgctct ccctctcggg gcgtctagcc 3720
cgtgttgaga atgtccttag cggccttggt gaagatgcca gtaatgaaga aaggagctct 3780
ctttacgaga aaaggaagat cctggctggt cagcatgagg atgcccggga gctgaaggag 3840
aacctggatc gcagggagcg agtagtgctg ggcatcttgg ccaattacct ttcagaggag 3900
cagctccagg actaccagca cttcgtgaaa atgaagtcca cgctcctcat tgagcaacgg 3960
aagctggatg acaagatcaa gctgggccag gagcaggtca agtgtctgct ggagagcctg 4020
ccctcagatt tcattcccaa ggctggggcc ctggctctgc ccccaaacct cacgagtgag 4080
cccattcctg ctgggggctg tactttcagt ggtattttcc caacattaac ctctccactt 4140

taacctcttc taaaataccc aaccaaaaga tcactgtttc tctcaacact atttaatctg 4200

aaaaatgttt cagtacaaac cactgtttga actatctggg ttattggtgt ttgttcctga 4260

tgaaaggaaa aaaattctct ccaggaggaa gcctttttcc ttcttgccct tcctgattga 4320

tcttctgaga gctcgaatgc tgctggacac gtaccccttt ctattattac tttgtagtag 4380

aaagaaagtt aatgaaactg agaactgatt ggagggtgtt tgatcattta gtttttaaca 4440

ggctgaggca acatggatca gtgtgtgtcc ccctcaggaa tgtatccaca gtggccttcc 4500

ttgctggtgg gcagtgtatc ctgatggcag ggtacaagta ccattaatga agggtctgca 4560

acataaagcc ttaaaaagac acacactaag aaaactgtaa aaccttgaac attgttattt 4620

atattttta aaatggaaaa gatcactatg tttgttgtgc taaccactta tttgattctg 4680

ttttgtggtg gacatagatg attacgtttg agctttgtat tttgtgaaaa ccttaatgaa 4740

atgaattcca aagat 4755

<210> 7
<211> 2045
<212> DNA
<213> human

<400> 7
gaaacttgac cccggctcat cctgtctctg gctgtggccc ggcaaagcac tgaaaacccc 60

tctggtctca gagacagtag gggcagtgcc actttctaca acctgccaac ccacacactg 120

gagtaattct gaaaaaaatt attcctaaac tctctaagtg tggacggaga atgagcaagc 180

cccagaagta ttttacaacc agagtgggta atgaggaggg ggcttactgg aatcgtcata 240

tctctgaata ttgaaaacaa caactaaaaa agtggacctt ctcagaaaaa aagggcagca 300

aatgaccaag ggcgcccctt ctggccgtgc ttggcttgag taactgtctc tctttcccca 360

cccccatcac agggctttca gtttggcaaa ggaaaagcag ataaaaacag aacattccat 420

atgtttcttt ctccatcggc caaaaacatt ttgacacaat gtttgtgaaa cacctttgga 480

gaggtgcact tctgaatgct gcctctgccg taaatcctgg ggcaagggat cagcctcttc 540

ccaggaacca tcgccttcta taaaccgtga actcaagcag gcattttttt tttcttaccg 600

aaaggctgct attgtgcaag ggcacataat gggtctgttg ctcttattgg cttccaaatg 660

tgcatggcaa agagagagat gtgggcctag agcagatata ttcagcaagg tgacagcttc 720

ccataacaat tctaacactt cttatcttat gtgagaataa aatatttaag ggttgaacct 780

tattttgcca aatgtatctt ttctgctttt gaattgggca gaagatttta gcaactatat 840

tctacaaatg ttacttataa cacacacaca cacatctgaa atatatgccg aaaattgacg 900

tctttgacct cagggagagc acctgtccag gtctgcctaa aggaaatggc tccagtgggt 960

ctaaacaacc acatcctatc catggatagg tctagtcata acactttaga gagaatgtca 1020

gagcaggagg gaggcaagcc gcctcttctc ggccatcaac tgcagatgat gaaagagcgg 1080

gattcaactt tgttttcttt tcctgtggcc ccagtgaaac ctcctgccct ccctgcacgt 1140

ctgtgtcttc atttctaaaa tgggggtgat gctttcatat tgacctcacc ccatactacc 1200

tcacagatgt gttgtgagga ttaataaaat tatgtctatg gtattttcag tttctggaga 1260
aaaatactta tagacagttt aactattaca tagatatata agtgatctca gtttcttgtt 1320
tgctgtgata ctaatgtgtt gttttaactt attccataaa atgacagttg tgtcctagcc 1380
acatcagaca gctatctaag ctctggacta cccctttgtg cagctgaatc actgcagggt 1440
cgaccatgcc tggtgccaca gccatggttt ccatttctag atgaaaggat ggcctaggac 1500
ataggtctca aagactcttg gatcagaatc aggagattag ggaaaacagg atggatacct 1560
gagcactaac agcagtagac gtagacctct gtcctttacc atctgaggtc ttctggattc 1620
tttgtggggt taattttgat ttgatgtcat ctgtttgccc ttcatcttgc ttgcaagtgt 1680
gcatggttca atccctcaca tccaggaaat gaattttgca attgggccag atgctaattt 1740
gcacgttgat tcaccttctt tgcctttaag cctttttttt cttttttttt tttttggca 1800
aatgaatgta ccatttcaac tttgatttta atagtgctag ttgatattgg taataatgct 1860
aaccaagaga tcaatgccag atttttctct tggggtaagt tagctgaagt catttaaaga 1920
tggaaaggtg ggaaaattct ttgatatttg atgtcattgt atccacattt gttgtaagac 1980
atattgcata ccaattataa ttatatcaat taaagttgat aaaagcttca aaaaaaaaaa 2040
aaaaa 2045

<210> 8
<211> 2096
<212> DNA
<213> human

<400> 8
atggagaacg agcctgtagc ccttgaggaa actcagaaga cagatcctgc tatggaacca 60
cggttcaaag tggtggattg ggacaaggac ctggtggact ggcgaaagcc tctcctgtgg 120
caggtgggcc acttgggaga gaagtacgat gagtgggttc accagccggt gaccaggccc 180
atccgcctct tccactcaga cctcattgag ggcctctcta agactgtctg gtacagtgtc 240
cccatcatct gggtgcccct ggtgctgtat ctcagctggt cctactaccg aacctttgcc 300
cagggcaacg tccgactctt cacgtcattt acaacagagt acacggtggc agtgcccaag 360
tccatgttcc ccgggctctt catgctgggg acattcctct ggagcctcat cgagtacctc 420
atccaccgct tcctgttcca catgaagccc cccagcgaca gctattacct catcatgctg 480
cacttcgtca tgcacggcca gcaccacaag gcacccttcg acggctcccg cctggtcttc 540
ccccctgtgc cagcctccct ggtgatcggc gtcttctact tgtgcatgca gctcatcctg 600
cccgaggcag tagggggcac tgtgtttgcg gggggcctcc tgggctacgt cctctatgac 660
atgacccatt actacctgca ctttggctcg ccgcacaagg gctcctacct gtacagcctg 720
aaggcccacc acgtcaagca ccactttgca catcagaagt caggatttgg tatcagcact 780
aaattgtggg attactgttt ccacaccctc actccagaga aaccccacct gaagacgcag 840
tgacaactcc cacccctcc gtcctgccct cagcccggcc ctggcccctt cccgaccccc 900

acccgccatt cagaccccat taagaaggtt ggcttggcca ggcaggatgg gctgtgtccg 960
gccctgcagc ctagtggaag gtgctgaggg ggccctgagg caggaccgcc ctcctgaccc 1020
ctggtaggag ggtcacatcc acttggtgca ggtggccctt ggtgacccac ttcttcctgg 1080
agcgtccctg cctagagctc agcccacagg actgcttcag gccgtggcca caggtagcag 1140
ccgcaagggg aaatgaagaa aactgagccc tcgtggccac ctgtgtcacc cttgtgcctt 1200
agcctcatgg gctgcctagg agctgcctgc acggcacagc tcgctttcac agtcagaagt 1260
gggtctgtgg gatctgtggt ccctgtcctc cctgctgtcc cttctgggga ggctttggtg 1320
gctctgaggt ggacaaagag ctctcgcaag aagagacagc gtgatgcctc ccacagtcca 1380
ccccagaccc tggggcagcc cctctggccc tgccagctgc ctgcgtcgtt gggcccaggg 1440
tggctggcag gagtcccagc tgcttgcttt aggacctggc agcttttctt gccgtccctc 1500
ccctgcctcc agaatcacag cccttctccc caagggaggc tgaggaggct tctccaccag 1560
tggcagcccc accccgtccc tggccattct tggcctccac cccgctcagg cccctactcg 1620
ggcgctccca gaaggagcca cctctcagtg cctcacctcc ccctgcctcc cagcctccgc 1680
agatgaggtt cctgcccctt cctcctcgta accaaaaccc tcactgctcc caggacggtc 1740
ttatttataa accagataca tgttcttagt ctggtcccag accaaggagc tggtcagacg 1800
gccctttcta atcctacatg ttgagcttat gtaaaaaatg ttgtttcctc ctgtttttgg 1860
ttcctttctt acccacaaac cattactact tgaaacttaa aaaactcgcc aagtgtaaag 1920
gctaaagaga agcagtttga cggaccttgt gatttgtact gtttgctgcg gagctattta 1980
aagattttgg aataaatata caaaactacg gttgtgaaat aaaaacttaa attgtatatt 2040
ttgaaaaata aaacactgaa aagaaaccaa caaaaaaaaa aaaaaaaaaa aaaaaa 2096

<210> 9
<211> 5640
<212> DNA
<213> human

<400> 9
ggaaacgcag aaaacagaga gaggcattct gagtcatctg actggatgaa gactgttcca 60
agttacaacc aaacaaatag ctccatggac tttagaaatt atatgatgag agatgagact 120
ctggaaccac tgcccaaaaa ctgggaaatg gcctacactg acacagggat gatctacttc 180
attgaccaca ataccaagac aaccacctgg ttggatcctc gtctttgtaa gaaagccaaa 240
gcccctgaag actgtgaaga tggagagctt ccttatggct gggagaaaat agaggaccct 300
cagtatggga catactatgt tgatcacctt aaccagaaaa cccagtttga aaatccagtg 360
gaggaagcca aaaggaaaaa gcagttagga caggttgaaa ttgggtcttc aaaaccagat 420
atggaaaaat cacacttcac aagagatcca tcccagctta aaggtgtcct tgttcgagca 480
tcactgaaaa aaagcacaat gggatttggt tttactatta ttggtggaga tagacctgat 540
gagttcctac aagtgaaaaa tgtgctgaaa gatggtcccg cagctcagga tgggaaaatt 600
gcaccaggcg atgttattgt agacatcaat ggcaactgtg tcctcggtca cactcatgca 660

gatgttgtcc agatgtttca attggtacct gtcaatcagt atgtaaacct cactttatgt 720
cgtggttatc cacttcctga tgacagtgaa gatcctgttg tggacattgt tgctgctacc 780
cctgtcatca atggacagtc attaaccaag ggagagactt gcatgaatcc tcaggatttt 840
aagccaggag caatggttct ggagcagaat ggaaaatcgg gacacacttt gactggtgat 900
ggtctcaatg gaccatcaga tgcaagtgag cagagagtat ccatggcatc gtcaggcagc 960
tcccagcctg aactagtgac tatccctttg attaagggcc ctaaagggtt tgggtttgca 1020
attgctgaca gccctactgg acagaaggtg aaaatgatac tggatagtca gtggtgtcaa 1080
ggccttcaga aaggagatat aattaaggaa atataccatc aaaatgtgca gaatttaaca 1140
catctccaag tggtagaggt gctaaagcag tttccagtag gtgctgatgt accattgctt 1200
atcttaagag gaggtcctcc ttcaccaacc aaaactgcca aaatgaaaac agataaaaag 1260
gaaaatgcag gaagtttgga ggccataaat gagcctattc ctcagcctat gccttttcca 1320
ccgagcatta tcaggtcagg atccccaaaa ttggatcctt ctgaggtcta cctgaaatct 1380
aagactttat atgaagataa accaccaaac accaaagatt tggatgtttt tcttcgaaaa 1440
caagagtcag ggtttggctt cagggtgcta ggaggagatg gacctgacca gtctatatat 1500
attggggcta ttattcccct gggagcagct gagaaagatg gtcggctccg cgcagctgat 1560
gaactaatgt gcattgatgg aattcctgtt aaagggaaat cacacaaaca agtcttggac 1620
ctcatgacaa ctgctgctcg aaatggccat gtgttactaa ctgtcagacg gaagatcttc 1680
tatggagaaa aacaacccga ggacgacagc tctcaggcct tcatttcaac acagaatgga 1740
tctccccgcc tgaaccgggc agaggtccca gccaggcctg caccccagga gccctatgat 1800
gttgtcttgc aacgaaaaga aaatgaagga tttggctttg tcatcctcac ctccaaaaac 1860
aaaccacctc caggagttat tcctcataaa attggccgag tcatagaagg aagtccggct 1920
gaccgctgtg gaaaactgaa agttggagat catatctctg cagtgaatgg gcagtccatt 1980
gttgaactgt ctcatgataa cattgttcag ctgatcaaag atgctggtgt caccgtcaca 2040
ctaacggtca ttgctgaaga agagcatcat ggtccaccat caggaacaaa ctcagccagg 2100
caaagcccag ccctgcagca caggcccatg ggacagtcac aggccaacca catacctggg 2160
gacagaagtg ccctagaagg tgaaattgga aaagatgtct ccacttctta cagacattct 2220
tggtcagacc acaagcacct tgcacagcct gacaccgcag taatttcagt tgtaggcagt 2280
cggcacaatc agaaccttgg ttgttatcca gtagagctgg agagaggccc ccggggcttt 2340
ggattcagcc tccgaggggg gaaggagtac aacatggggc tgttcatcct tcgtcttgct 2400
gaagatggtc ctgccatcaa agatggcaga attcatgttg gtgaccagat tgttgaaatc 2460
aatggggaac ctacacaagg aatcacacat actcgagcaa ttgagctcat tcaggctggt 2520
ggaaataaag ttcttcttct tttgaggcca ggaactggct tgatacctga ccatggtttg 2580
gctccttccg gtctgtgctc ctacgtgaaa cccgagcaac attaaggctt tcagggcttt 2640
tcttggtctt tccttaaaaa gacttggtga ttgggatatt aataatcctt cgtcttcaaa 2700

tgtgatttat gatgaacagt caccattacc cccatcttca cattttgctt ccatatttga 2760
agagtctcac gtgccagtaa ttgaagaatc tttgagagtt cagatatgtg aaaaggcaga 2820
agaattaaag gacattgtgc ctgaaaagaa aagcacttta aatgaaaatc agcctgagat 2880
aaagcatcag tctcttctcc agaaaaatgt gagtaagagg gatccaccca gcagtcatgg 2940
gcacagtaac aagaaaaatc tattaaaagt agaaaatggt gttacacgaa gaggtagatc 3000
ggttagtccc aaaaagccag ccagtcaaca ttcagaggaa catttggata agattcctag 3060
tcctctaaaa aataacccca aaagaagacc cagagatcaa tccctcagcc ccagcaaagg 3120
ggaaaataaa agttgtcagg tcagcaccag ggcaggctct ggacaagatc agtgcagaaa 3180
aagcagaggt cggtcggcca gcccaaaaaa gcagcaaaaa attgaaggaa gcaaagctcc 3240
atcaaatgct gaggccaaat tattagaggg taagagtcga agaatagcag gctatacggg 3300
cagtaatgct gagcagatcc cagatgggaa ggaaaaatca gacgtcatca ggaaagatgc 3360
aaagcagaat cagttggaaa aaagcagaac aaggtctcca gagaaaaaaa tcaaaagaat 3420
ggttgagaaa tctcttccat ccaaaatgac taataagact acaagtaaag aagtatctga 3480
aaatgaaaaa ggaaagaaag taaccacagg agaaacaagt tctagtaacg ataaaatagg 3540
agaaaatgtc cagctatcag aaaagaggct gaagcaagaa cctgaagaga aggtagtttc 3600
aaacaaaaca gaagatcaca aagggaaaga actagaggca gctgacaaaa acaaagagac 3660
tggaaggttc aaaccggaaa gcagttctcc agttaagaaa acactgataa ctccagggcc 3720
ctggaaggtt ccaagtggaa ataaagtcac aggcactatt ggtatggctg agaaacggca 3780
gtaaccttta gtataaaaca aagaaaaaca agttgtaatc ttttcttaca gcagcatttt 3840
tccagaaaaa gccttttttt ttttttcaga tattctgaaa cagataagta catgttaatg 3900
tgagcctcaa gttacctagg ctgcatgaag ggcctttagg attgctaaga accaactgtc 3960
cccctggccg gctgccctcc ctcgctctca ggaaggagct gcatccacat gctcatctga 4020
cccgccctgc tcaggctgcc cagctcgtct tcatgagtgt ctgaacaaat gacatatgtt 4080
gatattaaca atgtggtcac aactcacttt gtatttgtgc caagttatct actgtatcat 4140
gtctgttttt atcctttttg ttcagctgtt tccacagtaa tgaaaaagtt aggtttggct 4200
tggaagttga tgatctcaat agcatgttgc atgtttacag agagaaatat gtgagtcctt 4260
gcagaagaag agactgttaa ctcatcgtta aagatggccg ttgtctcttc taacagctac 4320
tgatgatgtc ccactttaaa aataaaaccc ccaaacatca ctactttaag gaaaaaaaaa 4380
atgtagtcca atattgatgc tttcttatgg ctttttattt taatttggct ggataagttg 4440
tttcaaataa ctgttaaaga tattacttac aattgaatgt ttgaaataag aaagtacttt 4500
aagcaataga gttcatctcc tgctgtgtta tccaacctcg atgtatactt acagcatctc 4560
aggtcaccct ttttatttca gttatttaat tatgaaacca taaagaagca tgtggaaata 4620
gtgtttattg ctctttgaag aaaaaccacc aactatttct ggatattttg gctgtaccta 4680
ctactaaagt cattagtctt taatacataa tacatatttg aaaagtaaac atattatata 4740

```
gattatgtga gggacttaat catgaaacca gtttcacagt ccaagtacca actcttctgg    4800
tagcaggtgc acaagcttgg gtgtttaaaa acaacctgtg tagggtatgc ccagcaaatg    4860
aggacaaatg tgtagacagt acttactgga tcttatttaa cttttagcta cattaactaa    4920
ctttcttatt taaaaacaag aaagggagac taaacatctg cttaacttgt acacattttc    4980
agaattcttt ttaaaagtct agttaaagat gtttcttaga agttggagac tgttaacaac    5040
ttccataaaa tagatccagg tttttcagtt ccctgaagca gcattcagta gcatctatat    5100
aaataaaggc accttctgag aataaaacta ttttatggag tgtgtgaaca cacttgttct    5160
gtcacctggg ttcatcttgt tgtgaagcac attaggtcca ggtccttccc tctgggagtc    5220
tgactgtgaa actctttaac ccaacaactc aattagcccc tgtagataag acatgcttcc    5280
cagagtgaga tttttgaaat cccctttca tccagaacta tatttaccca cctattgtaa    5340
ctattcaaat agagcaaaat taggaggctt gataaatact aagaatttag taccacagaa    5400
attatttatt attttccctg tagtccacaa ttagtgataa cgaatcctat ttttgttaac    5460
tgtgacataa ctttgatgtc atatgttgtc ctatgtggtt cttcctaagt aaactctgta    5520
ctgattatat actgacttag caatgtggcc ttggaatgct gagcaaaatg tggatgtact    5580
ggttgtaaat gtttatatat tgtacagtac ctttatatat acacttgagg ttctgattag    5640
```

<210> 10
<211> 457
<212> DNA
<213> human

<220>
<221> misc_feature
<222> (242)..(242)
<223> any kind of base

<220>
<221> misc_feature
<222> (369)..(369)
<223> any kind of base

<220>
<221> misc_feature
<222> (406)..(406)
<223> any kind of base

<220>
<221> misc_feature
<222> (457)..(457)
<223> any kind of base

<220>
<221> misc_feature
<222> (394)..(394)
<223> any kind of base

<400> 10

```
tcagtcactc tttcaccctg ccaaagcttc actgtcctac tgattgaatt gtatgtgaga      60
aataaaatgt catcatatta agccactggg atttgtatgt ttatctgtta tagcagcaag     120
tcttaattta cctaatacac acattgtgac agatgttctt aatgtcccac cccatattgt     180
tacatgtcca gctttgagga tccctggcat gtgggggtag gagtttctgg gcatgctgga     240
tncaattccc acttttaagg catctgtggc ctctgtggcc tctgtggcct tcactgttat     300
ggaagggatt tatctggggc accataggaa actttaccat ggcacagtgg acaacctagg     360
agggggtgng gaggaggggc cttcaggccc aacngggggg accagngttc gtggggttag     420
ggtggtttgg ggggttttcc ctcttacccg tgggggn                              457
```

```
<210>  11
<211>  1493
<212>  DNA
<213>  human

<400>  11
aatagggttg gcggctgcag cgggcggcaa acagcccgcc cggcaccacc atgctcgccc      60
tggaggctgc acagctcgac gggccacact tcagctgtct gtacccagat ggcgtcttct     120
atgacctgga cagctgcaag cattccagct accctgattc agagggggct cctgactccc     180
tgtgggactg gactgtggcc ccacctgtcc cagccacccc ctatgaagcc ttcgacccgg     240
cagcagccgc ttttagccac ccccaggctg cccagctctg ctacgaaccc cccacctaca     300
gccctgcagg gaacctcgaa ctggccccca gcctggaggc cccggggcct ggcctccccg     360
cataccccac ggagaacttc gctagccaga ccctggttcc cccggcatat gccccgtacc     420
ccagccctgt gctatcagag gaggaagact taccgttgga cagccctgcc ctggaggtct     480
cggacagcga gtcggatgag gccctcgtgg ctggccccga ggggaaggga tccgaggcag     540
ggactcgcaa gaagctgcgc ctgtaccagt tcctgctggg gctactgacg cgcggggaca     600
tgcgtgagtg cgtgtggtgg gtggagccag gcgccggcgt cttccagttc tcctccaagc     660
acaaggaact cctggcgcgc cgctggggcc agcagaaggg gaaccgcaag cgcatgacct     720
accagaagct ggcgcgcgcc ctccgaaact acgccaagac cggcgagatc cgcaaggtca     780
agcgcaagct cacctaccag ttcgacagcg cgctgctgcc tgcagtccgc cgggcctgag     840
cacacccgag gctcccacct gcggagccgc tgggggacct cacgtcccag ccaggatccc     900
cctggaagaa aaagggcgtc cccacactct aggtgatagg acttacgcat ccccaccttt     960
tggggtaagg ggagtgctgc cctgccataa tccccaagcc cagcccgggc ctgtctggga    1020
ttccccactt gtgcctgggg tccctctggg atttctttgt catgtacaga ctccctggga    1080
tcctcatgtt ttgggtgaca ggacctatgg accactatac tcggggaggc agggtagcag    1140
tgcttccaga gtcccaagag cttctctggg attttcttgt gatatctgat tccccagtga    1200
ggcctgggac ctttttaaga tcgctgtgtg tctgtaaacc ctgaatctca tctggggtgg    1260
gggccctgct ggcaaccctg agccctgtcc aaggttccct cttgtcagat ctgagatttc    1320
ctagttatgt ctggggccct ctgggagctg ttatcatctc agatctcttc gcccatctat    1380
```

ggctgtgttg tcacatctgt cccctcattt ttgagatccc ccaattctct ggaactattc 1440

tgctgcccct ttttatgtgt ctggagttcc ccaatcacat ctagggctcc tcc 1493

<210> 12
<211> 2292
<212> DNA
<213> human

<400> 12
ccatgggttc cccttcagcc tgtccataca gagtgtgcat tccctggcag gggctcctgc 60

tcacagcctc gcttttaacc ttctggaacc tgccaaacag tgcccagacc aatattgatg 120

tcgtgccgtt caatgtcgca gaagggaagg aggtccttct agtagtccat aatgagtccc 180

agaatcttta tggctacaac tggtacaaag gggaaagggt gcatgccaac tatcgaatta 240

taggatatgt aaaaaatata agtcaagaaa atgccccagg gcccgcacac aacggtcgag 300

agacaatata ccccaatgga accctgctga tccagaacgt tacccacaat gacgcaggat 360

tctataccct acacgttata aaagaaaatc ttgtgaatga agaagtaacc agacaattct 420

acgtattctc ggagccaccc aagccctcca tcaccagcaa caacttcaat ccggtggaga 480

acaaagatat tgtggtttta acctgtcaac ctgagactca gaacacaacc tacctgtggt 540

gggtaaacaa tcagagcctc ctggtcagtc ccaggctgct gctctccact gacaacagga 600

ccctcgttct actcagcgcc acaaagaatg acataggacc ctatgaatgt gaaatacaga 660

acccagtggg tgccagccgc agtgacccag tcaccctgaa tgtccgctat gagtcagtac 720

aagcaagttc acctgacctc tcagctggga ccgctgtcag catcatgatt ggagtactgg 780

ctgggatggc tctgatatag cagccttggt gtagtttctg catttcggga agagtgtttt 840

tattatccac ctgcagactg gactggattc ttctagctcc ttcaatccca ttttctcctg 900

tggcatcact aagtataaga cctgctctct tcctgaagac ctataagctg gaggtggaca 960

actcaatgta aatttcaagg aaaaaccctc atgcctgaga tgtgggccac tcagagctaa 1020

ccaaaatgtt caacaccata actagagaca ctcaaattgc caaccaggac aagaagttga 1080

tgacttcatg ctgtggacag tttttcccaa gatgtcccaa gcctcatcgt gacgaggctc 1140

ttatcccact ccatttttcc ctgctcatgc ctgcctcttt aatttggtaa gataatgctg 1200

taactagaat ttcacaatca gcgccttgtg caggcaattt gacagagtgt tggatgtgtc 1260

atgtcatcat gtcaaaccca aatatttgac ctaagggatc ctttattctg cccagtggct 1320

aactttaaca acatccctaa tacaactgtt tattcaaatg cacggtggtc cctgttagag 1380

ttagacctct agactcacct gttctcacgc cctgttttaa tttaacccag ctatgggatg 1440

ccagataaca gaattgctgc ctacgagctg aacagggagg agtttgtgca gttgctgaca 1500

cttcttgttg cacataaata aatacagtgg gtactataga gactcagttg caaaaattaa 1560

caaatatgct gcttgattaa aatgggtagg cttctcatgt ggctcattct ttaatctatt 1620

ctcttttatt tggtttggtt catggggtct ctgcctatgg atcatacttc aaactcttgg 1680

tgtgatcctc ctgattgtca caatattagt taccctggtg tgctgtattc tctaaaacct 1740

ttaaatgttt gcatgcagcc attcgtcaaa tgtcaaatat tctctctttg gctggaatga 1800

caaaaactca aataaatgta tgattaggag gacatcataa cctatgaatg atggaagtcc 1860

aaaatgatgg taactgacag tagtgttaat gccttatgtt tagtcaaact ctcatttagg 1920

tgacagcctg gtgactccag aatggagcca gtcatgctaa atgccatata ctcacactga 1980

aacatgagga agcaggtaga tcccagaaca gacaaaactt tcctaaaaac atgagagtcc 2040

aggctgtctg agtcagcaca gtaagaaagt cctttctgct ttaactctta gaaaaaagta 2100

atatgaagta ttctgaaatt aaccaatcag tttatttaaa tcaatttatt tatattcttc 2160

tgttcctgga ttcccatttt acaaaaccca ctgttctact gttgtattgc ccagtaggag 2220

ctatcactat attttgcaga atggaaactg ccctgactct tgaatcacaa ataaaagcca 2280

attgtatctg tt 2292

<210> 13
<211> 519
<212> DNA
<213> human

<220>
<221> misc_feature
<222> (212)..(212)
<223> any kind of base

<220>
<221> misc_feature
<222> (451)..(451)
<223> any kind of base

<400> 13
gaaacaacaa cagtgtaatc tttaacaggg atgttaaagg taagaagtca ggaagataaa 60

ccaaaatgat tgagtatgat aaagaatttt gcatggcgat taaaatagaa aacctataaa 120

tgtagaaaaa gcaggtctgg acttagcaaa gaaacaatat agtttggaga aggcatgaaa 180

taagttcttt tcatgttcac tgctggtcac ancataacag agagtgatgt ggagagcttt 240

gggaaggttt cacgttgagt tacatcagtg gtcaacaatg gagcaacaag actccgtaga 300

ggatgccacc ctgggagaat tgcaagggaa aggaggctga agcacaactg gtaatagcct 360

tcagatattt aatggatatg caaataaagc tctgattaat tgtattttca cttattatat 420

atcatctttg gacctttcta aaagtgggac nctagaaaag atatactgaa actccaaaag 480

aatacttcag ctcgagttga atggattcaa gatgttgtt 519

<210> 14
<211> 5294
<212> DNA
<213> human

<400> 14
ggctcgcatc cccatagtgc tgggttacag tgaaggtacg ccccgcgctc tgctctggag 60

aggcagggtg ggatagggaa cgtctcgagt ggcgcccgca gtcatggtgg tgttcgttgg 120
ccgccgcctc ccggcgctcc tagggctgtt taagaagaag ggctctgcca aggctgagaa 180
tgacaaacat ctaagtgtag ggcctggcca ggggccaggg tctgcagtgg atgagcacca 240
ggacaacgtc ttctttccca gtgggcgacc cccccacctg gaagagctgc acactcaggc 300
ccaggagggg ctccgctccc tacaacacca agagaaacag aaactgaaca agggtggctg 360
ggaccatgga gacacccaga gtatccagtc ctcccggacg gggccggatg aagacaacat 420
ctccttctgc agtcagacca catcctacgt ggctgagagc tccacagcag aggacgcgct 480
ctccatccgc tcggagatga tccagcgcaa aggctccacc ttccgacccc atgactcatt 540
tcccaaatct ggaaagtcag ggcggcgtcg gcgggagcgg cggagcactg tgctgggact 600
cccgcagcat gtgcagaagg agcttggcct gaggaatgag cgtgaggcac caggcacgcc 660
ccgggctcct ggtgcacggg atgccgtacg catccccaca gtggacggcc gcccccgagg 720
cacctcaggg atgggggccc gggtgtccct gcaggcgctg gaggcggagg cagaggctgg 780
cgctgagaca gaggccatgc tgcagcgcca cattgaccgt gtctaccggg atgacacctt 840
tgttggccgg tccacgggta cccgggcccc accattgacc cggcccatgt ccctagcagt 900
gcctggattg acaggagggg cagggcctgc agagcccctg agcccggcca tgtccatctc 960
cccccaggcc acctacctgt cgaagttgat tccacatgct gtgctgccgc ctacagtgga 1020
cgtggtggcc ctaggccgct gcagcctgcg cacactaagc cgctgcagcc tgcactcggc 1080
cagcccagcc tcagtccgct cgctggggcg cttctcctcc gtctccagcc cacagccccg 1140
cagccgccac ccatcctcct ccagtgacac ctggagccac tctcaatcct ccgacaccat 1200
tgtgtctgac ggttccaccc tctcctctaa gggtggctct gagggccagc cggagagctc 1260
tacggctagc aatagcgtgg taccccctcc ccagggaggc agtgggaggg gctctcccag 1320
tgggggcagc actgctgagg cctcagacac actcagcatt cggagcagtg ggcagttgtc 1380
tggccggagt gtgtccctgc gtaagctgaa gcggcctcca cccccctcccc gccggaccca 1440
ctccctccat cagcggggct tagcagtgcc tgatgggcca ttagggttgc cccctaagcc 1500
tgagcgtaag cagcagcccc agctgcctcg gccacccacc actggtggct cagaaggggc 1560
gggggcagca ccctgtccac ccaacccagc caacagctgg gtacctggct tgtctccggg 1620
tggttcccgg cgcccccac ggtccccaga acggacactt tcgccctcca gtggatactc 1680
gagccaaagt ggtactccca ccctccctcc caagggcctg gcaggtcccc ctgcttcccc 1740
aggcaaggcc cagcccccta aaccagagcg tgtcacgtct cttcgctccc ctggggcctc 1800
cgtctcctct tccctcacgt ctttatgttc ctcctcctct gacccagccc cctcagaccg 1860
ctctgggcca cagatattga cccccctggg tgacaggttt gtcatacctc ctcaccccaa 1920
ggtgcctgcc cccttctccc cacctccctc caagcccagg agccctaacc cagctgcccc 1980
tgctctagcc gcccctgctg tggttcctgg gcctgtttct accactgacg ccagtcctca 2040
gtcccctccc actccccaga caaccttgac tccactgcag gagtctcctg tcatctccaa 2100

agaccagtca cccccacctt ccccaccccc atcttatcat ccaccccac cacccactaa 2160
gaagccagag gtggttgtgg aggcaccatc tgcctcagag actgctgagg agcccctcca 2220
agatcccaac tggccccctc ccccaccccc tgcccctgag gagcaggacc tgtccatggc 2280
tgacttcccc ccaccagagg aggctttttt ctctgtggcc agccctgagc ctgcaggccc 2340
ttcaggctcc ccagagcttg tcagctcccc ggctgcttcg tcctcctcag ctactgcttt 2400
gcagattcag cccccgggta gcccagaccc tcctccagct ccgccagccc cagctcctgc 2460
tagttccgcc ccagggcatg tggccaagct ccctcagaag gaaccggtgg gctgtagcaa 2520
gggtggtggg cctcccaggg aggacgtagg tgcgcccctg gtcacgccct cgctcctgca 2580
gatggtgcgg ctgcgctccg tgggtgctcc aggaggggct cccaccccag cactggggcc 2640
atcggccccc cagaaaccac tgcgaagggc cctgtcaggg cgggccagcc cagtgcctgc 2700
cccctcctca gggctccatg ctgcggtccg actcaaggcc tgcagcctgg ccgccagtga 2760
aggcctctca agtgctcagc ccaacggacc gcctgaggca gagccacggc ctccccagtc 2820
ccctgcctca acggccagtt tcatcttctc caagggctct aggaagctgc agctggagcg 2880
gcccgtgtcc cctgagaccc aggctgacct ccagcggaat ctggtggcag aactccggag 2940
catctcagag cagcggccac cccaggcccc aaagaagtca cctaaggctc cccccacctgt 3000
ggcccgcaag ccgtctgtgg gagtcccccc acccgcctcc cccagttacc ctcgagctga 3060
gccccttact gctcctccca ccaatgggct ccctcacacc caggacagga ctaagaggga 3120
gctggcggag aatggaggtg tcctgcagct ggtgggccca gaggagaaga tgggcctccc 3180
gggctcagac tcacagaaag agctggcctg accaccaggc acctcactgg cactgctgac 3240
ccatcccaga aacacaatct cagggacccg agcagctcca aggacgagag gatacagcag 3300
acacaaccta atagagaggg cgcctgcagc cttaacctcc acggccttcg atacttatgc 3360
aagcctggtg ttgctcctgt cctcagagtc atcctgcgct catgcctttt cccgaatggg 3420
ttcacctctg gcagttgccg cttcagtctt ggccttagcc tcatcttgaa gtgggtagct 3480
ggcgggagag ggtggctgcg ccccctgctg gccctgaggc tgcagagttg ggagcaggac 3540
acctcacctg agtttcattt tttttcatgt ccaaaccatg cacatactat agtccagaat 3600
caaagcactt ttgaaaagtg gctgcatggc catcctccag ggcccaggaa gttgcattcc 3660
aagggcctgt ttacatggca gcagaatcca tccccggcag tcagcccata gcttgggacc 3720
agtctgtgcc ctcctgccca gtccagttta ctcctcttgg ttcctgaagg tggccaagtc 3780
attgtgttcc cacaggcttc tctaggctgg gggcaggtgt ggggctgtgg aattccaaag 3840
cacaaaaggt gcagaggggga ttggccttcc tgtgcctcaa ctcaccaacc accctcctgc 3900
cttccagttc tgccaggtgc tccatgctgg ggacaagtag gagactgcca gggcccaaag 3960
aaatgggtga gcagtagagt catctcgggg cacttggcag tgtcaagcac ctgccccttg 4020
cctccttgac cacactgggg tgggtgggcc cccagcactt cagaggcagg agcctttggg 4080
ctgagcaagc actgaggagg tggatggaag ggagcatctg gagggggga gcttccttga 4140

gcagtgggcc caggcctggc cctccacact tcattctctg acctttctct ctcctcattt 4200
cggtgcatgt cctttctgca gctgcctttc agcacaggtg gttccactgg gggcagctaa 4260
cgctgagtga caaggatggg aagccacagg tgcattttac tcaagtcttc tctagtcaat 4320
gaggggcacc cagtgcttct agggcaggct gggtggtggt cccctaggta tcagcctctc 4380
ttactgtact ctccgggaat gttaaccttt ctattttcag cctgtgccac ctgtctaggc 4440
aagctggctt ccccattggc ccctgtgggt ccacagcagc gtggctgccc cccagggcca 4500
ccgcttcttt cttgatcctc tttccttaac agtgacttgg gcttgagtct ggcaaggaac 4560
cttgctttta gcttcaccac caaggagaga ggttgacatg acctccccgc cccctcacca 4620
aggctgggaa cagaggggat gtggtgagag ccaggttcct ctggccctct ccagggtgtt 4680
ttccactagt cactactgtc ttctccttgt agctaatcaa tcaatattct tcccttgcct 4740
gtgggcagtg gagagtgctg ctgggtgtac gctgcacctg cccactgagt tggggaaaga 4800
ggataatcag tgagcactgt tctgctcaga gctcctgatc taccccaccc cctaggatcc 4860
aggactgggt caaagctgca tgaaaccagg ccctggcagc aacctgggaa tggctggagg 4920
tgggagagaa cctgacttct ctttccctct ccctcctcca acattactgg aactctatcc 4980
tgttaggatc ttctgagctt gtttccctgc tgggtgggac agaggacaaa ggagaaggga 5040
gggtctagaa gaggcagccc ttctttgtcc tctggggtaa atgagcttga cctagagtaa 5100
atggagagac caaaagcctc tgatttttaa tttccataaa atgttagaag tatatatata 5160
catatatata tttctttaaa tttttgagtc tttgatatgt ctaaaaatcc attccctctg 5220
ccctgaagcc tgagtgagac acatgaagaa aactgtgttt catttaaaga tgttaattaa 5280
atgattgaaa cttg 5294

<210> 15
<211> 988
<212> DNA
<213> human

<400> 15
gtcgtgaggc gggccttcgg gctggctcgc cgtcggctgc cgggggggttg gcctgggtgt 60
cattggctct gggaagcggc agcagaggca gggaccactc ggggtctggt gtcggcacag 120
ccatggcggg cgcgttggtg cggaaagcgg cggactatgt ccgaagcaag gatttccggg 180
actacctcat gagtacgcac ttctggggcc cagtagccaa ctggggtctt cccattgctg 240
ccatcaatga tatgaaaaag tctccagaga ttatcagtgg gcggatgaca tttgccctct 300
gttgctattc tttgacattc atgagatttg cctacaaggt acagcctcgg aactggcttc 360
tgtttgcatg ccacgcaaca aatgaagtag cccagctcat ccagggaggg cggcttatca 420
aacacgagat gactaaaacg gcatctgcat aacaatggga aaaggaagaa caaggtcttg 480
aagggacagc attgccagct gctgctgagt cacagatttc attataaata gcctccctaa 540
ggaaaataca ctgaatgcta tttttactaa ccattctatt tttatagaaa tagctgagag 600
tttctaaacc aactctctgc tgccttacaa gtattaaata ttttacttct ttccataaag 660

agtagctcaa aatatgcaat taatttaata atttctgatg atgttttatc tgcagtaata 720

tgtatatcat ctattagaat ttacttaatg aaaaactgaa gagaacaaaa tttgtaacca 780

ctagcactta agtactcctg attcttaaca ttgtctttaa tgaccacaag acaaccaaca 840

gctggccacg tacttaaaat tttgtcccca ctgtttaaaa atgttacctg tgtatttcca 900

tgcagtgtat atattgagat gctgtaactt aatggcaata aatgatttaa atatttgtta 960

aaaaaaaaaa aaaaaaaaaa aaaaaaaa 988

<210> 16
<211> 4908
<212> DNA
<213> human

<400> 16
ggataacctc gcagggtggg ccggagggcg ggcgccgccg ctgcctgtgc tgcggcgatg 60

gcccagtgtg tacaatcagt gcaggagcta atcccggact ccttcgtccc ctgtgtcgct 120

gcgctgtgca gcgacgaagc cgagcggctc actcgtctca atcacctcag cttcgcggag 180

ctgcttaagc ccttctcccg cctcacttcc gaggttcaca tgagagatcc taataatcaa 240

cttcacgtaa ttaaaaattt gaagatagca gtaagcaaca ttgtcaccca gccacctcag 300

cctggagcca tccggaagct tttgaatgat gttgtttctg gcagtcagcc tgcagaagga 360

ttagtagcta atgtgattac agcaggagat tatgacctta acatcagtgc cactactcca 420

tggtttgagt cttacagaga aacctttctt cagtcgatgc cagcatcgga tcatgaattt 480

ctgaaccact atttagcatg tatgttggta gcgtcatcta gtgaagctga acctgtggaa 540

cagttttcaa agttgtcaca agaacagcat cgaattcagc acaacagtga ttattcctac 600

cccaagtggt ttataccaaa tacacttaaa tactatgtac ttttacatga tgtaagtgca 660

ggagatgaac agagagctga atcaatttat gaagaaatga aacagaaata tggaactcag 720

ggttgctatt tacttaaaat taattctcga acatctaatc gagcatcaga tgaacagata 780

ccagatcctt ggagtcagta tctccagaaa aatagtattc aaaaccagga atcatatgaa 840

gatggccctt gtactataac ttcaaataag aattctgata ataacttgct ttcattggat 900

ggattagata acgaagtcaa agatggctta ccaaataact ttagagctca cccacttcag 960

ttggagcaat ccagtgaccc ttctaacagt attgatggcc cagatcatct aagatctgct 1020

tcatcgttac atgaaacaaa gaaaggaaat actggaataa ttcatggtgc atgtttaaca 1080

cttactgatc atgatagaat tcgacagttt atacaagagt tcacatttcg ggccttttg 1140

ccacatatag agaaaacaat taggcaatta aacgatcagc taatatcaag aaaaggtttg 1200

agtcgatctc tattttctgc aactaaaaaa tggtttagtg gcagtaaagt tccagaaaag 1260

agcattaatg acctgaaaaa tacatctggc ttgctgtatc ctccggaagc accagaactt 1320

caaatcagga aaatggctga cttatgtttt ttggtgcagc attatgattt ggcttacagt 1380

tgctatcata ctgcaaagaa agattttctt aatgatcaag caatgcttta tgcagctggt 1440

gccttggaaa tggcagcagt gtctgctttt cttcaaccag gagcacctag gccatatcct 1500
gctcattaca tggatacagc aattcagaca tacagagata tctgcaagaa tatggtgttg 1560
gctgaaagat gtgtgttgct tagtgctgaa cttttaaaaa gccaaagcaa atattcagag 1620
gctgcagctc tcctaatacg gttgaccagt gaggattctg atcttcgaag tgcacttctt 1680
ttggaacagg cagcacattg ctttataaac atgaaaagtc ccatggttag aaaatatgca 1740
tttcatatga tattggcagg ccatcgattt agtaaagcag ggcagaaaaa gcatgcttta 1800
cgctgttatt gtcaagccat gcaagtttac aaaggaaaag gctggtctct tgcagaggat 1860
cacattaatt tcactattgg gcgccagtcc tatactctta gacagctgga taatgctgtg 1920
tctgctttta ggcatattct aattaatgaa agtaaacaat ctgctgctca acaggggggct 1980
ttcctcagag aatatcttta tgtttacaag aatgtaagtc agctgtcacc agatggtcct 2040
ttgccacagc ttcctttacc gtatattaac agttcagcaa cacgggtttt ttttggccat 2100
gacagacgac cagcggatgg tgaaaaacaa gcagctactc atgtaagtct tgatcaagaa 2160
tatgattctg aatcctctca gcagtggcga gaacttgagg aacaagttgt ttctgtggtt 2220
aacaaaggag taattccatc caattttcat cccacacaat actgtttgaa cagttactca 2280
gataattcaa gatttccact tgcagttgta gaagaaccaa ttacagtgga agtggctttt 2340
agaaaccctt tgaaagttct acttttgttg actgatttgt cattgctttg gaagtttcat 2400
cctaaagatt tcagtggaaa ggataatgaa gaagttaaac aactagttac aagtgaacct 2460
gaaatgattg gagctgaagt tatttcagag ttcttaatta atggcgaaga atcaaaagtg 2520
gcaagactaa agctctttcc ccatcacata ggggagctgc atattctggg agttgtttat 2580
aatcttggca ctattcaggg ctctatgaca gtagatggca ttggtgctct tcccggatgt 2640
cacacaggaa aatattcctt gagtatgtca gtccgaggga agcaggattt agaaattcaa 2700
ggtcctcgac ttaacaacac aaaagaagag aaaacatctg ttaaatatgg ccctgatcga 2760
cgtttagatc ccataatcac agaagaaatg ccactgttgg aggtgttctt tatacatttt 2820
cctacagggc ttctctgtgg agaaatccga aaagcatatg tagaatttgt caatgtcagc 2880
aaatgtccac ttactggatt gaaggttgtt tctaaacgtc cagagttctt tactttcggt 2940
ggtaatactg ctgttctaac accactaagt ccctcagctt ctgagaattg tagtgcttac 3000
aagactgttg tgacagatgc tacctctgtg tgtacagcac tcatatcatc agcttcttct 3060
gtagactttg gcattggcac aggaagtcaa ccagaggtga ttcctgttcc ccttcctgac 3120
actgttcttc tacccggagc ctcagtgcag ctgccaatgt ggttacgtgg gcctgatgaa 3180
gaaggtgtcc atgaaattaa ctttttgttt tactatgaaa gtgtcaaaaa gcagccaaaa 3240
atacggcaca gaatattaag acacactgca attatttgta ccagtcggtc tttaaatgta 3300
cgggccactg tctgcagaag taattctctt gaaaatgaag aaggcagagg aggcaatatg 3360
ctagtctttg tggatgtgga aaataccaat actagtgaag caggcgttaa ggaattccac 3420
atagtgcaag tatcaagtag tagcaaacac tggaagttac agaaatctgt aaatctttct 3480

```
gaaaacaaag atgccaaact tgccagtagg gagaagggaa agttttgctt taaggcaata   3540
agatgtgaga aagaagaagc ggccacacag tcctctgaaa aatatacctt tgcagatatc   3600
atctttggaa atgaacagat aataagttca gcaagcccat gtgcagactt cttttatcga   3660
agtttatctt ctgaattgaa aaaaccacaa gctcacttgc ctgtgcatac agaaaaacag   3720
tcaacagagg atgctgtgag attgattcaa aaatgcagtg aggtagattt gaatattgtc   3780
atattatgga aggcatacgt tgtggaagac agtaaacagc ttattttgga aggtcaacat   3840
catgttattc ttcgcactat aggaaaagaa gccttttcat atcctcagaa acaggagcca   3900
ccagaaatgg aactattgaa atttttcagg ccagaaaaca ttacagtttc ctcaaggcca   3960
tcagtagagc agctttctag tctcattaaa acgagtcttc actacccaga atcatttaat   4020
catccatttc atcaaaaaag cctttgttta gtaccagtca ctcttttact ttccaattgt   4080
tctaaggctg atgtagatgt catagttgat cttcggcata aaacaacaag tccagaagca   4140
ctggaaatcc atggatcatt cacatggctt ggacaaacac agtataaact tcaacttaaa   4200
agccaggaga ttcacagtct gcagctgaaa gcatgctttg ttcatacagg tgtttataac   4260
cttggaactc ctagggtatt tgccaagtta tcggaccaag ttacagtgtt tgaaacaagt   4320
cagcagaatt ccatgcctgc cctgatcatc atcagtaatg tgtgacaact tggaaatttg   4380
tactgaaatc cacaataatc agtttttgct ggatgggttt tacagcagta tttgatatac   4440
ctaacttgtt atggaggttg attgatatct gatccctgca aaatactttg acttgtcatt   4500
ttgttgatga tgcaaagcac gttggactga gaatacttaa cattcttttt ctgtatttct   4560
ttaaaccctg agaataattt acatgctcat aatacaggat atcagcatat ttgtgcacct   4620
tattaagccc catcttaaga aaacacaaag tctaagtctg ctgttacaac ttgtcaatgg   4680
tatacgaata ttaggagatg attctgagaa aggaaaggcc ttgttggcag tactcctgtt   4740
aagccattag tctctaaatt ccagctttac tgtgaagttc tatagagtgt taaatacaaa   4800
ttttcctgtc ttgcttcaca cagttcctta aaatcagttt tgaactttgg tcatagagtc   4860
ttcatatttc agtatttggt ggtccctatg acttatacat aactttgt                4908
```

<210> 17
<211> 435
<212> DNA
<213> human

<220>
<221> misc_feature
<222> (30)..(30)
<223> any kind of base

<220>
<221> misc_feature
<222> (49)..(49)
<223> any kind of base

<220>
<221> misc_feature

<222> (75)..(75)
<223> any kind of base

<220>
<221> misc_feature
<222> (76)..(76)
<223> any kind of base

<220>
<221> misc_feature
<222> (78)..(78)
<223> any kind of base

<220>
<221> misc_feature
<222> (79)..(79)
<223> any kind of base

<220>
<221> misc_feature
<222> (109)..(109)
<223> any kind of base

<220>
<221> misc_feature
<222> (136)..(136)
<223> any kind of base

<220>
<221> misc_feature
<222> (137)..(137)
<223> any kind of base

<220>
<221> misc_feature
<222> (149)..(149)
<223> any kind of base

<220>
<221> misc_feature
<222> (227)..(227)
<223> any kind of base

<220>
<221> misc_feature
<222> (236)..(236)
<223> any kind of base

<220>
<221> misc_feature
<222> (246)..(246)
<223> any kind of base

<220>
<221> misc_feature
<222> (342)..(342)
<223> any kind of base

<220>
<221> misc_feature
<222> (363)..(363)
<223> any kind of base

<220>
<221> misc_feature
<222> (389)..(389)
<223> any kind of base

<220>
<221> misc_feature
<222> (426)..(426)
<223> any kind of base

<400> 17
ggtagaaatg attgtgatgt acaaattttn tattttgatc atacttaana agacagagca 60

gactcacatt cattnncnna atagtatcac tgtacacata gcgaatttnt ggcgctttta 120

gattgctctg aaaatnnctg aagagttgnc catagcagcc tggtaagcct tttcctttcc 180

cccaaagctc tcctgcccctt tgcagaaaga ctgttggtga caactgntgc taactnaata 240

gcatgnggtt gaacttcgcc aaaatccttc cacctcctcc catagggcaa caggggtgac 300

ttgggcttaa agggcattga gtaagcaagt aggttatcag anaacagagg gaagattcca 360

ttntagataa tttccaaata ttacaattng tggaactcag agttcaactg ctcagttcct 420

tcttcngctg accct 435

<210> 18
<211> 2224
<212> DNA
<213> human

<400> 18
ctttagatct gtgcagcctt tgcgtgccaa acttgtgaaa ttccttttac cttttttgga 60

gtacttgcta taaagccacc tgtcaacaaa cccccattat gtacagaata ggacctatcc 120

agtagccagg ccagtaggca gttggggaag gtgggaagga tccagcgagg cccctgagcc 180

tgcacctgga caggtgtacg tctgcaccca tcaccctcag caccaggcca ccctgcagtc 240

cacttactgt actgtgttgt ggaaggatat gctaagtgat gaaagttgcg agcagtctca 300

ctggtcgtgt aaactttttt tttttttgg aaattgaagc tgtagagtgc tgcccgaaat 360

ctctaggaag ttggtggcaa gggacagcac tcacactctt ctggtcatga tctctgatct 420

ccacctcaaa tgacaataaa aaactggtcc aacgaagaca ctgctcagca cttcagccat 480

caggactaat ccatcgatga ctggaaaaga ggctagcttt gaggaaaaca gcctgggctc 540

ttgggagcag agtccagtgg gtgtgaggct gacttgccga cggtcggcag gtaatggctc 600

tcagccggcg aggcggtccc acagctctcc tcccagggca gcctgaggag gaggaggccg 660

ggtgcctgtt tggtggcagc ttcagcctag ggatacctga agctgttgag caacaccttt 720

atgaaatgtt gccagagcag caacacttcc ctgtgggcac agccccggga aatccggtac 780

caagtgagca aggtggcagg acccacccaa gcctgatacg catctgggcc cgccgggctc 840

agcaggggag gctgctacgg ctgcccactt cccagcaccg tctgtcaggc ttgaacccct 900

ctgtgctgtt cccttcctgg ctaataggga gacccttcgc aggcacccac tgtttcaact 960

tgaccctccc accccctgct actctcctcc acacacccct ccgttccgct agcctaccct 1020

gtcagccttt caataaaagt tatgcacaaa tgtgaacacc tgagatggag ctgaacattt 1080

cttcactttg ttctttttct gaagtcaaac tcttatcaaa tgccctaaaa ttattaccac 1140

ccaagagaaa caggaaaaag gttacatgtt tttgtttact gagagtaaga tcacctgcat 1200

ctggaagacg ggctggtaaa ttggtttggc tacagaacag aaagaaaaca aaaacaaacc 1260

tcgtaaggga agtatcgcac tcagacacca ccacttccta gagccaaatg agcaatccca 1320

aactgcaagt gccgtaagtg ggcctgtgac gtcacaccgc ccggcccgag gtatcgcatg 1380

tgcgggggag gcccacacta cagctgtcct ctcgtctaga aggcaccacc tcgctttcat 1440

gtcccgtgtg ttttggaaaa gcagtatggt gtgtcatgtc tagcggcgaa cacttccctc 1500

cctctgtcct tgaggttgta atataaaaac tgtgtttctg tacgtgtggg tgggaattct 1560

ctgacggtgc tcgttcatag cacaagctta cgctgagttc tgaactgtcg ttcacagctg 1620

cgtgtctgca tggtgtcgca tctgttgtac ctttggggaa aatttgtatg taaatgtaca 1680

gaaataaaaa cgttgcccca ttaacagatt tcctctggaa tgtcttccct acctcacctg 1740

atggtatcca ccgaagggca tttcactacc attaatggtg agtaataaaa tcctccgtgt 1800

tcattcagac ctcactgcgt cactactttg aacgcctctg taagctgtgt cttcacccgc 1860

cccgaggtgg gtggagggag gcctctcact ctgcttcgag tcctggtctt aaaggtagtc 1920

agaggcagag gctggattaa acacacactg tttaccaagt gccactctca gaccacctga 1980

gagacggggg gccatcagta aaattaagag gaattttttt cccttgttcg tgtatgttct 2040

gctgatccgt ggcctgaagg ttcctagaga cgtcaagaaa tgaatatctt acactgtgat 2100

tctgtgagga aagactggta acccaaaact ctcttctcta atgtattttt taacgaaaat 2160

gacaatattt ctttaataaa gtatttatac caaaaaaaaa aaaaaaaaaa aaaaaaaaaa 2220

aaaa 2224

<210> 19
<211> 2244
<212> DNA
<213> human

<400> 19
gttgtttaaa agcaaggcat gcttgtggat gactctgtaa cagactaatt ggaattgttg 60

aagctgctcc ctggttccac tctggagagt aatctgggac atcttagtgt tttgttttgt 120

ttttttccct cctctttttt tggggggag tgtgtgtggg gtttgttttt tagtcttgtt 180

tttttaattc attaaccagt ggttagcctt aaggggagga ggacggattg attccacatt 240

ccacttccta gatctagttt agaaaacatg ttccccatct ggtgctctta ggaaggagta 300

```
tagtaaatgc ctcatttaat aacatactcc tttttgaaag ttgccttttc tctccaccct    360
tgagtagatc cagtatttga tgaaactcat gaaagtgggt ggagcccatc ttccccctcc    420
tcttttctag gacgcactat atgtgactgt gactttaagg acatttgttt gccatttgct    480
gatttttttg ggaagttaat ttctaacttc tttcactgat aaatgaagaa aagtattgca    540
cctttgaaat gcaccaaatg aattgagttt gtaattaaaa aaattttttt tccctttcag    600
tcattgtctt atatgcttag catagatttg cagctcagta gtatatgtgt tcctagaatg    660
cagctgaaga cctgttatgt agaggaaata cgaggggtgg tgctagaaga cagacatctg    720
tggaatgatt cacatcctct caagttagga ggatggaggc ctgcttcatt aagaagctgg    780
gggtagggtg ggggtgggga gaacacttaa caacatgggg accagtcagg ggaatcccct    840
tatttctgtt ttgcatatga ggaaccctag agcagccagg tgaggctctc tagtttaata    900
aaaatcatgg aaagactctt aatgcagact cttcttaagt gttaataggg atttttttcag    960
cttattttgg ttgcagtttc caatttttaa aaatgttgag gtaatctttc ccaccttccc   1020
aaacctaatt cttgtagatg cattagtgtt gaaccaatgc ttctcatgtc tcaatcttgt   1080
atatcatctt ttcagatgta ttaacaaaca aaaccttaaa aagagtagat gaattgccaa   1140
acacaattcc taccaataat aaatcgatca actctatcta ttcaggaaag caggaagcat   1200
ttggaccaca gtgcatgaaa acttcaacat tctgttatta gataatgaat caaccaaatg   1260
aacaatccag agaaaagaaa attgcaataa taaaaggtaa attaacagaa agataatata   1320
agcaagatag taatagttga ccattctgaa aagcttataa catcactcat catccagcat   1380
cctttctgaa aacaaaggat ttttaaatca ctttatgcac atatacaaca taggaggttg   1440
gcaaaataat gcactatttc ttaacagcca tgtctcttgt agaacttcaa gttaatctac   1500
aaatgaccat tgtgtcttaa tttagattat gaataccaca ttagtcaggt atttgcacta   1560
acccttaata gtatatacag tttctatgga aaattcagtg gtccaaaaat ttccgtagaa   1620
tttgagagga cgttggtggg ctgaagatag ctccttgagg gtcactgatg taggctgcaa   1680
tgggggttca caaggccctg acaccgtatt tatagtctaa ccttttttatg aaaatctgac   1740
tacagctatt taaggagtag tcttaatagc tgaaaatgaa gatagagaaa gacaccaaga   1800
atatgacaca gtttacattc tagtgaggga cacaacaaaa tcaaatttaa aaaagagtgt   1860
aatagatgct gataaatact gtagataaag cacataagaa aatagaaata aaggctgtca   1920
atggagaagt catgattttt attttattta tttatttatt tatttgagac agagtcaggc   1980
tctgtgcagg ctggagtgca atggtgtgat ctcgctcact acaacctctg ctcctggctc   2040
aagctatcct cccacctcag ctctcaagta gctgggatca caggtgcgtg ctaccatgcc   2100
cggctaattt tttgtagaga tgaggttttg ccatgttgcc caggctggtc tcgaactcct   2160
ggactcaact gaccccacct cggcctctca aagtgctgag attataggcg tgcagccggc   2220
agctggccat tgtttatgtt ctgc                                          2244
```

<210> 20
<211> 351
<212> DNA
<213> human

<220>
<221> misc_feature
<222> (62)..(62)
<223> any kind of base

<220>
<221> misc_feature
<222> (121)..(121)
<223> any kind of base

<220>
<221> misc_feature
<222> (207)..(207)
<223> any kind of base

<220>
<221> misc_feature
<222> (220)..(220)
<223> any kind of base

<220>
<221> misc_feature
<222> (276)..(276)
<223> any kind of base

<220>
<221> misc_feature
<222> (300)..(300)
<223> any kind of base

<220>
<221> misc_feature
<222> (315)..(315)
<223> any kind of base

<220>
<221> misc_feature
<222> (336)..(336)
<223> any kind of base

<400> 20
tctacttcca catcggcgag accgagaagc gctgtttcat cgaggaaatc cccgacgaga 60

cnatggtcat cggcaactat cgtacccaga tgtgggataa gcagaaggag gtcttcctgc 120

nctcgacccc tggcctgggc atgcacgtgg aagtgaagga ccccgacggc aaggtggtgc 180

tgtcctggca gtacggctcg gagggcnctt tcacgttcan ctcccacacg cccggtgacc 240

atcaaatctg tctgcactcc aattcttacc aggatngctc tctttcgctg gtgggcaaan 300

tgcgtgttgc atctngacat ccaggtttgg gggagnatgc caacaaatta c 351

<210> 21

<211> 2631
<212> DNA
<213> human

<400> 21
accttccaac ccagccctcg gctgagccgc gccgcaccat gcccgccgtg gacaagctcc 60

tgctagagga ggcgttgcag gacagccccc agactcgctc tttactgagc gtgtttgaag 120

aagatgctgg caccctcaca gactatacca accagctgct ccaggcaatg cagcgcgtct 180

atggagccca gaatgagatg tgcctggcca cacaacagct ttctaagcaa ctgctggcat 240

atgaaaaaca gaactttgct cttggcaaag gtgatgaaga agtaatttca acactccact 300

atttttccaa agtggtggat gagcttaatc ttctccatac agagctggct aaacagttgg 360

cagacacaat ggttctacct atcatacaat tccgagaaaa ggatctcaca gaagtaagca 420

ctttaaagga tctatttgga ctcgctagca atgagcatga cctctcaatg gcaaaataca 480

gcaggctgcc taagaaaaag gagaatgaga aggtgaagac cgaagtcgga aaagaggtgg 540

ccgcggcccg gcggaagcag cacctctcct cccttcagta ctactgtgcc ctcaacgcgc 600

tgcagtacag aaagcaaatg gccatgatgg agcccatgat aggctttgcc catggacaga 660

ttaacttttt taagaaggga gcagagatgt tttccaaacg tatggacagc tttttatcct 720

ccgttgcaga catggttcaa agcattcagg tagaactgga accgaggcgg aaaagatgcg 780

ggtgtcccag caagaattac tttctgttga tgaatctgtt tacactccag actctgatgt 840

ggccgcacca cagatcaaca ggaacctcat ccagaaggct ggttacctta atcttagaaa 900

caaaacaggg ctggtcaccg ccacctggga gaggctttat ttcttcaccc aaggcgggaa 960

tctcatgtgt cagcccaggg gagccgtggc tggaggtttg atccaggacc tggacaactg 1020

ctcagtgatg gccgtggatt gcgaagaccg gcgctactgc tttcagatca ccacgcccaa 1080

tggaaaatcg ggaataatcc tccaggctga gagcagaaag gaaaatgaag agtggatatg 1140

tgcaataaac aacatctcca gacagatcta cctgaccgac aaccctgagg cagtcgcgat 1200

caagttgaat cagaccgctc tgcaagcagt gactcctatt acaagttttg gaaaaaaaca 1260

agaaagctca tgccccagcc agaacctgaa aaattcagag atggaaaatg aaaatgacaa 1320

gattgttccc aaagcaacag ccagtctacc tgaagcagag gagctgatcg cgcctggagc 1380

gccgattcaa ttcgatattg tgcttcctgc tacagaattc cttgatcaga acagagggag 1440

caggcgtacc aaccctttg gtgaaactga ggatgaatca tttccagaag cagaagattc 1500

tcttttgcag cagatgttta tagttcggtt tttgggatca atggcagtta aaacagacag 1560

cactactgaa gtgatttatg aagcgatgag acaagtattg gctgctcggg ctattcataa 1620

catcttccgc atgacagaat cccatctgat ggtcaccagc caatctttga ggttgataga 1680

tccacagact caagtatcaa gggccaattt tgaacttacc agtgtcacac aatttgctgc 1740

tcatcaagaa aacaagagac tggttggttt tgtcatccgt gttcctgaat ccactggaga 1800

agaatctctg agtacataca tttttgaaag caactcagaa ggcgaaaaga tatgttatgc 1860

tattaatttg ggaaaagaaa ttattgaggt tcagaaggat ccagaagcac tggctcaatt 1920

aatgctgtcc ataccactaa ccaatgatgg aaaatatgta ctgttaaacg atcaaccaga 1980
tgacgatgat ggaaatccaa atgaacatag aggcgcagaa tccgaagcat aactcacttg 2040
cgcctgtggg ggaagagcga acaggaagga gagctacctc ctaagggttt taacgtctct 2100
gacatacagg cacactgacc tgatttccga aggctgacaa tcgtttgtgg aatgtaatct 2160
tgatgccttg atactgagac ttgggaggga aactaagaaa tggttgacag cgttcccacc 2220
catctacaat gttattttag gtgctttgtg gtaagtcttt tttcttagat tgcgctaaaa 2280
tttcttagat tgttcagcgc tcagaacaaa agtttgaaaa atgcattgtt catatgaatg 2340
tcatctcttt tcagtttcca gtatcctttt taaaaaatgg caaaagccta gatttacaat 2400
ttgatgaaca ctaaatattt cttattaata taatctattt ttgtatttta cttaatgagc 2460
tttaagtgcc tgtcgttctg aaaattgtgt atttataatt cagcttatct cataattgga 2520
cctaatagca tttctttgtg cagttaggtg atgagcactg ctttgaggcc caagcactag 2580
tagagatgcg cgatacaggt ctagtttcgg taactgttcc agacatcaag c 2631

<210> 22
<211> 2851
<212> DNA
<213> human

<400> 22
agcatctcag gccatcatcc tgaaacttgg cagccttcgt ggagtataag gacagcatta 60
ttagccatca ttgggtttac tgccaacaaa aggagaggga gccataggtt ctctagatta 120
cactcctgag gaaagaagag cacttgccaa aaaatcacaa gatttctgtt gtgaaggatg 180
tggctctgcc atgaaggatg tcctgttgcc tttaaaatct ggaagcgatt caagccaagc 240
tgaccaagaa gccaaagaac tggctaggca aataagcttt aaggcagaag tcaattcatc 300
tggaaagact atctctgagt cagacttaaa ccactctttt tcactaactg atttacaaga 360
tgatatacct acaacattcc agggtgctac ggccagtaca tcgtacggac tccagaattc 420
ctcagcagca tcctttcatc aacctaccca acctgtagct aagaatacct ccatgagccc 480
tcgacagcgc cgggcccagc agcagagtca gagaaggttg tctacttcac cagatgtaat 540
ccagggccac cagccaagag acaaccacac tgatcatggt gggtcagctg tactgattgt 600
catcctgact ttggcattgg cagctcttat attccgacga atatatctgg caaacgaata 660
catatttgac tttgagttat aatatggttt tgtgacttat gagctgtgac tcaactgctt 720
cattaaacat tctgcattgg gtataatcta agaattgttt acaaaaagat tattttgtat 780
ttacccttca ttcctttttt tgatccttgt aagtttagta taaatatatc tagacattca 840
gactgtgtct agcagttacg tcctgcttaa agggactaga agtcaaagtt ccttgtctca 900
ctatttgatc tgctttgcag ggaaataact tgtttttttct catgtttcat cttcttttta 960
tgtaaatttg taatactttc ctatattgcc ctttgaaatt tttggataaa agatgatgtt 1020
ttaagttcca atgagtatta ctagttactc aataccactt attgagtact ctgtttctac 1080

gtatgtagaa tgtataggga tagaagagtt gaaaagggaa agcaaaactt cttaagtggc 1140
ttccttaaaa tgtcattcat aggagatgta ctggaattgc tcattctgtg actttatttg 1200
tgtcctaaac attcttcagt gaaaataatt ttatttcagt caaacattta tgaggaaatg 1260
agatcacatc tttgtcactg gatgctactt gaagagggag tactttgtaa ccactttgat 1320
atgctgttat caccaccccc tgccctctgc tgccataatc acacaaattt aaaaagaaag 1380
aaaacagtct tccatagatt tttaaggaag aaagggccca agtcaggaga tcgcttggtt 1440
ttcttccaga agttaaatgg ggggatctga agatttgaat gttcggtctg ctttgaaatg 1500
tatgtctttt gggaatgtat tatatgccta gctttataat caggtataaa attttaatta 1560
ttcccaggaa tatgcataat attgaatatt tcatgtccta ttttaataga aaacctcagg 1620
gcccaagtaa ccagtgatag aagttagaaa aaccccttta cttagaattg tccacctagt 1680
cagagcccaa gaaagaattt tcagtggaaa aatcaatata taacttagtg ctagctagcg 1740
ccacagactc tagtagataa tattatcatc ataatggctg gtgaaaccat ataatcacag 1800
aaaaacattg ccttcagcat gttcagttcg cagcactgag ggcactcttg agggtgttgt 1860
taatgaagat ttaattttta aatacaggtg gttccaagct ttcaaatagg ttatgctcca 1920
aaagtgttat ttgtaagtta atttttttac aagtcaaaca atgttggaag tggtatttag 1980
gttctagatc ggtccacgaa agttagccca tatgtatatc ttgaatagta taggggaggg 2040
tattcataaa gtccttatgt ggttttaact aagtgaaatt atggacaaga gaaataattg 2100
taaaatcgtc ttaaaggcaa atttaatttt taccccctgtt tatgggacat tcgttctatt 2160
aactgtcaga cacaatttct gttttcatct gagagccagg tttcctttat ttctacatct 2220
aaaataagaa catattgtac actattatat aatacagaat tgtcttacac tttaataaat 2280
tcgcatttta aaggtgttta caggattatt ttttatatct gtagctgaat ttgttaaagt 2340
ctaaaaagct caaggacttt atgaagatct cattatatga ggaaaatcat aggttaccat 2400
tttataactc tattgccata agaaaataca ctctaaaatc ttgatttgaa acatattaga 2460
aaccttgatt cagtgctcag tggtctccta gtaagaagtc accgacggta gcgtcatatg 2520
agaagaaaga aatccccacc acctcaacct ctgctgagat tgtgtgctag gaacagcctt 2580
ccctccgttt cccctcagtc aaacttgagc cagcctctgg atcgatgtga tcttattgca 2640
tgtttccatg ggtgtacct atactttaag ccaatcctgc tgcattcact gctaagttaa 2700
ataaaaagcc aagaagaaaa aaaaaatttt gcactgtgca gatcctttgc tatctgactt 2760
gcatctcttc ccccacctgt cagctagcca cctgcttgtt tgtgttggga tattttttag 2820
cacctgaagc accatctgaa aggggcacca t 2851

<210> 23
<211> 3473
<212> DNA
<213> human

<400> 23
aagagcagcg gcgaggcggc ggtggtggct gagtccgtgg tggcagaggc gaaggcgaca 60

```
gctctagggg ttggcaccgg ccccgagagg aggatgcggg tccggatagg gctgacgctg     120
ctgctgtgtg cggtgctgct gagcttggcc tcggcgtcct cggatgaaga aggcagccag     180
gatgaatcct tagattccaa gactactttg acatcagatg agtcagtaaa ggaccacact     240
actgcaggca gagtagttgc tggtcaaata tttcttgatt cagaagaatc tgaattagaa     300
tcctctattc aagaagagga agacagcctc aagagccaag agggggagag tgtcacagaa     360
gatatcagct ttctagagtc tccaaatcca gaaaacaagg actatgaaga gccaaagaaa     420
gtacggaaac cagctttgac cgccattgaa ggcacagcac atggggagcc ctgccacttc     480
ccttttcttt tcctagataa ggagtatgat gaatgtacat cagatgggag ggaagatggc     540
agactgtggt gtgctacaac ctatgactac aaagcagatg aaaagtgggg cttttgtgaa     600
actgaagaag aggctgctaa gagacggcag atgcaggaag cagaaatggt gtatcaaact     660
ggaatgaaaa tccttaatgg aagcaataag aaaagccaaa aaagagaagc atatcggtat     720
ctccaaaagg cagcaagcat gaaccatacc aaagccctgg agagagtgtc atatgctctt     780
ttatttggtg attacttgcc acagaatatc caggcagcga gagagatgtt tgagaagctg     840
actgaggaag gctctcccaa gggacagact gctcttggct ttctgtatgc ctctggactt     900
ggtgttaatt caagtcaggc aaaggctctt gtatattata catttggagc tcttgggggc     960
aatctaatag cccacatggt tttgggttac agatactggg ctggcatcgg cgtcctccag    1020
agttgtgaat ctgccctgac tcactatcgt cttgttgcca atcatgttgc tagtgatatc    1080
tcgctaacag gaggctcagt agtacagaga atacggctgc ctgatgaagt ggaaaatcca    1140
ggaatgaaca gtggaatgct agaagaagat ttgattcaat attaccagtt cctagctgaa    1200
aaaggtgatg tacaagcaca ggttggtctt ggacaactgc acctgcacgg agggcgtgga    1260
gtagaacaga atcatcagag agcatttgac tacttcaatt tagcagcaaa tgctggcaat    1320
tcacatgcca tggccttttt gggaaagatg tattcggaag gaagtgacat tgtacctcag    1380
agtaatgaga cagctctcca ctactttaag aaagctgctg acatgggcaa cccagttgga    1440
cagagtgggc ttggaatggc ctacctctat gggagaggag ttcaagttaa ttatgatcta    1500
gcccttaagt atttccagaa agctgctgaa caaggctggg tggatgggca gctacagctt    1560
ggttccatgt actataatgg cattggagtc aagagagatt ataaacaggc cttgaagtat    1620
tttaatttag cttctcaggg aggccatatc ttggctttct ataacctagc tcagatgcat    1680
gccagtggca ccggcgtgat gcgatcatgt cacactgcag tggagttgtt taagaatgta    1740
tgtgaacgag gccgttggtc tgaaaggctt atgactgcct ataacagcta taaagatggc    1800
gattacaatg ctgcagtgat ccagtacctc ctcctggctg aacagggcta tgaagtggca    1860
caaagcaatg cagcctttat tcttgatcag agagaagcaa gcattgtagg tgagaatgaa    1920
acttatccca gagctttgct acattggaac agggccgcct ctcaaggcta tactgtggct    1980
agaattaagc tcggagacta ccatttctat gggtttggca ccgatgtaga ttatgaaact    2040
gcatttattc attaccgtct ggcttctgag cagcaacaca gtgcacaagc tatgtttaat    2100
```

ctgggatata tgcatgagaa aggactgggc attaaacagg atattcacct tgcgaaacgt 2160
ttttatgaca tggcagctga agccagccca gatgcacaag ttccagtctt cctagccctc 2220
tgcaaattgg gcgtcgtcta tttcttgcag tacatacggg aaacaaacat tcgagatatg 2280
ttcacccaac ttgatatgga ccagcttttg ggacctgagt gggaccttta cctcatgacc 2340
atcattgcgc tgctgttggg aacagtcata gcttacaggc aaaggcagca ccaagacatg 2400
cctgcaccca ggcctccagg gccacggcca gctccacccc agcaggaggg gccaccagag 2460
cagcagccac cacagtaata ggcactgggt ccagccttga tcagtgacag cgaaggaagt 2520
tatctgctgg gaacacttgc atttgattta ggaccttgga tcagtggtca cctcccagaa 2580
gaggcacggc acaaggaagc attgaattcc taaagctgct tagaatctga tgcctttatt 2640
ttcagggata agtaactctt acctaaactg agctgaatgt ttgtttcagt gccatatgga 2700
ataacaactt tcagtggctt tttttttct tttctggaaa catatgtgag acactcagag 2760
taatgtctac tgtatccagc tatctttctt ggatcctttt ggtcattatt tcagtgtgca 2820
taagttctta atgtcaacca tctttaaggt attgtgcatc gacactaaaa actgatcagt 2880
gtaaaaagga aaacccagtt gcaagtttaa acgtgttcga aagtctgaaa atagaacttg 2940
ccttttaagt taaaaaaaaa aaaagctatc ttgaaaatgt tttggaactg cgataactga 3000
gaaactctta ccagtccaca tgcaattaga catattcagc atatttgtta ttttaaaagg 3060
gagggttggg aggtttctta ttggtgattg tcacacggta taccatactc ctctccttca 3120
aagaatgaaa ggccttgtta aggagttttt tgtgagcttt acttctttgg aatggaatat 3180
acttatgcaa aaccttgtga actgactcct tgcactaacg cgagtttgcc ccacctactc 3240
tgtaatttgc ttgtttgttt tgaatataca gagccttgat ccagaagcca gaggatggac 3300
taagtgggag aaattagaaa acaaaacgaa ctctggttgg ggtactacga tcacagacac 3360
agacatactt ttcctaaagt tgaagcattt gttcccagga tttattttac tttgcatttc 3420
cttttgcaca aagaacacat caccatttcc ttttgcacaa agaacacatc acc 3473

<210> 24
<211> 401
<212> DNA
<213> human

<220>
<221> misc_feature
<222> (252)..(252)
<223> any kind of base

<220>
<221> misc_feature
<222> (303)..(303)
<223> any kind of base

<220>
<221> misc_feature
<222> (390)..(390)

<223> any kind of base

<400> 24
ttagattatt ttcaatttat tattcagaat aaatatatct tttttcttta acttctcaaa 60

tagttattga attgtattgg tttaaattaa atgcgtcatg tgtatatatc agtattaatt 120

caagagatac aaaaggaaat tgagtgaaaa ataagtctgc ctccttccca tcactctcat 180

gtctctacct agaggcaatt attgtcaaca gtttttgatg tgtctttcaa aaaatagtcc 240

attaagcctg gngtactaga tctcttttaa aagtttacaa cctgttacag aatatatata 300

aangttcaat tactagtaac accttattac agatacagat tacaacttag gaaatatatt 360

ttcatggacc attgatgtca tttggattcn cccctacaat c 401

<210> 25
<211> 1820
<212> DNA
<213> human

<400> 25
aatgtcttag aaaaaggctt tctaaaagaa aaagagcaag aggccatttc ttttcaagat 60

agatacaaag aacttcagga aaaacataaa caagaattgg aagacatgag gaaagctggt 120

cacgaagccc tcagcattat tgtggatgaa tataaggcac tactgcagtc ttcagttaag 180

caacaagtag aagctattga aaaacagtac atttctgcaa ttgagaaaca ggcacacaag 240

tgtgaggagt tgctaaatgc tcagcatcag aggctccttg aaatgctaga tacagagaag 300

gaactgttaa aagaaaaaat aaaggaagct ttgattcagc aatctcaaga acagaaggaa 360

atattggaaa agtgtttgga ggaagaaagg caaagaaata aagaggcatt agtatccgct 420

gcaaagcttg aaaaagaagc agtgaaggat gcagttttaa aagtcgtaga agaagaaaga 480

aaaaatttag aaaaagcgca tgctgaagaa agggaattat ggaagacaga acatgcaaaa 540

gatcaagaaa aagtatctca ggaaattcaa aaagctatac aagaacaaag aaaaataagt 600

caggaaactg ttaaggcagc aataatagaa gagcagaaac gaagtgaaaa ggctgtggaa 660

gaggcagtga aagaacaaag agatgaattg atagagtata taaaagaaca gaaaaggctc 720

gatcaagtca tccgccaaag aagcctgtcc agtttggaac tgttcctctc ctgtgcacag 780

aaacagttaa gtgctttaat agctacggaa ccagttgaca ttgaataaaa agaacatgac 840

aaacccacac tggcattgga taaatcatat tacaccttca aaatacacac tctgaattat 900

aaagatgtgt ttgttttctt tccaaatcat gtagaattga tttccagttc aaggataaac 960

caaaacaata tttagaacta tcaagtgatc taatttattt tcttttggtt tcttctttac 1020

atttactgtt attttattat tattagtagt agcagcaaca gagtatgata tgacccaaaa 1080

gccattgtaa agtgccacat taccaaaatt aattaagtaa actttatagc ctgtgggagt 1140

ctattatata ttattttgca aaagtagtaa atatattatt gtttcatgat gactcttgat 1200

gagatgctag aatgtaacca tacatttatc ttattttgag gatagaaata gcatggattt 1260

caacatcact tatttatctg tataattgga aataaaacac cgatatgata gagaatcatt 1320

ccggcattac ctaacctctt ctgcagttgg atctatgtat tttcattggt ctactgaaaa 1380

cgaacaatac aattaaaagc actaaagatt attatattaa ttcaactttg atctgatata 1440

tcacttaaac taaaggggtg tgtgtggtgt atgcttgttt cctatttctg ctctttaaag 1500

atactttgaa tcaataaaac cattagtcta caaatcaaat tgtgaactta atctctagaa 1560

agagaatata actcagccat ttataggaat ttaggttcaa gtacaggata tatgaaatct 1620

tttcccagta tttcagaatg tacttaattc acaggcagga tgcttcaatg caaaatcatg 1680

aatattttta attcaaaact aaaatgtcat taatatgtat gtatgcaaat gttttatctt 1740

attttctgaa atgcatctac tttcatgggc tttgtacgtt tctgagattt ctcagtgtaa 1800

taaaaagagc tcccaaactt 1820

<210> 26
<211> 280
<212> DNA
<213> human

<220>
<221> misc_feature
<222> (237)..(237)
<223> any kind of base

<220>
<221> misc_feature
<222> (261)..(261)
<223> any kind of base

<400> 26
tcaagtcata agataaagtt taatcatttg atcatgttaa aagacacaaa acacagccaa 60

tctaaccaaa tttcaggcat gcatttacat aaatatatta aattaagaaa agaaattgta 120

cacttaaacg tccttttcac ctagaaatca ttaaatccac agatcaacaa taaaaccaat 180

tctctgcatt taccacttca agatacaatt gttctatttt aaagataaca caaactncac 240

tagtctggtt aggaatttat ntgcattata catatattat 280

<210> 27
<211> 392
<212> DNA
<213> human

<400> 27
ttggtttgaa atggcacccc aggactttgg gcctgcctta cttgatagcc tcgttcagtg 60

agcaaagact tagtgagcag ctcttgtatg ccaagtattt tgctaagctc tggaaaaaag 120

ataaacaaga catggttctt gctttcaagg agtgtgtaat tctttagcca gatatggaaa 180

cctggaccct gagtgggaga aaggagacag atgaaaggag tccgtgattt tgtaaccaag 240

agctgcctgc atggttatga gtatcactga ttttagggac gcccacagag ctaaagcatt 300

tttttaatcc gagaagactt ttgtaactca tattagttaa tcttctagct ctgagatagc 360

aacacagctc ttagaattct gtaagtaagc tt 392

<210> 28
<211> 2299
<212> DNA
<213> human

<400> 28
cgaaccccca cagctggagg gcgaggccag ctgtacccgg ccccagtgcc ctttcgcggc 60
cacaagcggc cgtcctcctg gtccggtgct ccggcgcctg atctaggttc atggagccgg 120
ggctgtggct ccttttcggg ctcacagtga cctccgccgc aggattcgtg ccttgctccc 180
agtctgggga tgctggcagg cgcggcgtgt cccaggcccc cactgcagcc agatctgagg 240
gggactgtga agagactgtg gctggccctg gcgaggagac tgtggctggc cctggcgagg 300
ggactgtggc cccgacagca ctgcagggtc caagccctgg aagccctggg caggagcagg 360
cggccgaggg ggcccctgag caccaccgat ccaggcgctg cacgtgcttc acctacaagg 420
acaaggagtg tgtctactat tgccacctgg acatcatttg gatcaacact cccgaacaga 480
cggtgcccta tggactgtcc aactacagag gaagcttccg ggcaagaggg tctgcggggc 540
cacttccagg gaatctgcag ctctcacatc ggccacactt gcgctgcgct tgtgtgggga 600
gatatgacaa ggcctgcctg cacttttgca cccaaactct ggacgtcagc agacaggttg 660
aagtcaagga ccaacaaagc aagcaggctt tagacctcca ccatccaaag ctcatgcccg 720
gcagtggact cgccctcgct ccatctacct gcccccgctg cctctttcag gaaggagccc 780
cttaggagga caggcctgca gcatcctggt ctcgggaggc ttctgtcatt gctcacacac 840
agttcagatt tccacctctt tatagacaag aagtgaattt gcctggggca gaacacccac 900
ccaaagagtc cccacttaac aatacccccc ccccacggca agaatgccca aatccgaatg 960
accccagttt tcctaatgag taaaatgatc ccagatgtgc cccagagcat gacgcctgca 1020
gctccggttt catgcaggaa attggttttg gagagttttg gcaagttgga aagccactta 1080
ctggcttttg acatgacttc tcttggagaa taagtggact ccaagctaac tctttgcaaa 1140
tgtaaacaca tgtccatctt gtaataaatg caaaatgccc gtgcagcaga agcatgcgac 1200
tttcatatcc ttgcctagaa taggctgcat ggtgtatgtc agtgagggcc acgaggcgtc 1260
ggctttagac acagatcata gctctacagg agtttatgaa tttgaagctt atgggatttt 1320
ggcagagaaa ttttcagctg tgcttgatac ccaccaaaag aatgtatctc gaaagaatga 1380
aggaagaaga aaaaaggatc cttgatgttt gtgacaagaa aatgagaaag ttagtatctg 1440
caatacagag cttgttcctg ttcagtgact gaccctctgt attctgtata gacaccaggc 1500
cgatacacag tggagttccc aggccttgtt tgcaggaagc cgactgtaaa gacagcccca 1560
gctcaaggct attaggttga atatttgctt tcatgagtaa atgtggatct ttggggaatg 1620
gcttcaaaat aagtcacgaa cacaaattct ttgtaaatta tgtaaattcc tgtttatata 1680
aattggcaac aacttatacc gtctgacagt tcaaaatctc tttcagctgc gctcttccca 1740
ccgagccgag cttactgtga gtgtggagat gttatcccac catgtaaagt cgcctgcgca 1800

ggggagggct gcccatctcc ccaacccagt cacagagaga taggaaacgg catttgagtg 1860
ggtgtccagg gccccgtaga gagacattta agatggtgta tgacagagca ttggccttga 1920
ccaaatgtta aatcctctgt gtgtatttca taagttatta caggtataaa agtgatgacc 1980
tatcatgagg aaatgaaagt ggctgatttg ctggtaggat tttgtacagt ttagagaagc 2040
gattatttat tgtgaaactg ttctccactc caactccttt atgtggatct gttcaaagta 2100
gtcactgtat atacgtatag agaggtagat aggtaggtag attttaaatt gcattctgaa 2160
tacaaactca tactccttag agcttgaatt acatttttaa aatgcatatg tgctgtttgg 2220
caccgtggca agatggtatc agagagaaac ccatcaattg ctcaaatact cagaaagtac 2280
tgtcaaaagc ctaataaaa 2299

<210> 29
<211> 1339
<212> DNA
<213> human

<400> 29
ctaaacaaaa tcattcactt ccctgatttt gataagaaaa ttcctgtaaa gctgtttcct 60
ctgcctctcc tctacgttgg aaaccacata agtggattat caagcacaag taaattaagc 120
ctaccgatgt tcaccgtgct caggaaattc accattccac ttaccttact tctggaaacc 180
atcatacttg ggaagcagta ttcactcaac atcatcctca gtgtctttgc cattattctc 240
ggggctttca tagcagctgg gtctgacctt gcttttaact tagaaggcta tatttttgta 300
ttcctgaatg atatcttcac atcagcaaat ggagtttata ccaaacagaa aatggaccca 360
aaggagctag ggaaatacgg agtacttttc tacaatgcct gcttcatgat tatcccaact 420
cttattatta gtgtctccac tggagacctc caacaggcta ctgaattcaa ccaatggaag 480
aatgttgtgt ttatcctaca gtttcttctt tcctgttttt tggggtttct gctgatgtac 540
tccacggttc tgtgcagcta ttacaattca gccctgacga cagcagtggt tggagccatc 600
aagaatgtat ccgttgccta cattgggata ttaatcggtg gagactacat tttctctttg 660
ttaaactttg tagggttaaa tatttgcatg gcagggggct tgagatattc ctttttaaca 720
ctgagcagcc agttaaaacc taaacctgtg ggtgaagaaa acatctgttt ggatttgaag 780
agctaaagag tctgcagcag gattggagac tgacttgtga ctgcgggctg ggggggcatt 840
cccagtagga atgtgaagcc agaggtttcg gattcgtgac atccaccccc tgggcaagtg 900
agagcatctg caaaatgcaa agagaactac ctcatatgca ggatgagcca atggcagtct 960
caagaaatgt actcgggcga caccttacct gtggaaagca aatcttttca aaataagcca 1020
ctgggactcg gtaggtggag ccccagctgc tcttctaggg acctatgggg ccttcgtggc 1080
atctctgtgc tgtgtgctgg ggaggaggtt gatgtaatgg tgactctttt ctgatcagca 1140
ccttggccgt gattcccaag gtcccagcca aagcaaaggg ccagttgttt cagtttaaac 1200
agacatgtct ttagtctaat aaaattagtt aactgccagt aaagttattt gttagctttg 1260
atgaaagcta tgttggtatc tttccctaat catcaaagta aataaaaaat catttctatg 1320

taaaaaaaaa aaaaaaaaa 1339

<210> 30
<211> 4250
<212> DNA
<213> human

<400> 30
gaacacatcg cgtttgcatc ccagaaagta gtcgccgcga ctatttcccc caaagagaca 60

agcacacatg taggaatgac aaaggcttgc gaaggagaga gcgcagcccg cggcccggag 120

agatcccctc gataatggat tactaaatgg gatacacgct gtaccagttc gctccgagcc 180

ccggccgcct gtccgtcgat gcaccgaaaa gggtgaagta gagaaataaa gtctccccgc 240

tgaactacta tgaggtcaga agccttgctg ctatatttca cactgctaca ctttgctggg 300

gctggtttcc cagaagattc tgagccaatc agtatttcgc atggcaacta tacaaaacag 360

tatccggtgt ttgtgggcca caagccagga cggaacacca cacagaggca caggctggac 420

atccagatga ttatgatcat gaacggaacc ctctacattg ctgctaggga ccatatttat 480

actgttgata tagacacatc acacacggaa gaaatttatt gtagcaaaaa actgacatgg 540

aaatctagac aggccgatgt agacacatgc agaatgaagg gaaaacataa ggatgagtgc 600

cacaacttta ttaaagttct tctaaagaaa aacgatgatg cattgtttgt ctgtggaact 660

aatgccttca acccttcctg cagaaactat aagatggata cattggaacc attcggggat 720

gaattcagcg gaatggccag atgcccatat gatgccaaac atgccaacgt tgcactgttt 780

gcagatggaa aactatactc agccacagtg actgacttcc ttgccattga cgcagtcatt 840

taccggagtc ttggagaaag ccctaccctg cggaccgtca agcacgattc aaaatggttg 900

aaagaaccat actttgttca agccgtggat tacggagatt atatctactt cttcttcagg 960

gaaatagcag tggagtataa caccatggga aaggtagttt tcccaagagt ggctcaggtt 1020

tgtaagaatg atatgggagg atctcaaaga gtcctggaga aacagtggac gtcgttcctg 1080

aaggcgcgct tgaactgctc agttcctgga gactctcatt tttatttcaa cattctccag 1140

gcagttacag atgtgattcg tatcaacggg cgtgatgttg tcctggcaac gttttctaca 1200

ccttataaca gcatccctgg gtctgcagtc tgtgcctatg acatgcttga cattgccagt 1260

gtttttactg ggagattcaa ggaacagaag tctcctgatt ccacctggac accagttcct 1320

gatgaacgag ttcctaagcc caggccaggt tgctgtgctg gctcatcctc cttagaaaga 1380

tatgcaacct ccaatgagtt ccctgatgat accctgaact tcatcaagac gcacccgctc 1440

atggatgagg cagtgccctc catcttcaac aggccatggt tcctgagaac aatggtcaga 1500

taccgcctta ccaaaattgc agtggacaca gctgctgggc catatcagaa tcacactgtg 1560

gtttttctgg gatcagagaa gggaatcatc ttgaagtttt tggccagaat aggaaatagt 1620

ggttttctaa atgacagcct ttcctggag gagatgagtg tttacaactc tgaaaaatgc 1680

agctatgatg gagtcgaaga caaaaggatc atgggcatgc agctggacag agcaagcagc 1740

tctctgtatg ttgcgttctc tacctgtgtg ataaaggttc cccttggccg gtgtgaacga 1800
catgggaagt gtaaaaaaac ctgtattgcc tccagagacc catattgtgg atggataaag 1860
gaaggtggtg cctgcagcca tttatcaccc aacagcagac tgacttttga gcaggacata 1920
gagcgtggca atacagatgg tctgggggac tgtcacaatt cctttgtggc actgaatgac 1980
atttcaactc ctctaccaga taatgaaatg tcttacaaca cagtgtatgg gcattccagt 2040
tccctcttgc ccagcacaac cacatcagat tcgacggctc aagaggggta tgagtctagg 2100
ggaggaatgc tggactggaa gcatctgctt gactcacctg acagcacaga ccctttgggg 2160
gcagtgtctt cccataatca ccaagacaag aagggagtga ttcgggaaag ttacctcaaa 2220
ggccacgacc agctggttcc cgtcaccctc ttggccattg cagtcatcct ggctttcgtc 2280
atgggggccg tcttctcggg catcaccgtc tactgcgtct gtgatcatcg gcgcaaagac 2340
gtggctgtgg tgcagcgcaa ggagaaggag ctcacccact cgcgccgggg ctccatgagc 2400
agcgtcacca agctcagcgg cctctttggg gacactcaat ccaaagaccc aaagccggag 2460
gccatcctca cgccactcat gcacaacggc aagctcgcca ctcccggcaa cacggccaag 2520
atgctcatta aagcagacca gcaccacctg gacctgacgg ccctccccac cccagagtca 2580
accccaacgc tgcagcagaa gcggaagccc agccgcggca gccgcgagtg ggagaggaac 2640
cagaacctca tcaatgcctg cacaaaggac atgcccccca tgggctcccc tgtgattccc 2700
acggacctgc ccctgcgggc ctcccccagc cacatcccca gcgtggtggt cctgcccatc 2760
acgcagcagg gctaccagca tgagtacgtg gaccagccca aaatgagcga ggtggcccag 2820
atggcgctgg aggaccaggc cgccacactg gagtataaga ccatcaagga acatctcagc 2880
agcaagagtc ccaaccatgg ggtgaacctt gtggagaacc tggacagcct gccccccaaa 2940
gttccacagc gggaggcctc cctgggtccc ccgggagcct ccctgtctca gaccggtcta 3000
agcaagcggc tggaaatgca ccactcctct tcctacgggg ttgactataa gaggagctac 3060
cccacgaact cgctcacgag aagccaccag gccaccactc tcaaaagaaa caacactaac 3120
tcctccaatt cctctcacct ctccagaaac cagagctttg gcaggggaga caacccgccg 3180
cccgccccgc agagggtgga ctccatccag gtgcacagct cccagccatc tggccaggcc 3240
gtgactgtct cgaggcagcc cagcctcaac gcctacaact cactgacaag gtcggggctg 3300
aagcgtacgc cctcgctaaa gccggacgta cccccaaac catcctttgc tccccttcc 3360
acatccatga agcccaatga tgcgtgtaca taatcccagg gggagggggt caggtgtcga 3420
accagcaggc aaggcgaggt gcccgctcag ctcagcaagg ttctcaactg cctcgagtac 3480
ccaccagacc aagaaggcct gcggcagagc cgaggacgct gggtcctcct ctctgggaca 3540
caggggtact cacgaaaact gggccgcgtg gtttggtgaa ggtttgcaac ggcggggact 3600
caccttcatt ctcttccttc actttccccc acaccctaca acaggtcgga cccacaaaag 3660
acttcagtta tcatcacaaa catgagccaa aagcacatac ctaccccatc cccacccccc 3720
acacacacac acacatgcac acaacacata cacacacacg cacagaggtg aacagaaact 3780

gaaacatttt gtccacaact tcacgggacg tggccagact gggtttgcgt tccaacctgc 3840
aaaacacaaa tacatttttt aaaatcaaga aaatttaaaa agacaaaaaa aaaagaattc 3900
attgataatt ctaactcaga ctttaacaat ggcagaagtt tactatgcgc aaatactgtg 3960
aaatgcccgc cagtgttaca gctttctgtt gcagcagata aatgccatgt tgggcaacta 4020
tgtcatagat ttctgctcct cctctctttt aatgaaataa cgtgaccgtt aacgcaagta 4080
actctttatt tattgttcac cctttttttc cttaaggaaa ggactcttcc aaatatcatc 4140
ctatgaacag ctcttcagaa agcccattga aagttaaact atttaacgtg aaatccatta 4200
actggaataa ttgagtttct ttatttttac aataaattca ctgagtaaat 4250

<210> 31
<211> 2785
<212> DNA
<213> human

<400> 31
ctttagccca acagtcaaaa ataattgatg ctaccctaca aatgtccaaa actctagtat 60
atcatatttc taagttacag caaatattag tcctgctaaa ccagggagct ttggcaaaaa 120
tgttttttga cagtaaattt gtccttgatt atatattaac tagtcaaaga ggtgtttgta 180
acattattag agcttcttgt tgtaggtggg ttaacaccac caatcaagag gtcattctaa 240
cagaaagcct ggatcagaaa accatcaccc taaaaaaaca tgccttacat atttaacaca 300
ctctgaaatc cagtcaaaat atgactaaag gcccttgcca tgactgatgt attctcctgg 360
ccaacgccaa acaaatggga gcctggttac gagtcagcct tcagggactt gtcacatttc 420
tacttggttt cttccttgtt attgtcataa taaaatgttt tctatgctgt ttagtgcaac 480
ttaggcccta ttctgtagaa gtctcctcta ctattcaggc cactcaaaca ccccaaataa 540
ttgagttcaa aatcgacatc aagatataaa ggaatcagtg actaaatata tttcatatat 600
ggtattttta ttgattattg tgctgtcttg acctagtatg gaggccttgg ctagaggctg 660
gtcagtttcc tctcttgagc agctgattaa atccacaccc caaccacttc ccttatcagg 720
ttctcacact ctggggccac tatgtaccca ctctaatcac cacagggcca gacatcagac 780
aattaaggac agcgcccatg ccccaaagcc cgccaaaatt atgcaaatta ttcaaaatta 840
ttcaacctag ctaaccccac ccttttttgct gtacataagc tgcccattcc ccctccagcc 900
tgtggtaccc agtcctcagg tgcaaccccc tgcgtggtcc tctgtggcag ccttctctca 960
ttcagagctg ttttccacag aggtagtgaa aagaactgga ttttcaagtt cactttgcaa 1020
gagaaaaaga aaactcagta gaagataatg gcaagtccag actggggata tgatgacaaa 1080
aatggtcctg aacaatggag caagctgtat cccattgcca atggaaataa ccaatcccct 1140
gttgatatta aaaccagtga aaccaaacat gacacctctc tgaaacctat tagtgtctcc 1200
tacaacccag ccacagccaa agaaattatc aatgtggggc attctttcca tgtaaatttt 1260
gaggacaacg ataaccgatc agtgctgaaa ggtggtcctt tctctgacag ctacaggctc 1320
tttcagtttc attttcactg ggcagtacaa aatgagcatg gttcagaaca tacagtggat 1380

```
ggagtcaaat attctgccga gcttcacgta gctcactgga attctgcaaa gtactccagc   1440
cttgctgaag ctgcctcaaa ggctgatggt ttggcagtta ttggtgtttt gatgaaggtt   1500
ggtgaggcca acccaaagct gcagaaagta cttgatgccc tccaagcaat taaaaccaag   1560
ggcaaacgag ccccattcac aaattttgac ccctctactc tccttccttc atccctggat   1620
ttctggacct accctggctc tctgactcat cctcctcttt atgagagtgt aacttggatc   1680
atctgtaagg agagcatcag tgtcagctca gagcagctgg cacaattccg cagccttcta   1740
tcaaatgttg aaggtgataa cgctgtcccc atgcagcaca acaaccgccc aacccaacct   1800
ctgaagggca gaacagtgag agcttcattt tgatgattct gagaagaaac ttgtccttcc   1860
tcaagaacac agccctgctt ctgacataat ccagttaaaa taataatttt taagaaataa   1920
atttatttca atattagcaa gacagcatgc cttcaaatca atctgtaaaa ctaagaaact   1980
taaattttag ttcttactgc ttaattcaaa taataattag taagctagca aatagtaatc   2040
tgtaagcata agcttatctt aaattcaagt ttagtttgag gaattcttta aaattacaac   2100
taagtgattt gtatgtctat ttttttcagt ttatttgaac caataaaata attttatctc   2160
tttctttctg ttgtgcattc agtttctaaa accattaagt ttctactcca tttacattca   2220
aaaatcttaa atactttact tgcaagagta ttttgcttca aatacaacaa cctaagagca   2280
gctggagatg aaatattggg aaattcattt gcttactcct gaagacaaaa atatagctga   2340
gatgaccact ggatttaata tcgttatgct ggcccaacat tgctaccatt tgtgttgtct   2400
gtgatcaaaa tgattatctt ttatatagga agatgacgct tctggatatt gctttcactt   2460
cttctcccca cgttagcaag gacaatgctt ctctgccatt attacaacta gttagtttgc   2520
atggagaatc tttactttaa aattggaaga aaagtcacaa gtgaatggtt tataaaaatg   2580
ctaaagaagt cattcttgct tagaatcata tagaaacatc atgcaatctt ttagtcagat   2640
gtgcgcttca ccttatgcta tttttatctt taattgacac acaataattg tacatgttta   2700
tggagtatag tgtggtgttt tctgtttgtt tgtttgtttt ttgagacaag gtctcactct   2760
gccagtcagg gtggagtgcg atggt                                         2785
```

```
<210>  32
<211>  9588
<212>  DNA
<213>  human

<400>  32
ccgaccaaca ccaacaccca gctccgacgc agctcctctg cgcccttgcc gccctccgag     60
ccacagcttt cctcccgctc ctgcccccgg cccgtcgccg tctccgcgct cgcagcggcc    120
tcgggagggc ccaggtagcg agcagcgacc tcgcgagcct tccgcactcc cgcccggttc    180
cccggccgtc cgcctatcct tggccccctc cgctttctcc gcgccggccc gcctcgctta    240
tgcctcggcg ctgagccgct ctcccgattg cccgccgaca tgagctgcaa cggaggctcc    300
cacccgcgga tcaacactct gggccgcatg atccgcgccg agtctggccc ggacctgcgc    360
```

tacgaggtga ccagcggcgg cgggggcacc agcaggatgt actattctcg gcgcggcgtg 420

atcaccgacc agaactcgga cggctactgt caaaccggca cgatgtccag gcaccagaac 480

cagaacacca tccaggagct gctgcagaac tgctccgact gcttgatgcg agcagagctc 540

atcgtgcagc ctgaattgaa gtatggagat ggaatacaac tgactcggag tcgagaattg 600

gatgagtgtt ttgcccaggc caatgaccaa atggaaatcc tcgacagctt gatcagagag 660

atgcggcaga tgggccagcc ctgtgatgct taccagaaaa ggcttcttca gctccaagag 720

caaatgcgag ccctttataa agccatcagt gtccctcgag tccgcagggc cagctccaag 780

ggtggtggag gctacacttg tcagagtggc tctggctggg atgagttcac caaacatgtc 840

accagtgaat gtttggggtg gatgaggcag caaagggcgg agatggacat ggtggcctgg 900

ggtgtggacc tggcctcagt ggagcagcac attaacagcc accggggcat ccacaactcc 960

atcggcgact atcgctggca gctggacaaa atcaaagccg acctgcgcga gaaatctgcg 1020

atctaccagt tggaggagga gtatgaaaac ctgctgaaag cgtcctttga gaggatggat 1080

cacctgcgac agctgcagaa catcattcag gccacgtcca gggagatcat gtggatcaat 1140

gactgcgagg aggaggagct gctgtacgac tggagcgaca agaacaccaa catcgctcag 1200

aaacaggagg ccttctccat acgcatgagt caactggaag ttaaagaaaa agagctcaat 1260

aagctgaaac aagaaagtga ccaacttgtc ctcaatcagc atccagcttc agacaaaatt 1320

gaggcctata tggacactct gcagacgcag tggagttgga ttcttcagat caccaagtgc 1380

attgatgttc atctgaaaga aaatgctgcc tactttcagt tttttgaaga ggcgcagtct 1440

actgaagcat acctgaaggg gctccaggac tccatcagga agaagtaccc ctgcgacaag 1500

aacatgcccc tgcagcacct gctggaacag atcaaggagc tggagaaaga acgagagaaa 1560

atccttgaat acaagcgtca ggtgcagaac ttggtaaaca agtctaagaa gattgtacag 1620

ctgaagcctc gtaacccaga ctacagaagc aataaaccca ttattctcag agctctctgt 1680

gactacaaac aagatcagaa aatcgtgcat aagggggatg agtgtatcct gaaggacaac 1740

aacgagcgca gcaagtggta cgtgacgggc ccgggaggcg ttgacatgct tgttccctct 1800

gtggggctga tcatccctcc tccgaaccca ctggccgtgg acctctcttg caagattgag 1860

cagtactacg aagccatctt ggctctgtgg aaccagctct acatcaacat gaagagcctg 1920

gtgtcctggc actactgcat gattgacata gagaagatca gggccatgac aatcgccaag 1980

ctgaaaacaa tgcggcagga agattacatg aagacgatag ccgaccttga gttacattac 2040

caagagttca tcagaaatag ccaaggctca gagatgtttg gagatgatga caagcggaaa 2100

atacagtctc agttcaccga tgcccagaag cattaccaga ccctggtcat tcagctccct 2160

ggctatcccc agcaccagac agtgaccaca actgaaatca ctcatcatgg aacctgccaa 2220

gatgtcaacc ataataaagt aattgaaacc aacagagaaa atgacaagca agaaacatgg 2280

atgctgatgg agctgcagaa gattcgcagg cagatagagc actgcgaggg caggatgact 2340

ctcaaaaacc tccctctagc agaccagggg tcttctcacc acatcacagt gaaaattaac 2400

gagcttaaga gtgtgcagaa tgattcacaa gcaattgctg aggttctcaa ccagcttaaa 2460
gatatgcttg ccaacttcag aggttctgaa aagtactgct atttacagaa tgaagtattt 2520
ggactatttc agaaactgga aaatatcaat ggtgttacag atggctactt aaatagctta 2580
tgcacagtaa gggcactgct ccaggctatt ctccaaacag aagacatgtt aaaggtttat 2640
gaagccaggc tcactgagga ggaaactgtc tgcctggacc tggataaagt ggaagcttac 2700
cgctgtggac tgaagaaaat aaaaaatgac ttgaacttga agaagtcgtt gttggccact 2760
atgaagacag aactacagaa agcccagcag atccactctc agacttcaca gcagtatcca 2820
ctttatgatc tggacttggg caagttcggt gaaaaagtca cacagctgac agaccgctgg 2880
caaaggatag ataaacagat cgactttaga ttatgggacc tggagaaaca aatcaagcaa 2940
ttgaggaatt atcgtgataa ctatcaggct ttctgcaagt ggctctatga tcgtaaacgc 3000
cgccaggatt ccttagaatc catgaaattt ggagattcca acacagtcat gcggtttttg 3060
aatgagcaga agaacttgca cagtgaaata tctggcaaac gagacaaatc agaggaagta 3120
caaaaaattg ctgaactttg cgccaattca attaaggatt atgagctcca gctggcctca 3180
tacacctcag gactggaaac tctgctgaac atacctatca agaggaccat gattcagtcc 3240
ccttctgggg tgattctgca agaggctgca gatgttcatg ctcggtacat tgaactactt 3300
acaagatctg gagactatta caggttctta agtgagatgc tgaagagttt ggaagatctg 3360
aagctgaaaa ataccaagat cgaagttttg gaagaggagc tcagactggc ccgagatgcc 3420
aactcggaaa actgtaataa gaacaaattc ctggatcaga acctgcagaa ataccaggca 3480
gagtgttccc agttcaaagc gaagcttgcg agcctggagg agctgaagag acaggctgag 3540
ctggatggga agtcggctaa gcaaaatcta gacaagtgct acggccaaat aaaagaactc 3600
aatgagaaga tcacccgact gacttatgag attgaagatg aaaagagaag aagaaaatct 3660
gtggaagaca gatttgacca acagaagaat gactatgacc aactgcagaa agcaaggcaa 3720
tgtgaaaagg agaaccttgg ttggcagaaa ttagagtctg agaaagccat caaggagaag 3780
gagtacgaga ttgaaaggtt gagggttcta ctgcaggaag aaggcacccg gaagagagaa 3840
tatgaaaatg agctggcaaa ggtaagaaac cactataatg aggagatgag taatttaagg 3900
aacaagtatg aaacagagat taacattacg aagaccacca tcaaggagat atccatgcaa 3960
aaagaggatg attccaaaaa tcttagaaac cagcttgata gactttcaag ggaaaatcga 4020
gatctgaagg atgaaattgt caggctcaat gacagcatct tgcaggccac tgagcagcga 4080
aggcgagctg aagaaaacgc ccttcagcaa aaggcctgtg gctctgagat aatgcagaag 4140
aagcagcatc tggagataga actgaagcag gtcatgcagc agcgctctga ggacaatgcc 4200
cggcacaagc agtccctgga ggaggctgcc aagaccattc aggacaaaaa taaggagatc 4260
gagagactca aagctgagtt tcaggaggag gccaagcgcc gctgggaata tgaaaatgaa 4320
ctgagtaagg taagaaacaa ttatgatgag gagatcatta gcttaaaaaa tcagtttgag 4380
accgagatca acatcaccaa gaccaccatc caccagctca ccatgcagaa ggaagaggat 4440

-

accagtggct accgggctca gatagacaat ctcacccgag aaaacaggag cttatctgaa 4500
gaaataaaga ggctgaagaa cactctaacc cagaccacag agaatctcag gagggtggaa 4560
gaagacatcc aacagcaaaa ggccactggc tctgaggtgt ctcagaggaa acagcagctg 4620
gaggttgagc tgagacaagt cactcagatg cgaacagagg agagcgtaag atataagcaa 4680
tctcttgatg atgctgccaa aaccatccag gataaaaaca aggagataga aaggttaaaa 4740
caactgatcg acaaagaaac aaatgaccgg aaatgcctgg aagatgaaaa cgcgagatta 4800
caaagggtcc agtatgacct gcagaaagca aacagtagtg cgacggagac aataaacaaa 4860
ctgaaggttc aggagcaaga actgacacgc ctgaggatcg actatgaaag ggtttcccag 4920
gagaggactg tgaaggacca ggatatcacg cggttccaga actctctgaa agagctgcag 4980
ctgcagaagc agaaggtgga agaggagctg aatcggctga agaggaccgc gtcagaagac 5040
tcctgcaaga ggaagaagct ggaggaagag ctggaaggca tgaggaggtc gctgaaggag 5100
caagccatca aaatcaccaa cctgacccag cagctggagc aggcatccat tgttaagaag 5160
aggagtgagg atgacctccg gcagcagagg gacgtgctgg atggccacct gagggaaaag 5220
cagaggaccc aggaagagct gaggaggctc tcttctgagg tcgaggccct gaggcggcag 5280
ttactccagg aacaggaaag tgtcaaacaa gctcacttga ggaatgagca tttccagaag 5340
gcgatagaag ataaaagcag aagcttaaat gaaagcaaaa tagaaattga gaggctgcag 5400
tctctcacag agaacctgac caaggagcac ttgatgttag aagaagaact gcggaacctg 5460
aggctggagt acgatgacct gaggagagga cgaagcgaag cggacagtga taaaaatgca 5520
accatcttgg aactaaggag ccagctgcag atcagcaaca accggaccct ggaactgcag 5580
gggctgatta atgatttaca gagagagagg gaaaatttga gacaggaaat tgagaaattc 5640
caaaagcagg ctttagaggc atctaatagg attcaggaat caaagaatca gtgtactcag 5700
gtggtacagg aaagagagag ccttctggtg aaaatcaaag tcctggagca agacaaggca 5760
aggctgcaga ggctggagga tgagctgaat cgtgcaaaat caactctaga ggcagaaacc 5820
agggtgaaac agcgcctgga gtgtgagaaa cagcaaattc agaatgacct gaatcagtgg 5880
aagactcaat attcccgcaa ggaggaggct attaggaaga tagaatcgga aagagaaaag 5940
agtgagagag agaagaacag tcttaggagt gagatcgaaa gactccaagc agagatcaag 6000
agaattgaag agaggtgcag gcgtaagctg gaggattcta ccagggagac acagtcacag 6060
ttagaaacag aacgctcccg atatcagagg gagattgata aactcagaca gcgcccatat 6120
gggtcccatc gagagaccca gactgagtgt gagtggaccg ttgacacctc caagctggtg 6180
tttgatgggc tgaggaagaa ggtgacagca atgcagctct atgagtgtca gctgatcgac 6240
aaaacaacct tggacaaact attgaagggg aagaagtcag tggaagaagt tgcttctgaa 6300
atccagccat tccttcgggg tgcaggatct atcgctggag catctgcttc tcctaaggaa 6360
aaatactctt tggtagaggc caagagaaag aaattaatca gcccagaatc cacagtcatg 6420
cttctggagg cccaggcagc tacaggtggt ataattgatc cccatcggaa tgagaagctg 6480

actgtcgaca gtgccatagc tcgggacctc attgacttcg atgaccgtca gcagatatat 6540
gcagcagaaa aagctatcac tggttttgat gatccatttt caggcaagac agtatctgtt 6600
tcagaagcca tcaagaaaaa tttgattgat agagaaaccg gaatgcgcct gctggaagcc 6660
cagattgctt caggggggtgt agtagaccct gtgaacagtg tctttttgcc aaaagatgtc 6720
gccttggccc gggggctgat tgatagagat ttgtatcgat ccctgaatga tccccgagat 6780
agtcagaaaa actttgtgga tccagtcacc aaaaagaagg tcagttacgt gcagctgaag 6840
gaacggtgca gaatcgaacc acatactggt ctgctcttgc tttcagtaca gaagagaagc 6900
atgtccttcc aaggaatcag acaacctgtg accgtcactg agctagtaga ttctggtata 6960
ttgagaccgt ccactgtcaa tgaactggaa tctggtcaga tttcttatga cgaggttggt 7020
gagagaatta aggacttcct ccagggttca agctgcatag caggcatata caatgagacc 7080
acaaaacaga agcttggcat ttatgaggcc atgaaaattg gcttagtccg acctggtact 7140
gctctggagt tgctggaagc ccaagcagct actggcttta tagtggatcc tgttagcaac 7200
ttgaggttac cagtggagga agcctacaag agaggtctgg tgggcattga gttcaaagag 7260
aagctcctgt ctgcagaacg agctgtcact gggtataatg atcctgaaac aggaaacatc 7320
atctctttgt tccaagccat gaataaggaa ctcatcgaaa agggccacgg tattcgctta 7380
ttagaagcac agatcgcaac cggggggatc attgacccaa aggagagcca tcgtttacca 7440
gttgacatag catataagag gggctatttc aatgaggaac tcagtgagat tctctcagat 7500
ccaagtgatg ataccaaagg atttttgac cccaacactg aagaaaatct tacctatctg 7560
caactaaaag aaagatgcat taaggatgag gaaacagggc tctgtcttct gcctctgaaa 7620
gaaaagaaga aacaggtgca gacatcacaa aagaataccc tcaggaagcg tagagtggtc 7680
atagttgacc cagaaaccaa taaagaaatg tctgttcagg aggcctacaa gaagggccta 7740
attgattatg aaaccttcaa agaactgtgt gagcaggaat gtgaatggga agaaataacc 7800
atcacgggat cagatggctc caccagggtg gtcctggtag atagaaagac aggcagtcag 7860
tatgatattc aagatgctat tgacaagggc cttgttgaca ggaagttctt tgatcagtac 7920
cgatccggca gcctcagcct cactcaattt gctgacatga tctccttgaa aaatggtgtc 7980
ggcaccagca gcagcatggg cagtggtgtc agcgatgatg tttttagcag ctcccgacat 8040
gaatcagtaa gtaagatttc caccatatcc agcgtcagga atttaaccat aaggagcagc 8100
tctttttcag acaccctgga agaatcgagc cccattgcag ccatctttga cacagaaaac 8160
ctggagaaaa tctccattac agaaggtata gagcggggca tcgttgacag catcacgggt 8220
cagaggcttc tggaggctca ggcctgcaca ggtggcatca tccacccaac cacgggccag 8280
aagctgtcac ttcaggacgc agtctcccag ggtgtgattg accaagacat ggccaccagc 8340
gtgaagcctg ctcagaaagc cttcataggc ttcgagggtg tgaagggaaa gaagaagatg 8400
tcagcagcag aggcagtgaa agaaaaatgg ctcccgtatg aggctggcca gcgcttcctg 8460
gagttccagt acctcacggg aggtcttgtt gacccggaag tgcatgggag gataagcacc 8520

gaagaagcca tccggaaggg gttcatagat ggccgcgccg cacagaggct gcaagacacc 8580
agcagctatg ccaaaatcct gacctgcccc aaaaccaaat taaaaatatc ctataaggat 8640
gccataaatc gctccatggt agaagatatc actgggctgc gccttctgga agccgcctcc 8700
gtgtcgtcca agggcttacc cagcccttac aacatgtctt cggctccggg gtcccgctcc 8760
ggctcccgct cgggatctcg ctccggatct cgctccgggt cccgcagtgg gtcccggaga 8820
ggaagctttg acgccacagg gaattcttcc tactcttatt cctactcatt tagcagtagt 8880
tctattgggc actagtagtc agttgggagt ggttgctata ccttgacttc atttatatga 8940
atttccactt tattaaataa tagaaaagaa aatcccggtg cttgcagtag agtgatagga 9000
cattctatgc ttacagaaaa tatagccatg attgaaatca aatagtaaag gctgttctgg 9060
ctttttatct tcttagctca tcttaaataa gcagtacact tggatgcagt gcgtctgaag 9120
tgctaatcag ttgtaacaat agcacaaatc gaacttagga tttgtttctt ctcttctgtg 9180
tttcgatttt tgatcaattc tttaattttg gaagcctata atacagtttt ctattcttgg 9240
agataaaaat taaatggatc actgatattt tagtcattct gcttctcatc taaatatttc 9300
catattctgt attaggagaa aattaccctc ccagcaccag ccccctctc aaaccccaa 9360
cccaaaacca agcattttgg aatgagtctc ctttagtttc agagtgtgga ttgtataacc 9420
catatactct tcgatgtact tgtttggttt ggtattaatt tgactgtgca tgacagcggc 9480
aatcttttct ttggtcaaag ttttctgttt attttgcttg tcatattcga tgtactttaa 9540
ggtgtcttta tgaagtttgc tattctggca ataaactttt agactttt 9588

<210> 33
<211> 366
<212> DNA
<213> human

<220>
<221> misc_feature
<222> (351)..(351)
<223> any kind of base

<400> 33
gaagtgccat ttatatttat acaaaaatat tacataattc agttagtatt ggtgacataa 60
tttagttagt atgggtgata taatggtcat aatttttagc atctaataaa gatcttttta 120
tgagtcccat ataaaatatg tgaacaaagc aatcttgtca taagatttgt gatgatttag 180
gagaaagtac tttgagataa ttttttctg tctctttgtg aactctctca acagtagttc 240
tctttagatt agagccagca ggtcggccat aacagttttc ttcaaatttg ggcaacagtt 300
atacaaatgc ttgaatttca agacaacata ttaaagggtc tatgaactgg naatctaacc 360
tgggtt 366

<210> 34
<211> 1466
<212> DNA
<213> human

<400> 34

```
agccccaagc ttaccacctg cacccggaga gctgtgtgtc accatgtggg tcccggttgt     60
cttcctcacc ctgtccgtga cgtggattgg tgctgcaccc ctcatcctgt ctcggattgt    120
gggaggctgg gagtgcgaga agcattccca accctggcag gtgcttgtgg cctctcgtgg    180
cagggcagtc tgcggcggtg ttctggtgca cccccagtgg gtcctcacag ctgcccactg    240
catcaggaac aaaagcgtga tcttgctggg tcggcacagc ctgtttcatc ctgaagacac    300
aggccaggta tttcaggtca gccacagctt cccacacccg ctctacgata tgagcctcct    360
gaagaatcga ttcctcaggc caggtgatga ctccagccac gacctcatgc tgctccgcct    420
gtcagagcct gccgagctca cggatgctgt gaaggtcatg gacctgccca cccaggagcc    480
agcactgggg accacctgct acgcctcagg ctggggcagc attgaaccag aggagttctt    540
gaccccaaag aaacttcagt gtgtggacct ccatgttatt tccaatgacg tgtgtgcgca    600
agttcaccct cagaaggtga ccaagttcat gctgtgtgct ggacgctgga caggggggcaa    660
aagcacctgc tcgggtgatt ctgggggccc acttgtctgt aatggtgtgc ttcaaggtat    720
cacgtcatgg ggcagtgaac catgtgccct gcccgaaagg ccttccctgt acaccaaggt    780
ggtgcattac cggaagtgga tcaaggacac catcgtggcc aacccctgag cacccctatc    840
aacccccctat tgtagtaaac ttggaacctt ggaaatgacc aggccaagac tcaagcctcc    900
ccagttctac tgacctttgt ccttaggtgt gaggtccagg gttgctagga aaagaaatca    960
gcagacacag gtgtagacca gagtgtttct taaatggtgt aattttgtcc tctctgtgtc   1020
ctggggaata ctggccatgc ctggagacat atcactcaat ttctctgagg acacagatag   1080
gatggggtgt ctgtgttatt tgtggggtac agagatgaaa gaggggtggg atccacactg   1140
agagagtgga gagtgacatg tgctggacac tgtccatgaa gcactgagca gaagctggag   1200
gcacaacgca ccagacactc acagcaagga tggagctgaa aacataaccc actctgtcct   1260
ggaggcactg ggaagcctag agaaggctgt gagccaagga gggagggtct tcctttggca   1320
tgggatgggg atgaagtaag gagagggact ggacccccctg gaagctgatt cactatgggg   1380
ggaggtgtat tgaagtcctc cagacaaccc tcagatttga tgatttccta gtagaactca   1440
cagaaataaa gagctgttat actgtg                                        1466
```

<210> 35
<211> 187
<212> DNA
<213> human

<400> 35

```
gatctggtgc attccggtcg acactctcgt ttatttggac tgtaagtctg acctctatga     60
ataattactt cagcccctga gtgctcccgg gccaagctcc ttggccaaac tttcacctta    120
gcttctgata agtcttgggc caagctaagc agcatctatc aatcatccct tcagctcctg    180
attgatc                                                              187
```

<210> 36
<211> 2913
<212> DNA
<213> human

<400> 36
actgggtacc gaggactggg tgtgtttaag gcagacagcc aggtgaggat cccagctact 60
ggggcctgct gtcatctcct gggagtaccc gggggtcagg agcctagggg actcttgcac 120
ttcacatcca gccatgctaa ttacactttt tggcaaagga aacagctagg agcagtttct 180
ttcactccta cagccccgtt ttctcagtgt ttagacctcg aattattact gggctagagg 240
gaaggcagcc tctgaagtgt ggcaggagga ggggaagtct gcctgcatct tggtgtgtct 300
gtcagatgcc agcactaata acctggcttc tgtgaggcct gtcagtgctc tcaggaatga 360
aaggggaccc ctgagaggtg ctcagtacca gcaggctgtg aatgctctct acccaccacc 420
ctcacctcct cgttaaagat ggtgctacct gccacacagc agacatctgg tcgctgcaca 480
cccgaaagac cccaaggcag tctgcccctt gtccagccac acgccagcac ccaccctcct 540
ggcccctgcc tcggcctccc cagaccagct gcacccagcc cccaacacgc accccttctc 600
cagatgtgtg cagggcctca ttttgcagag caaagacaga tgtttcagcc acacgcttta 660
ttaacttcta aaacctgtgc tcaggacact cttcaacagt catgaaaagt ttgatcactt 720
gccacagtca ggacctttgt gtggggctct gatctgatgt tcggtctcat catctcccaa 780
accagcagtc gtttgtaccc caaccctctg ctcaggggct cataccccca aatgattttc 840
ctgatttatg tatttcccta caaagggctt tctataccta gcatctgcct ccagcatgag 900
aagggggaat aggtgagacc catttgccag tagcagacgg ggaccctggg gagaaaatgg 960
cagagcctgt tggagactcc ctgtctccag ctgaccagcc aatgggattc ctcttccctc 1020
cactgtctcc cacaaagtag aagaatcctg gtacatttag cccatgagcc tggcacagat 1080
ccctatctag acatgaggcc ctttagacat gactttggca ttgaccagcc tgttggcaat 1140
gggtcgggga ggcagagggg atgctcacac cagtaattct catcccctga atgcttggga 1200
tcacctgggg agagttcaca aaatactggt gcaggggtcc cacctctgat gatgctgagt 1260
ggtgggtctg ggtgtggcc caggcatcat gatgtttcag gcccccaggt gacttcttag 1320
gcagcccagc taagccccta gagccttgca atttccccca aatgacctca gagggcccga 1380
tttgagggaa atgcctaact tcaggggccg taagaatccc ccagggagca tgtgaaatgc 1440
agataccagg cccaccccca gagatgagct gaggtgggtc aggggtgaag tgcagggatc 1500
agtgtttttc acaagctcca tacctccagg aaatggtgtt gtggttgggc ccgtagaaaa 1560
cattctgaga gtcctgttgc ctgtgccttg gtgcacgtgg ggtggaatcc cagtggccct 1620
gccttgagga ggatgtgcat taacgtggta ggggagacag agacagctcc acctgccccc 1680
tgtcccaccg gggacctcca aaaacttcat ggatgttaga gcaagcagcc atgctgcagc 1740
agaggatgag gctggcggat ttagtaagag ccctctgtgt ttgggctgag ttctttctct 1800
agttgccctg tcatctggcc tctggataac ccacctctcc tccctcatcc taaaattaca 1860

gatggcgaaa gacggccaca tttagtgaga cccctaaggt cctccaacta gggtgggtcc 1920
acagtggccc ctggtgcatg gaccacacac tctcttccct cctctggctc aggactacgg 1980
tctgaaatta gggagatatg aatgtctttc ttgaaaactt ctcttcccag tcttcccact 2040
ttgcttgggg gtccttggtc aaggccagct ttggactagg gcttgttgcg actaccagct 2100
gtctcatttt gctgtactgc aaactcaggc ttggttccaa gcttatgggg gccctgtcct 2160
tcccctagta gggtttgttt tggggtcaca tctggtcata cccttcagag agctcttccc 2220
cagcctctac atcagggaga gaggtaggta gggaggagca ttcaaggatt agaagaagga 2280
ctaaagtaca acagccttgg aggaactgcc aggaactaag ggcgagcact ggagaaggca 2340
acctgggacc ccctgcgctt ctgagcagga agaccaagac cttcaggggc cctaagcact 2400
gaaaacatca ttcctcatcc ccaagccctg gcatccccct gttcttctaa aataattctt 2460
ttctaggtat ttctgattgc aaaattctgg atgggttcat ccaagctgac ctttgctgtt 2520
ttttcccttc ccaacaaggc ctcacttttt ggagccacct tagttggtgc ctaggcagag 2580
gggcagtcag cagtggttat caggatcctg gctctatggg ttgccttcct cctggtctgt 2640
aaagcccctg caggcaggga cttcttagat agctgcttcc ttagggcatg gcatgtggtg 2700
ggtggttaat gaatggaaga gagggaatga gtgatcaagg gagggaggag ggagtggagt 2760
ggagatttct catcctttcc tgttaattta tgacatcctc ctgcctatga gtccttgact 2820
ctggagtttt acaaagcagt cacatttcaa ataaaagtct gggaaagcaa cacatcatcg 2880
ccaactttta attttgctaa ataaggatat tag 2913

<210> 37
<211> 1466
<212> DNA
<213> human

<400> 37
agccccaagc ttaccacctg cacccggaga gctgtgtgtc accatgtggg tcccggttgt 60
cttcctcacc ctgtccgtga cgtggattgg tgctgcaccc ctcatcctgt ctcggattgt 120
gggaggctgg gagtgcgaga agcattccca accctggcag gtgcttgtgg cctctcgtgg 180
cagggcagtc tgcggcggtg ttctggtgca cccccagtgg gtcctcacag ctgcccactg 240
catcaggaac aaaagcgtga tcttgctggg tcggcacagc ctgtttcatc ctgaagacac 300
aggccaggta tttcaggtca gccacagctt cccacacccg ctctacgata tgagcctcct 360
gaagaatcga ttcctcaggc caggtgatga ctccagccac gacctcatgc tgctccgcct 420
gtcagagcct gccgagctca cggatgctgt gaaggtcatg gacctgccca cccaggagcc 480
agcactgggg accacctgct acgcctcagg ctggggcagc attgaaccag aggagttctt 540
gaccccaaag aaacttcagt gtgtggacct ccatgttatt tccaatgacg tgtgtgcgca 600
agttcaccct cagaaggtga ccaagttcat gctgtgtgct ggacgctgga caggggggcaa 660
aagcacctgc tcgggtgatt ctgggggccc acttgtctgt aatggtgtgc ttcaaggtat 720
cacgtcatgg ggcagtgaac catgtgccct gcccgaaagg ccttccctgt acaccaaggt 780

ggtgcattac cggaagtgga tcaaggacac catcgtggcc aacccctgag cacccctatc 840
aaccccctat tgtagtaaac ttggaacctt ggaaatgacc aggccaagac tcaagcctcc 900
ccagttctac tgacctttgt ccttaggtgt gaggtccagg gttgctagga aaagaaatca 960
gcagacacag gtgtagacca gagtgtttct taaatggtgt aattttgtcc tctctgtgtc 1020
ctggggaata ctggccatgc ctggagacat atcactcaat ttctctgagg acacagatag 1080
gatggggtgt ctgtgttatt tgtggggtac agagatgaaa gaggggtggg atccacactg 1140
agagagtgga gagtgacatg tgctggacac tgtccatgaa gcactgagca gaagctggag 1200
gcacaacgca ccagacactc acagcaagga tggagctgaa aacataaccc actctgtcct 1260
ggaggcactg ggaagcctag agaaggctgt gagccaagga gggagggtct tcctttggca 1320
tgggatgggg atgaagtaag gagagggact ggaccccctg gaagctgatt cactatgggg 1380
ggaggtgtat tgaagtcctc cagacaaccc tcagatttga tgatttccta gtagaactca 1440
cagaaataaa gagctgttat actgtg 1466

<210> 38
<211> 462
<212> DNA
<213> human

<220>
<221> misc_feature
<222> (116)..(116)
<223> any kind of base

<220>
<221> misc_feature
<222> (197)..(197)
<223> any kind of base

<220>
<221> misc_feature
<222> (334)..(334)
<223> any kind of base

<220>
<221> misc_feature
<222> (402)..(402)
<223> any kind of base

<220>
<221> misc_feature
<222> (429)..(429)
<223> any kind of base

<220>
<221> misc_feature
<222> (438)..(438)
<223> any kind of base

<220>

<221> misc_feature
<222> (443)..(443)
<223> any kind of base

<220>
<221> misc_feature
<222> (459)..(459)
<223> any kind of base

<400> 38
taaggtttta taattatttt tatttttctt ttcttttttt tttatggctt ggatgacact 60
ttattttcag atccaatact agaagttgtt tccatgttca cattttcctt cctggnttaa 120
aaaaaagagt tgtatttttt ttttttgctt tttttaaatt atactttaag ttttagggta 180
catgtgcaca acgcagnggt tagctacata tgtatacatg tgccatgttg gcgtgctgca 240
tccagtaact cgtcatttaa cattaggtat atctccaaat gctatccttc cccccattgt 300
atttttcata gcttaaaaat cattgacata ggantaattc caactaaagt acggtattaa 360
atccctgggg gaataaattt tgtcttaaca agggtaaggt tngtgaaaag gatggttttg 420
tcacagggna aaaggganat ccncccattt taaaacccnc ct 462

<210> 39
<211> 1490
<212> DNA
<213> human

<400> 39
ctcgtgcccc ccacggaggg gactgctctc ccccgctgca tcctttctgt gaggtacctt 60
acccacctca gcacctgaga gggtgaaata gaattctaac ctcgacattc gggaagtgtt 120
tttgagaagt ctcggtcggt aagggaagtc ttccaagtcc gtgcagcact aacgtattgg 180
cacctgcctc ctcttcggcc accccccaga tgaggcagct gtgactgtgt caagggaagc 240
cacgactctg accatagtct tctctcagct tccactgccg tctccacagg aaacccagaa 300
gttctgtgaa caagtccatg ctgccatcaa ggcatttatt gcagtgtact atttgcttcc 360
aaaggatcag gggatcaccc tgagaaagct ggtacggggc gccaccctgg acatcgtgga 420
tggcatggct cagctcatgg aagtactttc cgtcactcca actcagagcc ctgagaacaa 480
tgaccttatt tcctacaaca gtgtctgggt tgcgtgccag cagatgcctc agataccaag 540
agataacaaa gctgcagctc ttttgatgct gaccaagaat gtggattttg tgaaggatgc 600
acatgaagaa atggagcagg ctgtggaaga atgtgaccct tactctggcc tcttgaatga 660
tactgaggag aacaactctg acaaccacaa tcatgaggat gatgtgttgg ggtttcccag 720
caatcaggac ttgtattggt cagaggacga tcaagagctc ataatcccat gccttgcgct 780
ggtgagagca tccaaagcct gcctgaagaa aattcggatg ttagtggcag agaatgggaa 840
gaaggatcag gtggcacagc tggatgacat tgtggatatt tctgatgaaa tcagccctag 900
tgtggatgat ttggctctga gcatatatcc acctatgtgt cacctgaccg tgcgaatcaa 960
ttctgcgaaa cttgtatctg ttttaaagaa ggcacttgaa attacaaaag caagtcatgt 1020

gacccctcag ccagaagata gttggatccc tttacttatt aatgccattg atcattgcat 1080
gaatagaatc aaggagctca ctcagagtga acttgaatta tgacttttca ggctcatttg 1140
tactctcttc ccctctcatc gtcatggtca ggctctgata cctgctttta aaatggagct 1200
agaatgcttg ctggattgaa agggagtgcc tatctatatt tagcaagaga cactattacc 1260
aaagattgtt ggttaggcca gattgacacc tatttataaa ccatatgcgt atatttttct 1320
gtgctatata tgaaaaataa ttgcatgatt tctcattcct gagtcatttc tcagagattc 1380
ctaggaaagc tgccttattc tctttttgca gtaaagtatg ttgttttcat tgtaaagatg 1440
ttgatggtct caataaaatg ctaacttgcc agtgattaaa aaaaaaaaaa 1490

<210> 40
<211> 1677
<212> DNA
<213> human

<400> 40
cttgacccta tttatagtgg ctctaaaggt ggtgttatta tgttttctag agcacttcga 60
ttatacaaac gtcaaggaat ccgagttaat gtgctttgcc ctgagtttgt tgaaacagac 120
atgggcacaa tgatcggtcc caaattcctt agtatgatgg ggggctttgt acctatggaa 180
atggtggtga aaggtgcttt tgagctcatc actgatgaga ataaagccgg cgattgccta 240
tggattacta atcggcgagg tcttgagtac tggcccaccc catcagaaga agcaaagtac 300
ttgctgcgtt ctacacgttc caggagaaga actgaataca aagctccacc aattaaacta 360
cctgagagtt ttgagaaaat agttgttcag accttgactc acaactttcg gaatgctacc 420
agtgtagtaa gagcaccact gagattacct atcaaaccaa actatgttct tgtgaagata 480
atctatgctg gtgtaaatgc tagtgatgta aattttagct caggtcgcta ttttggtggc 540
aataacagtg acactgcatc ccgtcttccg tttgatgcag gatttgaggc tgtgggagta 600
attgcagcag ttggggattc tgttactgac ttgaaagttg gcatgccttg tgcgttcatg 660
acttttggag gctatgctga atttacaatg attccttcga aatacgccct tccaatgcct 720
agaccagaac cggaaggtgt tgccatgctt acatcaggat taacagcttc aattgctcta 780
gaaaaggcag gacagatgga atctggaaaa gtggtccttg ttactgctgc ggcaggagga 840
actggtcagt ttgctgttca gcttgcaaaa ttagctggta ataccgtggt tgccacttgt 900
ggaggtgggg caaaggccaa gcttctgaaa gaattgggag tcgacagagt catagactat 960
cacagtgaag atataaaaac ggttctaagg aaagagttcc cgaaaggtat tgatatcatc 1020
tacgaatctg ttggtgggga catgttaaag ttgtgcttgg atgctttggc agtccatgga 1080
cgactcattg tcattggcat gatttctcag tatcaaggag aaaatggttg gacgccatca 1140
aaatatcctg gactatgtga gaagctcttg tcaaagagtc aaactgtggc tggctttttc 1200
ctggtgcaat atagtcacat gtaccaagaa caccttaaca agttatttga cctttactct 1260
tccggaaaac taaaggttgc tgtggatcca aagagattta taggccttca ttctgttgct 1320

gatgctgttg agtatctcca ttcaggcaaa agcgttggga aggtggttgt ctgcgtggac 1380
ccgaccttcg gtcatcaagt agccaaatta tgaatgaaca cggtgtcaaa tacagaaaga 1440
agtgaagttt tcaattctta gtctagagat tgttctcgaa tgttactgaa aatagctgct 1500
agaccagtgc tggaatattt attctcaatg ctttttcaat tttggattac ttgaaagaat 1560
aatccattta tgtataccat gtttatgttt acactataca acaactatga gcagaagaaa 1620
gcgagatatc tacaaaataa attataatcc tttcatttta aaaaaaaaaa aaaaaaa 1677

<210> 41
<211> 1330
<212> DNA
<213> human

<400> 41
atggcgcagt gggacagctt cactgatcaa caggaggaca ctgatagctg ttcagaatct 60
gtgaagtttg atgctcgctc caatacagct ttgcttcccc caaatcctaa aaatggccct 120
ccacttcaag aaaagctgaa atccttcaaa gctgcactga ttgcccttta tctccttgtg 180
tttgctgttc tcattcctat catcgcaata atggcagctc aactcctgaa gtgggaaatg 240
aagaattgca cagttggttc aattaatgca aacagtgtat cctccagtct cctgggaaga 300
ggaaatgaca gtgaagatga agtgagattt cgagaagttg ttatggaaca cattagcaag 360
atggagaaaa gaatccaata tatttcagat actgaagaaa atctcgtaga ttcagagcat 420
tttcaaaatt tcagtgtgac aactgatcaa cgatttgctg atgttcttct ccaactaagt 480
accttggttc ccacagtcca gggacatggg aatgccgtag atgaaatcac caggtcctta 540
ataagtctga ataccacgct gcttgatttg cacctctatg tagaaacact gaatgtcaaa 600
ttccaggaga atacacttaa agggcaagag gaaatcagca aattaaagga gcgtgtgcac 660
aatgcatcag cagaaattat gtctatgaaa gaagaacaag tgcatttgga acaggaaata 720
aaaagagaag tgaaagtcct gaataacatc actaatgatc tcaggctgaa agattgggaa 780
cattctcaga cgttgagaaa tatcacttta attcaaggtc ctcctggacc cccaggagaa 840
aaaggagata gaggtccaac cggagaaagt ggtccaccag gcgttccagg tccagtaggt 900
cctccaggtc ttaagggtga tcgaggatct attggctttc cgggaagtcg aggatatcca 960
ggacaatcag ggaagactgg gaggacagga tatcctggac caaaaggcca aaagggagaa 1020
aaaggcagtg gaagcatcct gactccttct gcgactgtcc gactggttgg tggccgtggc 1080
cctcatgagg gtagagtgga gatattgcac aatggacagt ggggcacagt ttgtgatgat 1140
cactgggaac tgcgtgccgg gcaggttgtc tgcaggagct tgggataccg aggtgttaag 1200
agtgtgcaca agaaagctta ttttggacaa ggtactggtc ccatttggct gaatgaagta 1260
ccctgtttgg ggatggagtc atccattgaa gagtgcaaaa tcagacagtg gggcgtgaga 1320
gtctgttcac 1330

<210> 42
<211> 431

<212> DNA
<213> human

<220>
<221> misc_feature
<222> (97)..(97)
<223> any kind of base

<220>
<221> misc_feature
<222> (347)..(347)
<223> any kind of base

<220>
<221> misc_feature
<222> (349)..(349)
<223> any kind of base

<220>
<221> misc_feature
<222> (361)..(361)
<223> any kind of base

<220>
<221> misc_feature
<222> (362)..(362)
<223> any kind of base

<220>
<221> misc_feature
<222> (363)..(363)
<223> any kind of base

<220>
<221> misc_feature
<222> (401)..(401)
<223> any kind of base

<220>
<221> misc_feature
<222> (428)..(428)
<223> any kind of base

<400> 42
cttttatat ttattttcat cgctacacaa acatttttta ggagtttgat tctacctcca 60

ttttggttag atatacaaac tctaccccat gagggantgt atggtgtatt tctagattta 120

gcaacaattt tcttgaaaaa tgtacaatac tatagaaaaa tgaagatagt aaataccagg 180

tataagttaa taacagtgtt tcttttgttc agtaataatg aactgtgtac tagcactgaa 240

ctttaggccc tcctatttgc gtattttctg tttgtatatt tttaaataga ggaattgtga 300

ttataatatt attattttgg aatatcctaa atcataaatt caaaacntna tttagttttt 360

nnnttttttt tttaagatgg agtcccgctt tgtcccaggc nggagtgcag tggcatgatc 420

tcagctcnct g 431

<210> 43
<211> 669
<212> DNA
<213> human

<220>
<221> misc_feature
<222> (641)..(641)
<223> any kind of base

<400> 43
ttcttttgga aaaccaaaca tgctttattt cattttttc acaatttatt taaacatctc 60

acatatacaa aataggtaca atttaatttt tctgcttgcc caagaaacaa agcttctgtg 120

gaaccatgga agaagatgaa aatgagactg gcaaagaaca aatgctgaat ctgaagaaga 180

ggacaacttt gggcaaataa tctgcatact tttaattggg aataagatgg aaaatatgaa 240

tgctaaatca aattttttaa aaaatacacc acacgataca actcaataca ggagtatttc 300

ttctcaaatt cttctagcac catcaacatt cttcaagtat ctgaaatact attaattagc 360

acctttgtat tatgaacaaa acaaaacaag gacctcagtt catctctgtc taggtcagca 420

cctaacaatg tggatcacac tcatgggaaa gtgttttgag gtagtttaaa cctttggaag 480

tttgggtttt aaacttccct ctgtggaaga tattcaaaag ccacaagtgg tgcaaatgtt 540

tatggttttt atttttcaat ttttattttg gttttcttac aaaggttgac atttttcata 600

acaggtgtaa gagtgttgaa aaaaaaattt caatttttgg ngggaacggg ggaaggagtt 660

aatgaaact 669

<210> 44
<211> 287
<212> DNA
<213> human

<400> 44
gccggagagt ctacaatgtt acccagcatg ctgttggcat tgttgtaaac aaacaagtta 60

agggcaagat tcttgccaag agaattaatg tgcgtattga gcacattaag cactctaaga 120

gccgagatag cttcctgaaa cgtgtgaagg aaaatgatca gaaaaagaaa gaagccaaag 180

agaaaggtac ctgggttcaa ctaaagcgcc acgctgctcc acccagagaa gcacactttg 240

tgagaaccaa tgggaaggag cctgagctgc tggaacctat tccctat 287

<210> 45
<211> 383
<212> DNA
<213> human

<220>
<221> misc_feature
<222> (147)..(147)
<223> any kind of base

<220>
<221> misc_feature

<222> (309)..(309)
<223> any kind of base

<220>
<221> misc_feature
<222> (349)..(349)
<223> any kind of base

<220>
<221> misc_feature
<222> (365)..(365)
<223> any kind of base

<220>
<221> misc_feature
<222> (372)..(372)
<223> any kind of base

<220>
<221> misc_feature
<222> (380)..(380)
<223> any kind of base

<400> 45
ggaacggaaa aggagaattc aagtgtgacc ctcatgaggc aacgtgttat gatgatggga 60

agacatacca cgtaggagaa cagtggcaga aggaatatct cggtgccatt tgctcctgca 120

catgctttgg aggccagcgg ggctcgnctt gtgacaactg ccgcagacct ggggggtgaa 180

cccagtcccg aaggcactac tggccagtcc tacaaccagt attcttcaga gataccattc 240

agagaacaaa cactaatgtt taatttgccc aatttgagtg cttcatgcct tttaggatgt 300

tacaggctng acagagaagg ttttcccgag gagttaaatc atctttttnc catttcccga 360

ggggncaagg cntgtttttn ttt 383

<210> 46
<211> 523
<212> DNA
<213> human

<400> 46
cagaggggca gggcggacgg ctaggagttc aagaaacatc ctggtctgag ggaaaggctg 60

cagctgcacc gccatgaata agcttttcag cttctggaag aggaagaatg agacccgcag 120

ccagggctac aaccttcgag aaaaggattt aaagaaactt cacagagctg cttcagtcgg 180

ggatttgaag aagctgaagg aataccttca gatcaagaaa tatgatgtaa atatgcagga 240

ctatgaatac agaacacctt tgcacctagc ctgtgctaat ggacatacag atgttgtact 300

tttcctaatt gagcaacaat gcaagataaa tgtccgggat agtgaaaaca aatccccatt 360

gattaaggca gtacagtgtc aaaatgagga ttgtgctact attctgctaa actttggtgc 420

agacccagat ctgagggata ttcgttataa tactgttctt cactatgctg tttgtggtca 480

aagtttgtca ttagttgaaa aactgcttga atacgaagct gat 523

<210> 47
<211> 390
<212> DNA
<213> human

<400> 47
tccaaggtca tggcaaaaca tctgaagttc atcgccagga ctgtgatggt acaggaaggg 60

aacgtggaaa gcgcatacag gaccctaaac agaatcctca ctatggatgg gctcattgag 120

gacattaagc atcggcggta ttatgagaag ccatgccgcc gcgacagagg gaaagctatg 180

aaaggtgccg gcggatctac aacatggaaa tggctcgcaa gatcaacttc ttgatgcgaa 240

agaatcgggc agatccgtgg cagggctgct gaggcctgtg ggtgggacac cagtgcgaaa 300

ccctcatcca gttttctctc catctctttt ctttgtacaa tcccatttcc tattaccatt 360

ctctgcaata aactcaaatc acatgtctgc 390

<210> 48
<211> 669
<212> DNA
<213> human

<220>
<221> misc_feature
<222> (641)..(641)
<223> any kind of base

<400> 48
ttcttttgga aaaccaaaca tgctttattt catttttttc acaatttatt taaacatctc 60

acatatacaa aataggtaca atttaatttt tctgcttgcc caagaaacaa agcttctgtg 120

gaaccatgga agaagatgaa aatgagactg gcaaagaaca aatgctgaat ctgaagaaga 180

ggacaacttt gggcaaataa tctgcatact tttaattggg aataagatgg aaaatatgaa 240

tgctaaatca aattttttaa aaaatacacc acacgataca actcaataca ggagtatttc 300

ttctcaaatt cttctagcac catcaacatt cttcaagtat ctgaaatact attaattagc 360

acctttgtat tatgaacaaa acaaaacaag gacctcagtt catctctgtc taggtcagca 420

cctaacaatg tggatcacac tcatgggaaa gtgttttgag gtagtttaaa cctttggaag 480

tttgggtttt aaacttccct ctgtggaaga tattcaaaag ccacaagtgg tgcaaatgtt 540

tatggttttt atttttcaat ttttattttg gttttcttac aaaggttgac atttttcata 600

acaggtgtaa gagtgttgaa aaaaaaattt caatttttgg ngggaacggg ggaaggagtt 660

aatgaaact 669

<210> 49
<211> 431
<212> DNA
<213> human

<220>
<221> misc_feature
<222> (97)..(97)
<223> any kind of base

<220>
<221> misc_feature
<222> (347)..(347)
<223> any kind of base

<220>
<221> misc_feature
<222> (349)..(349)
<223> any kind of base

<220>
<221> misc_feature
<222> (361)..(361)
<223> any kind of base

<220>
<221> misc_feature
<222> (362)..(362)
<223> any kind of base

<220>
<221> misc_feature
<222> (363)..(363)
<223> any kind of base

<220>
<221> misc_feature
<222> (401)..(401)
<223> any kind of base

<220>
<221> misc_feature
<222> (428)..(428)
<223> any kind of base

<400> 49
ctttttatat ttattttcat cgctacacaa acatttttta ggagtttgat tctacctcca 60

ttttggttag atatacaaac tctaccccat gagggantgt atggtgtatt tctagattta 120

gcaacaattt tcttgaaaaa tgtacaatac tatagaaaaa tgaagatagt aaataccagg 180

tataagttaa taacagtgtt tcttttgttc agtaataatg aactgtgtac tagcactgaa 240

ctttaggccc tcctatttgc gtattttctg tttgtatatt tttaaataga ggaattgtga 300

ttataatatt attattttgg aatatcctaa atcataaatt caaaacntna tttagttttt 360

nnnttttttt tttaagatgg agtcccgctt tgtcccaggc nggagtgcag tggcatgatc 420

tcagctcnct g 431

## Description

## BACKGROUND

This application claims the benefit of U.S Provisional Application No. 60/368,687 filed on March 29, 2002.

This invention relates to diagnostics and prognostics for colorectal cancer based on the gene expression profiles of biological samples.

Colorectal cancer is a heterogenous disease, consisting of tumors thought to emerge through three major molecular mechanisms: 1) mutations in the adenomatous polyposis coli (APC) gene, or the β-catenin gene, combined with chromosomal instability, 2) mutations in DNA mismatch repair genes, such as MLH1, MSH2, PMS1, PMS2 and MSH6, associated with microsatellite instability and mutations in genes containing short repeats, and 3) gene silencing induced by hypermethylation of the promoter regions of tumor suppressor genes. The genetic complement of individual colorectal cancers is likely to include different combinations of genetic instability, specific mutations, and gene silencing. Chromosomal instability (CIN) is a common feature of cancers in general. It implies an aneuploid phenotype, in which whole chromosomes or large parts of them are being lost or gained. Microsomal instability (MIN) is found in diploid tumors with an increased mutation rate in short repeats. Both forms of genetic instability are common in colorectal cancer.

Colorectal cancers thus have complex origins and involve a number of interactions in different biological pathways. Serum markers, histological, and cytological examinations historically used to assist in providing diagnostic, prognostic, or therapy monitoring decisions often do not have desired reliability. Likewise, while use of a single genetic marker (e.g., increased expression of a particular gene) may be beneficial, the diversity of the cancers make it more likely that a portfolio of genetic markers is the best approach.

## SUMMARY OF THE INVENTION

The invention is a method of assessing the presence or absence of colorectal cancer or the likely condition of a person believed to have colorectal cancer. In the method, a gene expression profile of a patient sample is analyzed to determine whether a patient has a colorectal cancer, whether a patient does not have colorectal cancer, whether a patient is likely to get colorectal cancer, or the response to treatment of a patient being treated for colorectal cancer.

Articles used in practising the methods are also an aspect of the invention. Such articles include gene expression profiles or representations of them that are fixed in machine-readable media such as computer readable media.

Articles used to identify gene expression profiles can also include substrates or surfaces, such as microarrays, to capture and/or indicate the presence, absence, or degree of gene expression.

## DETAILED DESCRIPTION

The mere presence or absence of particular nucleic acid sequences in a tissue sample has only rarely been found to have diagnostic or prognostic value. Information about the expression of various proteins, peptides or mRNA, on the other hand, is increasingly viewed as important. The mere presence of nucleic acid sequences having the potential to express proteins, peptides, or mRNA ( such sequences referred to as "genes") within the genome by itself is not determinative of whether a protein, peptide, or mRNA is expressed in a given cell. Whether or not a given gene capable of expressing proteins, peptides, or mRNA does so and to what extent such expression occurs, if at all, is determined by a variety of complex factors. Irrespective of difficulties in understanding and assessing these factors, assaying gene expression can provide useful information about the occurrence of important events such as tumerogenesis, metastasis, apoptosis, and other clinically relevant phenomena. Relative indications of the degree to which genes are active or inactive can be found in gene expression profiles. The gene expression profiles of this invention are used to diagnose and treat patients for colorectal cancer.

Sample preparation requires the collection of patient samples. Patient samples used in the inventive method are those that are suspected of containing diseased cells such as epithelial cells taken from a colon sample or from surgical margins. One useful technique for obtaining suspect samples is Laser Capture Microdisection (LCM). LCM technology provides a way to select the cells to be studied, minimizing variability caused by cell type heterogeneity. Consequently, moderate or small changes in gene expression between normal and cancerous cells can be readily detected. In a preferred method, the samples comprise circulating epithelial cells extracted from peripheral blood. These can be obtained according to a number of methods but the most preferred method is the magnetic separation technique described in U.S. Patent 6,136,182 assigned to Immunivest Corp which is incorporated herein by reference. Once the sample containing the cells of interest has been obtained, RNA is extracted and amplified and a gene expression profile is obtained, preferably via micro-array, for genes in the appropriate portfolios.

Preferred methods for establishing gene expression profiles include determining the amount of RNA that is produced by a gene that can code for a protein or peptide. This is accomplished by reverse transcriptase PCR (RT-PCR), competitive RT-PCR, real time RT-PCR, differential display RT-PCR, Northern Blot analysis and other related tests. While it is possible to conduct these techniques using individual PCR reactions, it is best to amplify complimentary DNA (cDNA) or complimentary RNA (cRNA) produced from mRNA and analyze it via microarray. A number of different array configurations and methods for their production are known to those of skill in the art and are described in U.S. Patents such as: 5,445,934; 5,532,128; 5,556,752; 5,242,974; 5,384,261; 5,405,783; 5,412,087; 5,424,186; 5,429,807; 5,436,327; 5,472,672; 5,527,681; 5,529,756; 5,545,531; 5,554,501; 5,561,071; 5,571,639; 5,593,839; 5,599,695; 5,624,711; 5,658,734; and 5,700,637; the disclosures of which are incorporated herein by reference.

Microarray technology allows for the measurement of the steady-state mRNA level of thousands of genes simultaneously thereby presenting a powerful tool for identifying effects such as the onset, arrest, or modulation of uncontrolled cell proliferation. Two microarray technologies are currently in wide use. The first are cDNA arrays and the second are oligonucleotide arrays. Although differences exist in the construction of these chips, essentially all downstream data analysis and output are the same. The product of these analyses are typically measurements of the intensity of the signal received from a labeled probe used to detect a cDNA sequence from the sample that hybridizes to a nucleic acid sequence at a known location on the microarray. Typically, the intensity of the signal is proportional to the quantity of cDNA, and thus mRNA, expressed in the sample cells. A large number of such techniques are available and useful. Preferred methods for determining gene expression can be found in US Patents 6,271,002 to Linsley, et al.; 6,218,122 to Friend, et al.; 6,218,114 to Peck, et al.; and 6,004,755 to Wang, et al., the disclosure of each of which is incorporated herein by reference.

Analysis of the expression levels is conducted by comparing such intensities. This is best done by generating a ratio matrix of the expression intensities of genes in a test sample versus those in a control sample. For instance, the gene expression intensities from a diseased tissue can be compared with the expression intensities generated from normal tissue of the same type (e.g., diseased colon tissue sample vs. normal colon tissue sample). A ratio of these expression intensities indicates the fold-change in gene expression between the test and control samples.

Gene expression profiles can also be displayed in a number of ways. The most common method is to arrange a raw fluorescence intensities or ratio matrix into a graphical dendogram where columns indicate test samples and rows indicate genes. The data is arranged so genes that have similar expression profiles are proximal to each other. The expression ratio for each gene is visualized as a color. For example, a ratio less than one (indicating down-regulation) may appear in the blue portion of the spectrum while a ratio greater than one (indicating up-regulation) may appear as a color in the red portion of the spectrum. Commercially available computer software programs are available to display such data including "GENESPRINT" from Silicon Genetics, Inc. and "DISCOVERY" and "INFER" software from Partek, Inc.

Modulated genes used in the methods of the invention are shown in Table 1. The genes that are differentially expressed are shown as being either up regulated or down regulated in diseased cells. Up regulation and down regulation are relative terms meaning that a detectable difference (beyond the contribution of noise in the system used to measure it) is found in the amount of expression of the genes relative to some baseline. In this case, the baseline is the measured gene expression of a normal cell. The genes of interest in the diseased cells are then either up regulated or down regulated relative to the baseline level using the same measurement method. Diseased, in this context, refers to an alteration of the state of a body that interrupts or disturbs, or has the potential to disturb, proper performance of bodily functions as occurs with the uncontrolled proliferation of cells. Someone is diagnosed with a disease when some aspect of that person's genotype or phenotype is consistent with the presence of the disease. However, the act of conducting a diagnosis or prognosis includes the determination disease/status issues such as therapy monitoring. In therapy monitoring, clinical judgments are made regarding the effect of a given course of therapy by comparing the expression of genes over time to determine whether the gene expression profiles have changed or are changing to patterns more consistent with normal tissue.

Preferably, levels of up and down regulation are distinguished based on fold changes of the intensity measurements of hybridized microarray probes. A 2.0 fold difference is preferred for making such distinctions or a p-value less than .05. That is, before a gene is said to be differentially expressed in diseased versus normal cells, the diseased cell is found to yield at least 2 more, or 2 times less intensity than the normal cells. The greater the fold difference, the more preferred is use of the gene as a diagnostic. Genes selected for the gene expression profiles of the instant invention have expression levels that result in the generation of a signal that is distinguishable from those of the normal or non-modulated genes by an amount that exceeds background using clinical laboratory instrumentation.

Statistical values can be used to confidently distinguish modulated from non-modulated genes and noise. Statistical tests find the genes most significantly different between diverse groups of samples. The Student's t-test is an example of a robust statistical test that can be used to find significant differences between two groups. The lower the p-value, the more compelling the evidence that the gene is showing a difference between the different groups. Nevertheless, since microarrays measure more than one gene at a time, tens of thousands of statistical tests may be asked at one time. Because of this, there is likelihood to see small p-values just by chance and adjustments for this using a Sidak correction as well as a randomization/permutation experiment can be made. A p-value less than .05 by the t-test is evidence that the gene is significantly different. More compelling evidence is a p-value less then .05 after the Sidak correct is factored in. For a large number of samples in each group, a p-value less than 0.05 after the randomization/permutation test is the most compelling evidence of a significant difference.

Another parameter that can be used to select genes that generate a signal that is greater than that of the non-modulated gene or noise is the use of a measurement of absolute signal difference. Preferably, the signal generated by the modulated gene expression is at least 20% different than those of the normal or non-modulated gene (on an absolute basis). It is even more preferred that such genes produce expression patterns that are at least 30% different than those of normal or non-modulated genes.

Genes can be grouped so that information obtained about the set of genes in the group provides a sound basis for making a clinically relevant judgment such as a diagnosis, prognosis, or treatment choice. These sets of genes make up the portfolios of the invention. In this case, the judgments supported by the portfolios involve colorectal cancer. Portfolios of gene expression profiles can be comprised of combinations of genes described in Example 3. As with most diagnostic markers, it is often desirable to use the fewest number of markers sufficient to make a correct medical judgment. This prevents a delay in treatment pending further analysis as well inappropriate use of time and resources. In this case, such a minimal portfolio can be comprised of a combination of genes from Example 4.

Preferably, portfolios are established such that the combination of genes in the portfolio exhibit improved sensitivity and specificity relative to individual genes or randomly selected combinations of genes. In the context of the instant invention, the sensitivity of the portfolio can be reflected in the fold differences exhibited by a gene's expression in the diseased state relative to the normal state. Specificity can be reflected in statistical measurements of the correlation of the signaling of gene expression with the condition of interest. For example, standard deviation can be a used as such a measurement. In considering a group of genes for inclusion in a portfolio, a small standard deviation in expression measurements correlates with greater specificity. Other measurements of variation such as correlation coefficients can also be used in this capacity.

The most preferred method of establishing gene expression portfolios is through the use of optimization algorithms such as the mean variance algorithm widely used in establishing stock portfolios. This method is described in detail in the co-pending patent application entitled "Portfolio Selection" by Tim Jatkoe, et. al., of equal date hereto. Essentially, the method calls for the establishment of a set of inputs (stocks in financial applications, expression as measured by intensity here) that will optimize the return (e.g., signal that is generated) one receives for using it while minimizing the variability of the return. Many commercial software programs are available to conduct such operations. "Wagner Associates Mean-Variance Optimization Application", referred to as "Wagner Software" throughout this specification, is preferred. This software uses functions from the "Wagner Associates Mean-Variance Optimization Library" to determine an efficient frontier and optimal portfolios in the Markowitz sense is preferred.

Use of this type of software requires that microarray data be transformed so that it can be treated as an input in the way stock return and risk measurements are used when the software is used for its intended financial analysis purposes. For example, when Wagner Software is employed in conjunction with microarray intensity measurements the following data transformation method is employed.

Genes are first pre-selected by identifying those genes whose expression shows at least some minimal level of differentiation. The preferred pre-selection process is conducted as follows. A baseline class is selected. Typically, this will comprise genes from a population that does not have the condition of interest. For example, if one were interested in selecting a portfolio of genes that are diagnostic for breast cancer, samples from patients without breast cancer can be used to make the baseline class. Once the baseline class is selected, the arithmetic mean and standard deviation is calculated for the indicator of gene expression of each gene for baseline class samples. This indicator is typically the fluorescent intensity of a microarray reading. The statistical data computed is then used to calculate a baseline value of (X*Standard Deviation + Mean) for each gene. This is the baseline reading for the gene from which all other samples will be compared. X is a stringency variable selected by the person formulating the portfolio. Higher values of X are more stringent than lower. Preferably, X is in the range of .5 to 3 with 2 to 3 being more preferred and 3 being most preferred.

Ratios between each experimental sample (those displaying the condition of interest) versus baseline readings are then calculated. The ratios are then transformed to base 10 logarithmic values for ease of data handling by the software. This enables down regulated genes to display negative values necessary for optimization according to the Markman mean-variance algorithm using the Wagner Software.

The preprocessed data comprising these transformed ratios are used as inputs in place of the asset return values that are normally used in the Wagner Software when it is used for financial analysis purposes.

Once an efficient frontier is formulated, an optimized portfolio is selected for a given input level (return) or variance that corresponds to a point on the frontier. These inputs or variances are the predetermined standards set by the person formulating the portfolio. Stated differently, one seeking the optimum portfolio determines an acceptable input level (indicative of sensitivity) or a given level of variance (indicative of specificity) and selects the genes that lie along the efficient frontier that correspond to that input level or variance. The Wagner Software can select such genes when an input level or variance is selected. It can also assign a weight to each gene in the portfolio as it would for a stock in a stock portfolio.

Determining whether a sample has the condition for which the portfolio is diagnostic can be conducted by comparing the expression of the genes in the portfolio for the patient sample with calculated values of differentially expressed genes used to establish the portfolio. Preferably, a portfolio value is first generated by summing the multiples of the intensity value of each gene in the portfolio by the weight assigned to that gene in the portfolio selection process. A boundary value is then calculated by (Y*standard deviation + mean of the portfolio value for baseline groups) where Y is a stringency value having the same meaning as X described above. A sample having a portfolio value greater than the portfolio value of the baseline class is then classified as having the condition. If desired, this process can be conducted iteratively in accordance with well known statistical methods for improving confidence levels.

Optionally one can reiterate this process until best prediction accuracy is obtained.

The process of portfolio selection and characterization of an unknown is summarized as follows:

1. Choose baseline class.
2. Calculate mean, and standard deviation of each gene for baseline class samples.
3. Calculate (X*Standard Deviation + Mean) for each gene. This is the baseline reading from which all other samples will be compared. X is a stringency variable with higher values of X being more stringent than lower.
4. Calculate ratio between each Experimental sample versus baseline reading calculated in step 3.
5. Transform ratios such that ratios less than 1 are negative (eg.using Log base 10). (Down regulated genes now correctly have negative values necessary for MV optimization).
6. These transformed ratios are used as inputs in place of the asset returns that are normally used in the software application.
7. The software will plot the efficient frontier and return an optimized portfolio at any point along the efficient frontier.
8. Choose a desired return or variance on the efficient frontier.
9. Calculate the Portfolio's Value for each sample by summing the multiples of each gene's intensity value by the weight generated by the portfolio selection algorithm.
10. Calculate a boundary value by adding the mean Portfolio Value for Baseline groups to the multiple of Y and the Standard Deviation of the Baseline's Portfolio Values. Values greater than this boundary value shall be classified as the Experimental Class.
11. Optionally one can reiterate this process until best prediction accuracy is obtained.

Alternatively, genes can first be pre-selected by identifying those genes whose expression shows some minimal level of differentiation. The pre-selection in this alternative method is preferably based on a threshold given by

$$1 \leq \left|\frac{(\mu_t - \mu_n)}{(\sigma_t + \sigma_n)}\right|,$$

where $\mu_t$ is the mean of the subset known to possess the disease or condition, $\mu_n$ is the mean of the subset of normal samples, and $\sigma_t + \sigma_n$ represent the combined standard deviations. A signal to noise cutoff can also be used by pre-selecting the data according to a relationship such as

$$0.5 \leq \left|\frac{(\mu_t - MAX_n)}{(\sigma_t + \sigma_n)}\right|.$$

This ensures that genes that are pre-selected based on their differential modulation are differentiated in a clinically significant way. That is, above the noise level of instrumentation appropriate to the task of measuring the diagnostic parameters. For each marker pre-selected according to these criteria, a matrix is established in which columns represents samples, rows represent markers and each element is a normalized intensity measurement for the expression of that marker according to the relationship:

$$\left|\frac{(\mu_t - I)}{\mu_t}\right|$$

where I is the intensity measurement.

It is also possible to set additional boundary conditions to define the optimal portfolios. For example, portfolio size can be limited to a fixed range or number of markers. This can be done either by making data pre-selection criteria more stringent (e.g,

$$.8 \leq \left|\frac{(\mu_t - MAX_n)}{(\sigma_t + \sigma_n)}\right|$$

instead of

$$0.5 \leq \left|\frac{(\mu_t - MAX_n)}{(\sigma_t + \sigma_n)}\right|)$$

or by using programming features such as restricting portfolio size. One could, for example, set the boundary condition that the efficient frontier is to be selected from among only the most optimal 10 genes. One could also use all of the genes pre-selected for determining the efficient frontier and then limit the number of genes selected (e.g., no more than 10).

The process of selecting a portfolio can also include the application of heuristic rules. Preferably, such rules are formulated based on biology and an understanding of the technology used to produce clinical results. More preferably, they are applied to output from the optimization method. For example, the mean variance method of portfolio selection can be applied to microarray data for a number of genes differentially expressed in subjects with breast cancer. Output from the method would be an optimized set of genes that could include some genes that are expressed in peripheral blood as well as in diseased breast tissue. If sample used in the testing method are obtained from peripheral blood and certain genes differentially expressed in instances of breast cancer could also be differentially expressed in peripheral blood, then a heuristic rule can be applied in which a portfolio is selected from the efficient frontier excluding those that are differentially expressed in peripheral blood. Of course, the rule can be applied prior to the formation of the efficient frontier by, for example, applying the rule during data pre-selection.

Other heuristic rules can be applied that are not necessarily related to the biology in question. For example, one can apply the rule that only a given percentage of the portfolio can be represented by a particular gene or genes. Commercially available software such as the Wagner Software readily accommodates these types of heuristics. This can be useful, for example, when factors other than accuracy and precision (e.g., anticipated licensing fees) have an impact on the desirability of including one or more genes.

One method of the invention involves comparing gene expression profiles for various genes (or portfolios) to conduct diagnoses as described above. The gene expression profiles of each of the genes comprising the portfolio are fixed in a medium such as a computer readable medium. This can take a number of forms. For example, a table can be established into which the range of signals (e.g., intensity measurements) indicative of disease is input. Actual patient data can then be compared to the values in the table to determine whether the patient samples are normal or diseased. In a more sophisticated embodiment, patterns of the expression signals (e.g., flourescent intensity) are recorded digitally or graphically. The gene expression patterns from the gene portfolios used in conjunction with patient samples are then compared to the expression patterns. Pattern comparison software can then be used to determine whether the patient samples have a pattern indicative of the disease in question. Of course, these comparisons can also be used to determine whether the patient results are normal. The expression profiles of the samples are then compared to the portfolio of a normal or control cell. If the sample expression patterns are consistent with the expression pattern for a colorectal cancer then (in the absence of countervailing medical considerations) the patient is diagnosed as positive for colorectal cancer. If the sample expression patterns are consistent with the expression pattern from the normal/control cell then the patient is diagnosed negative for colorectal cancer.

Numerous well known methods of pattern recognition are available. The following references provide some examples:

<u>Weighted Voting</u>: Golub, TR., Slonim, DK., Tamaya, P., Huard, C., Gaasenbeek, M., Mesirov, JP., Coller, H., Loh, L., Downing, JR., Caligiuri, MA., Bloomfield, CD., Lander, ES. *Molecular classification of cancer: class discovery and class prediction by gene expression monitoring.* Science 286:531-537, 1999

<u>Support Vector Machines</u>: Su, AI., Welsh, JB., Sapinoso, LM., Kern, SG., Dimitrov, P., Lapp, H., Schultz, PG., Powell, SM., Moskaluk, CA., Frierson, HF. Jr., Hampton, GM. *Molecular classification of human carcinomas by use of gene expression signatures.* Cancer Research 61:7388-93, 2001 and

Ramaswamy, S., Tamayo, P., Rifkin, R., Mukherjee, S., Yeang, CH., Angelo, M., Ladd, C., Reich, M., Latulippe, E., Mesirov, JP., Poggio, T., Gerald, W., Loda, M., Lander, ES., Gould, TR. *Multiclass cancer diagnosis using tumor gene expression signatures* Proceedings of the National Academy of Sciences of the USA 98:15149-15154, 2001

K-nearest Neighbors: Ramaswamy, S., Tamayo, P., Rifkin, R., Mukherjee, S., Yeang, CH., Angelo, M., Ladd, C., Reich, M., Latulippe, E., Mesirov, JP., Poggio, T., Gerald, W., Loda, M., Lander, ES., Gould, TR. *Multiclass cancer diagnosis using tumor gene expression signatures* Proceedings of the National Academy of Sciences of the USA 98:15149-15154, 2001

Correlation Coefficients: van 't Veer LJ, Dai H, van de Vijver MJ, He YD, Hart AA, Mao M, Peterse HL, van der Kooy K, Marton MJ, Witteveen AT, Schreiber GJ, Kerkhoven RM, Roberts C, Linsley PS, Bernards R, Friend SH. Gene expression profiling predicts clinical outcome of breast cancer. Nature. 2002 Jan 31;415(6871):530-6.

The gene expression profiles of this invention can also be used in conjunction with other non-genetic diagnostic methods useful in cancer diagnosis, prognosis, or treatment monitoring. For example, in some circumstances it is beneficial to combine the diagnostic power of the gene expression based methods described above with data from conventional markers such as serum protein markers (e.g., carcinoembryonic antigen). A range of such markers exists including such analytes as CA19-9, CA 125, CK-BB, and Guanylyl Cyclase C. In one such method, blood is periodically taken from a treated patient and then subjected to an enzyme immunoassay for one of the serum markers described above. When the concentration of the marker suggests the return of tumors or failure of therapy, a sample source amenable to gene expression analysis is taken. Where a suspicious mass exists, a fine needle aspirate is taken and gene expression profiles of cells taken from the mass are then analyzed as described above. Alternatively, tissue samples may be taken from areas adjacent to the tissue from which a tumor was previously removed. This approach can be particularly useful when other testing produces ambiguous.

Combining the use of genetic markers with other diagnostics is most preferred when the reliability of the other diagnostic is suspect. For example, it is known that serum levels of CEA can be substantially affected by factors having nothing to do with a patient's cancer status. It can be beneficial to conduct a combination gene expression/CEA assay when a patient being monitored following treatment for colon cancer shows heightened levels of routine CEA assays.

Articles of this invention include representations of the gene expression profiles useful for treating, diagnosing, prognosticating, and otherwise assessing diseases. These profile representations are reduced to a medium that can be automatically read by a machine such as computer readable media (magnetic, optical, and the like). The articles can also include instructions for assessing the gene expression profiles in such media. For example, the articles may comprise a CD ROM having computer instructions for comparing gene expression profiles of the portfolios of genes described above. The articles may also have gene expression profiles digitally recorded therein so that they may be compared with gene expression data from patient samples. Alternatively, the profiles can be recorded in different representational format. A graphical recordation is one such format. Clustering algorithms such as those incorporated in "GENSPRING" and "DISCOVER" computer programs mentioned above can best assist in the visualization of such data.

Different types of articles of manufacture according to the invention are media or formatted assays used to reveal gene expression profiles. These can comprise, for example, microarrays in which sequence complements or probes are affixed to a matrix to which the sequences indicative of the genes of interest combine creating a readable determinant of their presence. Alternatively, articles according to the invention can be fashioned into reagent kits for conducting hybridization, amplification, and signal generation indicative of the level of expression of the genes of interest for detecting colorectal cancer.

Kits made according to the invention include formatted assays for determining the gene expression profiles. These can include all or some of the materials needed to conduct the assays such as reagents and instructions.

The invention is further illustrated by the following non-limiting examples.

**Examples:** Genes analyzed according to this invention are identified by reference to Gene ID Numbers in the GenBank database. These are typically related to full-length nucleic acid sequences that code for the production of a protein or peptide. One skilled in the art will recognize that identification of full-length sequences is not necessary from an analytical point of view. That is, portions of the sequences or ESTs can be selected according to well-known principles for which probes can be designed to assess gene expression for the corresponding gene.

## Example 1- Sample Handling and LCM.

Twenty-seven fresh frozen tissue samples were collected from patients who had surgery for a colorectal tumor. Nineteen of the samples were colorectal malignancy specimens, and eight of the samples were of normal colon mucosa. The tissues were snap frozen in liquid nitrogen within 20-30 minutes of harvesting, and stored at -80C° thereafter. For laser capture, the samples were cut (6μm), and one section was mounted on a glass slide, and the second on film (P.A.L.M.), which had been fixed onto a glass slide (Micro Slides Colorfrost, VWR Scientific, Media, PA). The section mounted on a glass slide was after fixed in cold acetone, and stained with Mayer's Haematoxylin (Sigma, St. Louis, MO). A pathologist analyzed the samples for diagnosis and grade. The clinical stage was estimated from the accompanying surgical pathology and clinical reports, using the Dukes classification. The section mounted on film was after fixed for five minutes in 100% ethanol, counter stained for 1 minute in eosin/100% ethanol (100μg of Eosin in 100ml of dehydrated ethanol), quickly soaked once in 100% ethanol to remove the free stain, and air dried for 10 minutes.

Two of the colorectal adenocarcinomas were of grade 1, 10 of grade 2, and 5 of grade 3. One of the malignant samples was a carcinoid tumor of the caecum, and one a metastatic melanoma lesion. Two of the adenocarcinoma samples represented the mucinous subtype, and one the signet cell subtype. The Dukes staging of the adenocarcinomas divided them as follows: Dukes A: 2, Dukes B: 5, Dukes C: 7, Dukes D: 3. Six of the adenocarcinomas had been irradiated preoperatively.

Before use in LCM, the membrane (LPC-MEMBRANE PEN FOIL 1.35 μm No 8100, P.A.L.M. GmbH Mikrolaser Technologie, Bernried, Germany) and slides were pretreated to abolish RNases, and to enhance the attachment of the tissue sample onto the film. Briefly, the slides were washed in DEP $H_2O$, and the film was washed in RNase AWAY (Molecular Bioproducts, Inc., San Diego, CA) and rinsed in DEP $H_2O$. After attaching the film onto the glass slides, the slides were baked at +120°C for 8 hours, treated with TI-SAD (Diagnostic Products Corporation, Los Angeles, CA, 1:50 in DEP $H_2O$, filtered through cotton wool), and incubated at +37°C for 30 minutes. Immediately before use, a 10μl aliquot of RNase inhibitor solution (Rnasin Inhibitor 2500U=33U/μl N211A, Promega GmbH, Mannheim, Germany, 0.5μl in 400μl of freezing solution, containing 0.15 mol NaCl, 10 mmol Tris pH 8.0, 0.25 mmol dithiothreitol) was spread onto the film, where the tissue sample was to be mounted.

The tissue sections mounted on film were used for LCM. Approximately 2000 epithelial cells/sample were captured using the PALM Robot-Microbeam technology (P.A.L.M. Mikrolaser Technologie, Carl Zeiss, Inc., Thornwood, NY), coupled into Zeiss Axiovert 135 microscope (Carl Zeiss Jena GmbH, Jena, Germany). The surrounding stroma in the normal mucosa, and the occasional intervening stromal components in cancer samples, were included. The captured cells were put in tubes in 100% ethanol and preserved at -80°C.

**Example 2- RNA Extraction and Amplification.**

Zymo-Spin Column (Zymo Research, Orange, CA 92867) was used to extract total RNA from the LCM captured samples. About 2 ng of total RNA was resuspended in 10 ul of water and 2 rounds of the T7 RNA polymerase based amplification were performed to yield about 50 ug of amplified RNA.

**Example 3- cDNA Microarray Hybridization and Quantitation.**

A set of cDNA microarrays consisting of approximately 20,000 human cDNA clones was used to test the samples. About 30 plant genes were also printed on the microarrays as a control for non-specific hybridization. Cy3-labeled cDNA probes were synthesized from 5 ug of aRNA of the LCM captured cells. The probes were purified with Qiagen's Nucleotide Removal Columns and then hybridized to the microarrays for 14-16 hours. The slides were washed and air-dried before scanning. cDNA microarrays were scanned for cy3 fluorescence and ImaGene software (Biodiscovery, Los Angeles, CA) was used for quantitation. For each cDNA clone, four measurements were obtained using duplicate spots and duplicate arrays and the intensities were averaged.

cDNAs were printed on amino silane-coated slides (Corning) with a Generation III Micro-array Spotter (Molecular Dynamics). The cDNAs were PCR amplified, purified (Qiagen PCR purification kit), and mixed 1:1 with 10 M NaSCN printing buffer. Prior to hybridization micro-arrays were incubated in isopropanol at room temperature for 10 min. The probes were incubated at 95°C for 2 min, at room temperature for 5 min, and then applied to three replicate slides. Cover slips were sealed onto the slides with DPX (Fluka) and incubated at 42°C overnight. Slides were then washed at 55°C for 5min in 1X SSC/0.2% SDS and 0.1X SSC/0.2% SDS, dipped in 0.1X SSC and dried before being scanned by a GenIII Array Scanner (Molecular Dynamics). The fluorescence intensity for each spot was analyzed with AUTOGENE software (Biodiscovery, Los Angeles).

Chip intensities were linearly normalized forcing the intensity reading at the $75^{th}$ percentile equivalent to a value of 100 on each chip. Every gene on the chip was normalized to itself by dividing the intensity reading for that gene by the median of the gene's expression value readings over all the samples. Prior to clustering, genes that did not have an intensity reading of 100 or greater in at least one sample were filtered out in order to limit the background affect on the similarity metrics. A set of 6,225 genes was selected for clustering analysis. Hierarchical clustering was performed using correlation as a measure of similarity, which groups together samples with genes that are showing positive changes at the same time without any consideration for negative changes (Silicon Genetics, Sunnyville, CA). Each of the major nodes in the dendrogram was then considered a subgroup of samples. Differentially expressed genes were identified by comparing each tumor subgroup to the normal group. The selection was based on a signal to noise measurement threshold given by

$$1 \leq \left|\frac{(\mu_t - \mu_n)}{(\sigma_t + \sigma_n)}\right|,$$

where $\mu_t$ is the mean of the tumor subset, $\mu_n$ is the mean of the subset of normal samples, and $\sigma_t + \sigma_n$ represent the combined standard deviations. The within-group coefficient of variation of the intensity readings of a gene had to be less than 0.33, for the gene to be included in the pair-wise comparisons. The median of the tumor group over the median of the normal group had to be greater than, or equal to 2 for up-regulation, and less than, or equal to 0.5 for down-regulation. If a gene met all the criteria, it was selected. The genes selected in all the comparisons were considered consistently dysregulated in colorectal cancer. The p-values for the statistical significance were calculated using a T-test assuming unequal variance. The gene set for clustering was also subjected to principal component analysis (PCA) using a software package (Partek, St Louis, MO). The data was then projected onto the reduced 3-dimensional space. The normal and tumor colorectal samples were represented by the projected expression levels.

A list of genes with large up-regulated differentials was created to distinguish between the tumor and normal samples. One-hundred and twenty-three genes were preselected by using

$$0.5 \leq \left|\frac{(\mu_t - MAX_n)}{(\sigma_t + \sigma_n)}\right|$$

as a signal to noise cutoff. A ratio equal to, or greater than 1.5 was the minimal criterion for up-regulation. Genes were also included if

$$0.9 \leq \left|\frac{(\mu_t - \mu_n)}{(\sigma_t + \sigma_n)}\right|.$$

A portfolio of four genes was established, each having at least a three fold expression differential between tumor and normal cells.

<u>Differentially Expressed Genes in Colorectal Cancer.</u>

Thirty-nine genes were differentially expressed in all tumor samples as compared to normal colon mucosa. Thirty-seven of them were significantly down-regulated in all the tumors, except for an outlier. Two of them were up-regulated. The identities of the genes were verified by sequencing the cDNA clones placed on the microarray. Results are shown in Table 1.

Table 1

| Modulated Genes | | | | | |
|---|---|---|---|---|---|
| **ACCESSION** | **GENE DESCRIPTION** | **MEAN SIGNAL INTENSITY (NORMAL)** | **MEAN SIGNAL INTENSITY (TUMOR)** | **P-VALUE** | |
| AF071569 | CaM kinase II gene subtype delta 2. | 93 | 39 | 4.64E-09 | Seq. ID No. 1 |
| AB014530 | Homo sapiens mRNA for KIAA0630 protein | 108 | 50 | 4.83E-07 | Seq. ID No.2 |
| AK000319 | Human cDNA KIAA0630 | 236 | 69 | 7.84E-06 | Seq. ID No.3 |
| U81504 | beta-3A-adaptin subunit of the AP-3 complex mRNA, | 241 | 75 | 3.52E-05 | Seq. ID No.4 |
| AB011166 | Human cDNA KIAA0594 | 116 | 55 | 3.53E-05 | Seq. ID No.5 |

Table 1 (continued)

| Modulated Genes | | | | | |
|---|---|---|---|---|---|
| **ACCESSION** | **GENE DESCRIPTION** | **MEAN SIGNAL INTENSITY (NORMAL)** | **MEAN SIGNAL INTENSITY (TUMOR)** | **P-VALUE** | |
| AB040914 | Human cDNA KIAA1481 | 187 | 59 | 8.85E-05 | Seq. ID No.6 |
| AK025205 | Human cDNA FLJ21552 | 322 | 97 | 0.00013 | Seq. ID No.7 |
| AJ278219 | Fatty acid hydroxylase | 143 | 53 | 0.00011 | Seq. ID No.8 |
| AB046854 | Human cDNA KIAA1634 | 142 | 59 | 0.00020 | Seq. ID No.9 |
| R00585 | Unknown | 149 | 57 | 1.28E-09 | Seq. ID No.10 |
| S45844 | Spi-B transcription factor | 140 | 43 | 0.00043 | Seq. ID No.11 |
| X98311 | Carcinoembryonic antigen family member 2 (CGM2) | 6137 | 223 | 0.00044 | Seq. ID No.12 |
| BAA78050 | NADPH oxidoreductase homolog | 153 | 84 | 0.00048 | Seq. ID No.40 |
| N72128 | Unknown | 164 | 77 | 0.00068 | Seq. ID No.13 |
| AB040955 | Human cDNA K1AA1552 | 334 | 120 | 0.00067 | Seq. ID No.14 |
| AF125101 | HSPC040 protein | 363 | 115 | 0.0011 | Seq. ID No.15 |
| AB023229 | Human cDNA KIAA1012 | 263 | 88 | 0.00099 | Seq. ID No.16 |
| N95761 | a-L-fucosidase gene | 429 | 104 | 0.00047 | Seq. ID No.17 |
| AK025033 | Human cDNA FLJ21380 | 180 | 85 | 0.0010 | Seq. ID No.18 |
| L10844 | Human cellular growth regulating protein | 206 | 101 | 0.0013 | Seq. ID No.19 |
| H96534 | H.sapiens mRNA for gp25L2 protein. | 147 | 58 | 0.0015 | Seq. ID No.20 |
| AK001521 | Human cDNA FLJ10659 | 157 | 60 | 0.0019 | Seq. ID No.21 |
| AF151039 | HSPC205 protein | 117 | 60 | 0.0017 | Seq. ID No.22 |
| AF052059 | SEL 1L protein | 168 | 53 | 0.0016 | Seq. ID No.23 |
| N24597 | Unknown | 166 | 62 | 0.0016 | Seq. ID No.24 |
| AK001950 | Inner centromere protein | 148 | 64 | 0.0029 | Seq. ID No.25 |

Table 1 (continued)

| Modulated Genes | | | | | |
|---|---|---|---|---|---|
| **ACCESSION** | **GENE DESCRIPTION** | **MEAN SIGNAL INTENSITY (NORMAL)** | **MEAN SIGNAL INTENSITY (TUMOR)** | **P-VALUE** | |
| BAA02649 | Macrophage scavenger receptor type I | 118 | 44 | 0.0031 | Seq. ID No.41 |
| N75004 | Unknown | 98 | 48 | 0.0031 | Seq. ID No.26 |
| W16916 | Human cDNA KIAA0260 | 162 | 61 | 0.0037 | Seq. ID No.27 |
| X52001 | H.sapiens endothelin 3 mRNA. | 89 | 33 | 0.0042 | Seq. ID No.28 |
| T50788 | Unknown | 364 | 102 | 0.0059 | Seq. ID No.38 |
| AJ005866 | Putative Sqv-7 like protein | 381 | 163 | 0.0049 | Seq. ID No.29 |
| AF113535 | MAID protein | 218 | 100 | 0.0053 | Seq. ID No.39 |
| AB037789 | Human cDNA KIAA1368 | 164 | 62 | 0.0068 | Seq. ID No.30 |
| M33987 | Carbonic anhydrase | 652 | 46 | 0.0074 | Seq. ID No.31 |
| M77830 | Desmoplakin 1 (DPI) | 184 | 81 | 0.0092 | Seq. ID No.32 |
| H81220 | EST domain tanscription factor ELF1 | 113 | 55 | 0.017 | Seq. ID No.33 |
| AF000592 | Human chromosome 21q11-q21 genomic clone | 33 | 69 | 1.16E-05 | Seq. ID No.35 |
| AK021701 | Human cDNA FLJ11639 | 31 | 63 | 0.00070 | Seq. ID No.36 |

**Example 4: Optimized Portfolio for Colorectal Tumors.**

The mean-variance optimization algorithm was used to generate a multiple gene-based signature, where the genes that are included can be used in combination to distinguish between the normal and tumor samples. Intensity measurements were processed using the samples and microarrays described in Examples 1-3. The data to be analyzed was first preselected based on a pre-specified 5-fold differential between tumor and normal cells. The expression data from genes preselected according to this criteria were then used as follows. The mean and standard deviation of the intensity measurements for each gene were calculated using the non-metastatic samples as the baseline. A discriminating value of X*(Standard Deviation + Mean) was then calculated for each baseline gene ( X was assigned a value of 3). This value was used to ensure the resulting portfolio would be stringent. A ratio of the discriminating value to the baseline value was then calculated for each metastatic sample. This ratio was then converted to a common logarithm. This data was then imported into Wagner Software which produced an efficient frontier from which a portfolio of 4 genes was selected. The set included an unknown sequence, procollagen type I, large subunit of ribosomal protein L21, and fibronectin. These genes are identified as Seq. ID No 42, Seq. ID No. 43, Seq. ID No. 44 and Seq. ID No. 49 (further identified below). Alternatively, a combination of genes used to make up the portfolio can be used to produce diagnostic information that is useful for making clinical decisions regarding colorectal cancer. This is particularly beneficial in the case when a combination of genes selected from the portfolio are combined with additional markers (genetic or not).

Genes selected for the optimized portfolio:

>gi|1264443|gb|N92134.1|N92134 za23f09.r1 Soares fetal liver spleen 1NFLS Homo sapiens cDNA clone IMAGE: 293417 5' similar to gb|M87908|HUMALNE32 Human carcinoma cell-derived Alu RNA transcript, (rRNA); gb: X57025_rna1 INSULIN-LIKE GROWTH FACTOR IA PRECURSOR (HUMAN)
>gi|2221047|gb|AA490172.1|AA490172 ab06b08.s1 Stratagene fetal retina 937202 Homo sapiens cDNA clone IMAGE:839991 3' similar to gb:J03464 PROCOLLAGEN ALPHA 2(I) CHAIN PRECURSOR (HUMAN)
>gi|2188918|gb|AA464034.1|AA464034 zx86b09.r1 Soares ovary tumor NbHOT Homo sapiens cDNA clone IMAGE:810617 5' similar to SW:RL21_HUMAN P46778 60S RIBOSOMAL PROTEIN L21.
>gi|834491|gb|R62612.1|R62612 yi12d01.s1 Soares placenta Nb2HP Homo sapiens cDNA clone IMAGE:139009 3' similar to gb:X02761_cds1 FIBRONECTIN PRECURSOR (HUMAN);

Using a different set of criteria but the same method, a further four gene portfolio was selected by the software. These are Seq. ID No. 46, Seq. ID No. 47, Seq. ID No. 48 and Seq. ID No. 45. Two genes overlap with the first four-gene portfolio. The two optimized portfolios can also be combined to form a six-gene portfolio.

Optimized Gene Portfolio:

>gi|2114953|gb|AA431245.1|AA431245 zw78d06.r1 Soares_testis_NHT Homo sapiens cDNA clone IMAGE: 782315 5' similar to WP:F36H1.2 CE05814 ANKYRIN LIKE
>gi|2156172|gb|AA443497.1|AA443497 zw34d03.r1 Soares ovary tumor NbHOT Homo sapiens cDNA clone IMAGE:771173
>gi|2221047|gb|AA490172.1|AA490172 ab06b08.s1 Stratagene fetal retina 937202 Homo sapiens cDNA clone IMAGE:839991 3' similar to gb:J03464 PROCOLLAGEN ALPHA 2(I) CHAIN PRECURSOR (HUMAN)
>gi|1264443|gb|N92134.1|N92134 za23f09.r1 S oares fetal liver spleen 1NFLS Homo sapiens cDNA clone IMAGE: 293417 5' similar to gb|M87908|HUMALNE32 Human carcinoma cell-derived Alu RNA transcript, (rRNA); gb: X57025_rna1 INSULIN-LIKE GROWTH FACTOR IA PRECURSOR (HUMAN);

**Claims**

1. A method of assessing colorectal cancer status comprising identifying differential modulation of each gene (relative to the expression of the same genes in a normal population) in a combination of genes selected from the group consisting of Seq. ID. No.1-33, Seq. ID No. 35-36 and Seq. ID No. 38-41.

2. The method of claim 1 wherein there is at least a 2 fold difference in the expression of the modulated genes.

3. The method of claim 1 wherein the p-value indicating differential modulation is less than .05.

4. The method of claim 1 further comprising employing a colorectal diagnostic that is not genetically based.

5. The method of claim 4 wherein the cancer marker that is not genetically based is selected from the group consisting of carcinomebryonic antigen, CA19-9, CA 125, CK-BB, and Guanylyl Cyclase C.

6. A diagnostic portfolio comprising isolated nucleic acid sequences, their complements, or portions thereof of a combination of genes selected from the group consisting of Seq. ID. No.1-33, Seq. ID No. 35-36 and Seq. ID No. 38-41.

7. The diagnostic portfolio of claim 6 in a matrix suitable for identifying the differential expression of the genes contained therein.

8. The diagnostic portfolio of claim 7 wherein said matrix is employed in a microarray.

9. The diagnostic portfolio of claim 8 wherein said microarray is a cDNA microarray.

10. The diagnostic portfolio of claim 8 wherein said microarray is an oligonucleotide microarray.

11. A diagnostic portfolio comprising isolated nucleic acid sequences, their complements, or portions thereof of a combination of genes selected from the group consisting of Seq. ID. No.1-33, Seq. ID No. 35-36 and Seq. ID No. 38-41.

12. A kit for diagnosing colorectal cancer comprising isolated nucleic acid sequences, their complements, or portions thereof of a combination of genes selected from the group consisting of Seq. ID. No.1-33, Seq. ID No. 35-36 and Seq. ID No. 38-41.

13. The kit of claim 12 further comprising reagents for conducting a microarray analysis.

14. The kit of claim 12 further comprising a medium through which said nucleic acid sequences, their complements, or portions thereof are assayed.

15. A method of assessing response to treatment for colorectal cancer comprising identifying differential modulation of each gene (relative to the expression of the same genes in a normal population) in a combination of genes selected from the group consisting of Seq. ID. No.1-33, Seq. ID No. 35-36 and Seq. ID No. 38-41.

16. The method of claim 15 wherein the assessment of the response to therapy includes a determination of whether the patient is improving, not improving, relapsing, likely to improve, or likely to relapse.

17. Articles for assessing colorectal cancer status comprising isolated nucleic acid sequences, their complements, or portions thereof of a combination of genes selected from the group consisting of Seq. ID. No.1-33, Seq. ID No. 35-36 and Seq. ID No. 38-41.

18. Articles for assessing colorectal cancer status comprising representations of isolated nucleic acid sequences, their complements, or portions thereof of a combination of genes selected from the group consisting of Seq. ID. No. 1-33, Seq. ID No. 35-36 and Seq. ID No. 38-41.